# EUROPEAN PATENT SPECIFICATION

(11) **EP 2 285 787 B1**
(45) Date of publication and mention of the grant of the patent: **26.08.2015**
(21) Application number: 09758543.4
(22) Date of filing: 05.06.2009
(51) Int. Cl.: C07D 257/04, C07D 249/04, C07D 249/18, C07D 233/58, A61K 31/41, A61K 31/4174, A61K 31/4192, A61K 31/4418, A61P 25/00, A61P 25/04, A61P 25/22, A61P 25/24, C07D 401/06, C07D 403/06, C07D 405/06, C07D 409/06

(54) **3-SUBSTITUTED PROPANAMINE COMPOUNDS**
3-SUBSTITUIERTE PROPANAMINVERBINDUNGEN
COMPOSÉS DE PROPANAMINE À SUBSTITUTION 3

(30) Priority: 05.06.2008 US 59109 P
(43) Date of publication of application: 23.02.2011
(73) Proprietor: SK Biopharmaceuticals Co., Ltd., Seoul 110-110 (KR)
(72) Inventor: PARK, Chun-Eung, Seoul 151-864 (KR); MIN, Kyung-Hyun, Daejeon 305-712 (KR); SHIN, Yong-Je, Daejeon 302-740 (KR); SHIN, Yu-Jin, Daejeon 301-791 (KR); YOON, Hae-Jeong, Daejeon 305-509 (KR); KIM, Won, Daejeon 305-746 (KR); RYU, Eun-Ju, Daejeon 302-121 (KR); CHUNG, Coo-Min, Daejeon 302-739 (KR); KIM, Hui-Ho, Daejeon 302-858 (KR)
(74) Representative: Nederlandsch Octrooibureau
(86) International application number: PCT/KR2009/003041
(87) International publication number: WO 2009/148290

(56) References cited:
- WO-A1-97/23480
- WO-A1-2005/118539
- WO-A1-2005/118539
- WO-A1-2007/006132
- WO-A1-2008/140197
- WO-A2-2010/044585
- US-A- 4 710 502
- US-A1- 2007 123 535
- DATABASE CA [Online] CHEMICAL ABSTRACTS SERVICE, COLUMBUS, OHIO, US; 1991, ILARIONOV, I. ET AL: "Relationship between antireserpine and aphrodisiac effects. Pharmacologic aspects", XP002669686, retrieved from STN Database accession no. 1991:135923 & ILARIONOV, I. ET AL: "Relationship between antireserpine and aphrodisiac effects. Pharmacologic aspects", FARMATSIYA (SOFIA, BULGARIA) , 40(3), 32-8 CODEN: FMTYA2; ISSN: 0428-0296, 1990,
- AVRAMOVA, P. ET AL: "Synthesis and pharmacologic assessment of carbamic and carbonic acid esters with 1-aryl-3-dimethylamino-1-propanols. Part 3", PHARMAZIE, CODEN: PHARAT; ISSN: 0031-7144, vol. 38, no. 7, July 1983 (1983-07), pages 443-444, XP002920580,
- AVRAMOVA, P. ET AL: "Synthesis and pharmacologic study of the esters of carbamic and carbonic acids with 1-phenyl-2,2-dimethyl-3-dialkylamino-1-pro panols. Part 2", PHARMAZIE, CODEN: PHARAT; ISSN: 0031-7144, vol. 36, no. 6, June 1981 (1981-06), pages 410-412, XP002669687,
- SPASOV, S. ET AL: "Stereochemistry of diastereomeric 3-(dialkylamino)propanols and O-substituted derivatives", JOURNAL FUER PRAKTISCHE CHEMIE (LEIPZIG), CODEN: JPCEAO; ISSN: 0021-8383, vol. 323, no. 5, 1981, pages 793-800, XP002669688,
- DATABASE CA [Online] CHEMICAL ABSTRACTS SERVICE, COLUMBUS, OHIO, US; 1999, MATSUI, TAKEAKI ET AL: "Preparation of 3-(5-isoxazolyl)- or 3-phenylpropylamine derivatives as central muscle relaxants", XP002669689, retrieved from STN Database accession no. 1999:566027 -& WO 99/43656 A1 (MARUHO KABUSHIKIKAISHA, JAPAN) 2 September 1999 (1999-09-02)
- DATABASE CA [Online] CHEMICAL ABSTRACTS SERVICE, COLUMBUS, OHIO, US; 2005, OSADEBE ET AL: "Determination and correlation of the reversed-phase thin-layer chromatographic parameter (Rm) of a series of 3-(4-substituted-1-piperazinyl)-1-substitu ted-1-phenyl propanol derivatives with their LD50 values", XP002669690, retrieved from STN Database accession no. 2005:534821 & OSADEBE ET AL: "Determination and correlation of the reversed-phase thin-layer chromatographic parameter (Rm) of a series of 3-(4-substituted-1-piperazinyl)-1-substitu ted-1-phenyl propanol derivatives with their LD50 values", BOLLETTINO CHIMICO FARMACEUTICO , 143(6), 253-260 CODEN: BCFAAI; ISSN: 0006-6648, 2004,
- DATABASE REGISTRY [Online] CHEMICAL ABSTRACTS SERVICE, COLUMBUS, OHIO, US; 8 June 2008 (2008-06-08), XP002669691, Database accession no. 1026221-94-2
- DATABASE REGISTRY [Online] CHEMICAL ABSTRACTS SERVICE, COLUMBUS, OHIO, US; 8 June 2008 (2008-06-08), XP002669692, Database accession no. 1026357-55-0
- DATABASE REGISTRY [Online] CHEMICAL ABSTRACTS SERVICE, COLUMBUS, OHIO, US; 12 June 2008 (2008-06-12), XP002669693, Database accession no. 1027550-70-4

## Description

### Technical Field

The present invention relates, in general, to racemic or enantiomerically enriched novel 3-substituted propanamine derivatives and pharmaceutically useful salts thereof, a pharmaceutical composition comprising an effective amount of racemic or enantiomerically enriched novel 3-substituted propanamine derivatives as monoamine neurotransmitter re-uptake inhibitors to treat central nervous system diseases and a method of treating central nervous system diseases in a mammal. More particularly, the present invention relates to racemic or enantiomerically enriched novel 3-substituted propanamine derivatives having various azole moieties and pharmaceutically useful salts thereof, useful to treat the diseases of the central nervous system such as depression, anxiety and pain disorder.

### Background Art

The three biogenic amines, serotonin, norepinephrein and dopamine are most closely linked to CNS disorders such as depression. The majority of antidepressants in current use selectively inhibit the reuptake of serotonin and/or norepinephrine. Although a strong dopamine re-uptake inhibiting activity is considered with the risk of undesirable central stimulation effects, many reports have disclosed that the triple monoamine neurotransmitter, i.e serotonin, norepinephrine and dopamine, re-uptake inhibitors are useful for the treatment of CNS disorders such as depression, anxiety, attention deficit hyperactivity disorder, obesity, drug addiction and pain disorder. For example, International Patent Application No. WO 2004/072071 discloses the novel 8-aza-bicyclo[3,2,1]octane derivatives useful as monoamine neurotransmitter re-uptake inhibitors.

3-substituted propanamine compounds are effectively used for controlling various central nervous system (CNS) disorders. For example, International Patent Application No. WO 04/43931 discloses 3-substituted propanamine derivatives that are suitable for treating anxiety, depression, sleep disorder. Similarly heteroaryloxy 3-substituted propanamine is disclosed in U.S. Pat. No. 7037932 and 3-Amino-1-arylpropyl indole derivatives are disclosed in International Patent Application No. WO 05/118539 as monoamine reuptake inhibitor for the treatment of various central nervous system disorders.

### Disclosure of Invention

### Technical Problem

Active research and development efforts have continued to be directed to the application of 3-substituted propanamine compounds for the treatment of CNS disorders.

### Technical Solution

A principal object of the present invention is to provide 3-substituted propanamine derivatives, represented by the following structural formula (1) : 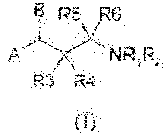 or any of its isomers or pharmaceutically acceptable salts thereof
wherein
A is selected from the group consisting of phenyl, naphthyl, benzothienyl, pyridyl, quinolyl, isoquinolyl, benzodioxolyl, benzodioxinyl, indolyl, fluorenyl and benzofuranyl, which can be substituted with one or more identical or different substituents selected from the group consisting of hydrogen, halogen, straight or branched C_{1∼4} alkyl, straight or branched C_{1∼3} alkoxy, phenyl, phenyloxy, nitro, cyano, trifluo- romethyl, trifluoromethoxy, methanesulfonyl and thienyl;
R₁ and R₂ are same or different and independently selected from the group consisting of hydrogen, straight or branched C_{1∼4} alkyl and substituted or unsubstituted phenyl C_{1∼4} alkyl; or
R₁ and R₂ are fused with nitrogen atom to form cyclic group which has 4 to 7 carbon atoms;
R₃, R₄, R₅ and R₆ are same or different and independently selected from the group consisting of hydrogen, straight or branched C_{1∼4} alkyl and substituted or unsubstituted phenyl;
B is an azole selected from the group consisting of triazole, benzotriazole, tetrazole, 5-methyl tetrazole and 5-phenyl tetrazole which are linked by nitrogen as represented by the following structural formula (II) :

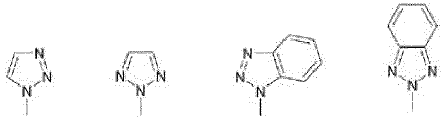

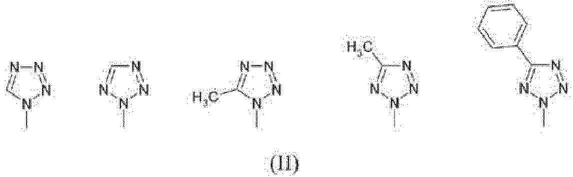

More specifically, the present 3-substituted propanamine compounds represented by the above formula (I) comprise racemic of enantiomerically enriched compounds represented by the following structural formula (III) : 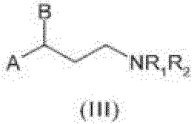 wherein A, B, R₁ and R₂ are as defined therein and each of R₃ through R₆ is hydrogen.

More specifically, the present 3-substituted propanamine compounds represented by the above formula (III) comprise any of its enantiomeric isomers represented by the following structural formula (IV) and (V): 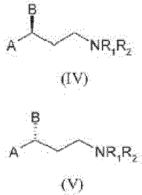 wherein
A, B, R₁ and R₂ are as defined above.

More specifically, the present 3-substituted propanamine compounds represented by the above formula (I) comprise racemic of enantiomerically enriched compounds represented by the following structural formula (XI) 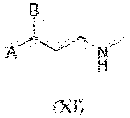 wherein A, B are as defined therein, each of R₃ through R₆ is hydrogen and one of R₁ and R₂ is hydrogen and the other is methyl

It is another object of the present invention to provide a pharmaceutical composition comprising an effective amount of racemic or enantiomerically enriched 3-substituted propanamine compounds represented by the above structural formula (I), in particular, the compounds represented by the above structural formula (III), (IV) and (V) for treating disorders of central nervous system such as depression, anxiety and pain disorder.

It is still another object of the present invention to provide racemic or enantiomerically enriched 3-substituted propanamine compounds represented by the above structural formula (I), in particular, the compounds represented by the above structural formula (III), (IV) and (V) and a pharmaceutical acceptable carrier, for use in a method of treating disorders of central nervous system such as depression, anxiety and pain disorder in a mammal by administering an effective amount to a mammal in need thereof.

### Best Mode for Carrying out the Invention

In accordance with the present invention, the compound represented by the structural formula (I) and pharmaceutical acceptable salts thereof can be prepared by techniques and procedures readily available to one of ordinary skill in the art, for example by following the procedures as set forth in the following schemes. These schemes are not intended to limit the scope of the invention in any way. All substituents, unless otherwise indicated, are previously defined. The reagents and starting materials are readily available to one of ordinary skill in the art.

In accordance with the present invention, the compounds of this invention represented by the structural formula (I), (III), (IV) and (V) and pharmaceutically acceptable salts thereof can be prepared by the following steps starting from readily available starting materials represented by the following general formula (VI) 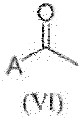 wherein
A is selected from the group consisting of phenyl, naphthyl, benzothienyl, pyridyl, quinolyl, isoquinolyl, benzodioxolyl, benzodioxinyl, indolyl,fluorenyl and benzofuranyl, which can be substituted with one or more identical or different substituents selected from the group consisting of hydrogen, halogen, straight or branched chain alkyl of from 1 to 4 carbon atoms, straight or branched chain alkoxy of from 1 to 3 carbon atoms, phenyl, phenyloxy, nitro, cyano, trifluoromethyl, trifluoromethoxy, methanesulfonyl and thienyl;

The method for preparing the novel compounds of the general formula (III) will be described below in detail.

Initially, the starting material represented by the above general formula (VI) is reacted with readily available amine (VII) and paraformaldehyde

HNR₁R₂ (VII)

wherein
R₁ and R₂ are same or different and independently selected from the group consisting of hydrogen, lower alkyl of from 1 to 4 carbon atoms, substituted or unsubstituted phenyl-C₁₋₄ alkyl or R₁ and R₂ are fused with nitrogen atom to form cyclic group which has 4 to 7 carbon atoms;
to synthesize 1,3-aminoketone compound represented by the general formula (VIII). 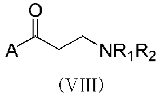 wherein
   A, R₁ and R₂ are the same as defined above.

The compound of formula (VIII) is followed by the treatment with sodium borohydride in methanol to yield the corresponding 1,3-diaminoalchol compound represented by the general formula (IX).

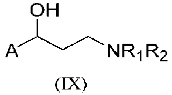

The compound of formula (IX) is reacted with triphenylphosphine, diisopropyl azodicarboxylate and azole compounds such as imidazole, triazole, tetrazole, 5-methyltetrazole, 5-phenyltetrazole, benzotriazole represented by the structural formula (II) to give the compound represented by the general formula (III). 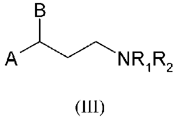 wherein
A and Bare the same as defined above.
R₁ and R₂ are same and different and independently selected from the group consisting of hydrogen, lower alkyl of from 1 to 4 carbon atoms, substituted or unsubstituted phenyl-C₁₋₄ alkyl or R₁ and R₂ are fused to form cyclic group with nitrogen atom;

This procedure is summarized as set forth in Reaction Scheme I below.

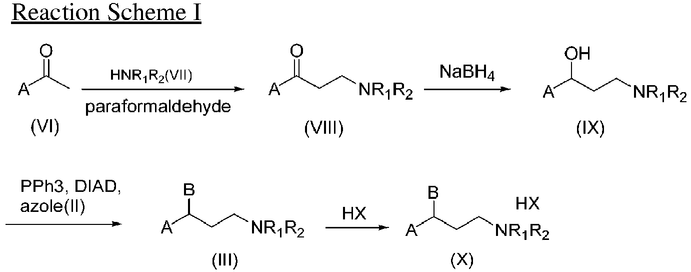

Details of the reaction condition described in reaction scheme I are as follows.

In the first step, the concentration of the starting material (VI) is about to 0.005 to 3 moles with paraformaldehyde ranging from about 3.0 to 5.0 equivalents, amine (VII) ranging from about 3.0 to 5.0 equivalents and aq. RCl. The reaction is carried out at temperature about 90?. For the conversion of the compound (VIII) to the compound (IX), sodium borohydride ranging from about 1.0 to 2.5 equivalents is used in methanol. The resulting compound is treated with 1.5 equivalents of PPh3, diisopropyl azodicarboxylate and azole compound to give the compound (III).

Additionally, the compounds of formula (III) may be converted into pharmaceutically acceptable salts (X) by treating the compound (III) with acid as described in the reaction scheme I. In the reaction scheme I, HX represents an acid capable of forming a pharmacologically useful salt with the basic nitrogen atom. Specific examples of the anhydrous acid used for the preparation of the compound (X) from the compound (III) include hydrochloric acid, hydrobromic acid, sulfuric acid, phosphoric acid, acetic acid, benzoic acid, carbonic acid, citric acid, malonic acid, salicylic acid, malic acid, fumaric acid, oxalic acid, succinic acid, tartaric acid, lactic acid, gluconic acid, ascorbic acid, maleic acid, aspartic acid, camphorsulfonic acid, p-toluenesulfonic acid, benzene sulfonic acid, methane sulfonic acid, ethane sulfonic acid, hydroxymethane sulfonic acid and hydroxyethane sulfonic acid and the like. Additional acids can refer to "Pharmaceutical Salts", J. Pharm. Sci., 1977; 66(1): 1-19. This preparation is executed in a reaction media which can be exemplified by an ethereal solvent such as THF, an alcoholic solvent such as methanol, an ester solvent such as ethyl acetate, and aromatic solvent, and any compositional mixture thereof. An ethereal solvent is recommended as an addition solution, including ethyl ether, propyl ether, isopropyl ether, butyl ether, isobutyl ether. The concentration of the compound (III) is on the order of about 0.01 to 5 moles.

The methods for preparing the compounds of the general formula (III) in which R₁ is hydrogen and R₂ is methyl, i.e. the compounds of the general formular (XI), will be described below.

The compounds of general formula (III) wherein R₁ and R₂ are methyl is treated with ethyl chloroformate, sodium bicarbonate and potassium hydroxide to yield the monomethyl amine compound represented by the structural formula (XI). 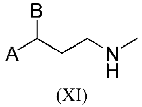 Wherein
A and Bare the same as defined above.

The compound of formula (XI) may be converted into pharmaceutically acceptable salt (XII) as described above.

This procedure of preparing the compounds of general formula (XI) and pharmaceutically acceptable salts thereof is summarized as set forth in Reaction Scheme II below.

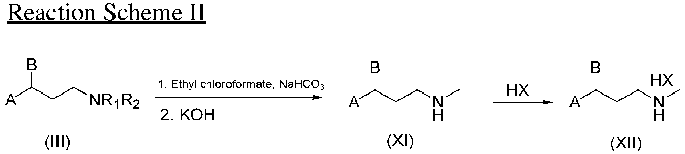

Details of the reaction condition described in reaction scheme II are as follows. For the conversion of compound(III) in which R₁ and R₂ are methyl, to the compound (XI), ethyl chloroformate ranging from 10 to 15 equivalents, sodium bicarbonate ranging from 20 to 25 equivalents and similar amount of potassium hydroxide are used at temperature about 100°C.

In reaction scheme II, HX represents an acid capable of forming a pharmacologically useful salt with the basic nitrogen atom as described above.

Enantiomeric isomers represented by the structural formula (IV) and (V) are obtained from racemic mixture (III) by means of chiral preparative Liquid Chromatography ("Prep-LC"). The methods for preparing the general formula (IV) and (V) will be described below.

The racemic mixture (III) is dissolved in a small amount of isopropylalcohol and separated by chiral preparative Liquid Chromatography. Separation is performed by using a CHIRALPACK OD-H column (manufactured by Daicel Chemical Industries, Ltd.) as the Prep-LC column, at a column temperature of 25°C, with n-hexane / isopropylalcohol including 0.1% triethylamine (90:10) as the eluent. Enantiomeric isomers represented by the structural formula (IV) and (V) can be obtained in the ratio of 1:1 from the racemic mixture (III).

This procedure is summarized as set forth in reaction scheme III below.

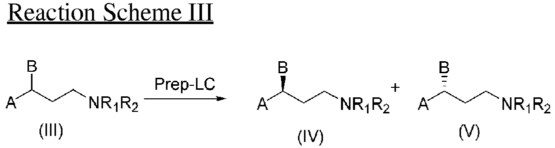

In reaction scheme III, HX represents an acid capable of forming a pharmacologically useful salt with the basic nitrogen atom as described above.

The compounds of formula (IV) and (V) may be converted into pharmaceutically acceptable salts (XIII) and (XIV) as described above.

The compounds prepared according to the above methods include the following compounds.
[3-(3,4-Dichloro-phenyl)-3-tetrazol-2-yl-propyl]-dimethyl-amine
[3-(3,4-Dichloro-phenyl)-3-tetrazol-2-yl-propyl]-methyl-amine
S-[3-(3,4-Dichloro-phenyl)-3-tetrazol-2-yl-propyl]-methyl-amine
[3-Benzotriazol-2-yl-3-(3,4-dichloro-phenyl)-propyl]-dimethyl-amine
Dimethyl-(3-naphthalen-2-yl-3-[1,2,3]triazol-2-yl-propyl)-amine
Dimethyl-(3-naphthalen-2-yl-3-tetrazol-2-yl-propyl)-amine
(3-Benzotriazol-2-yl-3-naphthalen-2-yl-propyl)-dimethyl-amine
Dimethyl-[3-(5-methyl-tetrazol-2-yl)-3-naphthalen-2-yl-propyl]-amine
[3-(6-Chloro- naphthalen- 2- yl)- 3- tetrazol-l-yl- propyl] -dimethyl-amine
Dimethyl-[3-(4-phenoxy-phenyl)-3-tetrazol-2-yl-propyl]-amine
Dimethyl-[3-(4-phenoxy-phenyl)-3-tetrazol-1-yl-propyl]-amine
[3-(6-Chloro-naphthalen-2-yl)-3-[1,2,3]triazol-2-yl-propyl]-dimethyl-amine
[3-(6-Chloro-naphthalen-2-yl)-3-[1,2,3] triazol-1-yl-propyl]-dimethyl-amine
[3-(6-Chloro-naphthalen-2-yl)-3-tetrazol-2-yl-propyl]-dimethyl-amine
[3-(4-Methoxy-phenyl)-3-1,2,3]triazol-2-yl-propyl]-dimethyl-amine
(3-Biphenyl-3-yl-3-tetrazol-2-yl-propyl)-dimethyl-amine
[3-(4-Benzyloxy-phenyl)-3-tetrazol-2-yl-propyl]-dimethyl-amine
(3-Biphenyl-4-yl-3-tetrazol-2-yl-propyl)-dimethyl-amine
Dimethyl-[3-naphthalen-2-yl-3-(5-phenyl-tetrazol-2-yl)-propyl]-amine
Dimethyl-[3-(6-methyl-naphthalen-2-yl)-3-tetrazol-2-yl-propyl]-amine
[3-(6-Methoxy-naphthalen-2-yl)-3-tetrazol-2-yl-propyl]-dimethyl-amine
[3-(6-Methoxy-naphthalen-2-yl)-3-[1,2,3]triazol-2-yl-propyl]-dimethyl-amine
[3-Benzotriazol-l-yl-3-(3,4-dichloro-phenyl)-propyl]-dimethyl-amine
2-[1-(3,4-Dichloro-phenyl)-3-pyrrolidin-1-yl-propyl]-2H-[1,2,3] triazole
[3-(3,4-Dichloro-phenyl)-3-[1,2,3]triazol-2-yl-propyl]-diethyl-amine
[3-(3,4-Dichloro-phenyl)-3-tetrazol-2-yl-propyl]-diethyl-amine
2-[1-(3,4-Dichloro-phenyl)-3-pyrrolidin-1-yl-propyl]-2H-tetrazole
Dimethyl-(3-naphthalen-1-yl-3-tetrazol-2-yl-propyl)-amine
Dimethyl-(3-naphthalen-1-yl-3-[1,2,3]triazol-2-yl-propyl)-amine
[3-(3,4-Dichloro-phenyl)-3-(5-methyl-tetrazol-2-yl)-propyl]-dimethyl-amine
[3-(3,4-Dichloro-phenyl)-3-(5-phenyl-tetrazol-2-yl)-propyl]-dimethyl-amine
[3-(3,4-Difluoro-phenyl)-3-tetrazol-1-yl-propyl]-dimethyl-amine
[3-(3,4-Difluoro-phenyl)-3-tetrazol-2-yl-propyl]-dimethyl-amine
[3-(3,4-Difluoro-phenyl)-3-[1,2,3]triazol-1-yl-propyl]-dimethyl-amine
[3-(3,4-Difluoro-phenyl)-3-[1,2,3]triazol-2-yl-propyl]-dimethyl-amine
[3-(3,4-Dimethyl-phenyl)-3-[1,2,3]triazol-1-yl-propyl]-dimethyl-amine
[3-(3,4-Dimethyl-phenyl)-3-[1,2,3]triazol-2-yl-propyl]-dimethyl-amine
[3-(3,4-Dimethyl-phenyl)-3-tetrazol-1-yl-propyl]-dimethyl-amine
[3-(3,4-Dimethyl-phenyl)-3-tetrazol-2-yl-propyl]-dimethyl-amine
[3-(4-Chloro-phenyl)-3-tetrazol-1-yl-propyl]-dimethyl-amine
[3-(4-Chloro-phenyl)-3-tetrazol-2-yl-propyl]-dimethyl-amine
Dimethyl-(3-phenyl-3-[1,2,3]triazol-1-yl-propyl)-amine
Dimethyl-(3-phenyl-3-[1,2,3]triazol-2-yl-propyl)-amine
[3-(4-Chloro-phenyl)-3-[1,2,3]triazol-1-yl-propyl]-dimethyl-amine
[3-(4-Chloro-phenyl)-3-[1,2,3]triazol-2-yl-propyl]-dimethyl-amine
[3-(3,4-Dichloro-phenyl)-3-[1,2,3]triazol-2-yl-propyl]-dimethyl-amine
[3-(3,4-Dichloro-phenyl)-3-imidazol-1-yl-propyl]-dimethyl-amine
[3-(3-Methoxy-naphthalen-2-yl)-3-tetrazol-2-yl-propyl]-dimethyl-amine
[3-(3-Methoxy-naphthalen-2-yl)-3-(5-methyl-tetrazol-2-yl)-propyl]-dimethyl-amine
[3-(6-Fluoro-naphthalen-2-yl)-3-tetrazol-2-yl-propyl]-dimethyl-amine
[3-(6-Fluoro-naphthalen-2-yl)-3-(5-methyl-tetrazol-2-yl)-propyl]-dimethyl-amine
[3-(1,4-Dimethoxy-naphthalen-2-yl)-3-tetrazol-2-yl-propyl]-dimethyl-amine
[3-(1,4-Dimethoxy-naphthalen-2-yl)-3-(5-methyl-tetrazol-2-yl)-propyl]-dimethyl-ami ne
[3-(3,5-Dimethoxy-naphthalen-2-yl)-3-tetrazol-2-yl-propyl]-dimethyl-amine
[3-(3,5-Dimethoxy-naphthalen-2-yl)-3-(5-methyl-tetrazol-2-yl)-propyl]-dimethyl-ami ne
[3-(3,5-Dimethoxy-naphthalen-2-yl)-3-tetrazol-1-yl-propyl]-dimethyl-amine
Dimethyl-[3-(4-methyl-naphthalen-1-yl)-3-tetrazol-2-yl-propyl]-amine
Dimethyl-[3-(4-methyl-naphthalen-1-yl)-3-(5-methyl-tetrazol-2-yl)-propyl]-amine
[3-(4-Fluoro-naphthalen-1-yl)-3-tetrazol-2-yl-propyl]-dimethyl-amine
[3-(4-Fluoro-naphthalen-1-yl)-3-(5-methyl-tetrazol-2-yl)-propyl]-dimethyl-amine
(3-Isoquinolin-l-yl-3-tetrazol-2-yl-propyl)-dimethyl-amine
[3-Isoquinolin-1-yl-3-(5-methyl-tetrazol-2-yl)-propyl]-dimethyl-amine
(3-Benzo[1,3]dioxol-5-yl-3-tetrazol-2-yl-propyl)-dimethyl-amine
[3-Benzo[1,3]dioxol-5-yl-3-(5-methyl-tetrazol-2-yl)-propyl]-dimethyl-amine
[3-Benzo[1,3]dioxol-5-yl-3-(5-methyl-tetrazol-1-yl)-propyl]-dimethyl-amine
[3-(3,4-Dimethoxy-phenyl)-3-tetrazol-2-yl-propyl]-dimethyl-amine
[3-(2,3-Dihydro-benzo[1,4]dioxin-2-yl)-3-tetrazol-2-yl-propyl]-dimethyl-amine
[3-(2,3-Dihydro-benzo[1,4]dioxin-2-yl)-3-(5-methyl-tetrazol-2-yl)-propyl]-dimethylamine
Dimethyl-(3-quinolin-2-yl-3-tetrazol-2-yl-propyl)-amine
Dimethyl-[3-(5-methyl-tetrazol-2-yl)-3-quinolin-2-yl-propyl]-amine
Dimethyl-[3-(1-methyl-1H-indol-2-yl)-3-tetrazol-2-yl-propyl]-amine
Dimethyl-[3-(1-methyl-1H-indol-2-yl)-3-(5-methyl-tetrazol-2-yl)-propyl]-amine
Dimethyl-[3-(1,2,3,4-tetrahydro-naphthalen-2-yl)-3-tetrazol-2-yl-propyl]-amine
[3-(6-Chloro-pyridin-3-yl)-3-tetrazol-2-yl-propyl]-dimethyl-amine
(3-Benzo[b]thiophen-2-yl-3-tetrazol-2-yl-propyl)-dimethyl-amine
[3-Benzo[b]thiophen-2-yl-3-(5-methyl-tetrazol-2-yl)-propyl]-dimethyl-amine
(3-Benzofuran-2-yl-3-tetrazol-2-yl-propyl)-dimethyl-amine
[3-Benzofuran-2-yl-3-(5-methyl-tetrazol-2-yl)-propyl]-dimethyl-amine
(3-Benzofuran-2-yl-3-tetrazol-1-yl-propyl)-dimethyl-amine
[3-(3,4-Difluoro-phenyl)-3-(5-methyl-tetrazol-2-yl)-propyl]-dimethyl-amine
Dimethyl-[3-tetrazol-2-yl-3-(2,4,5-trifluoro-phenyl)-propyl]-amine
Dimethyl-[3-(5-methyl-tetrazol-2-yl)-3-(2,4,5-trifluoro-phenyl)-propyl]-amine
[3-(3-Bromo-4-fluoro-phenyl)-3-tetrazol-2-yl-propyl]-dimethyl-amine
[3-(3-Bromo-4-fluoro-phenyl)-3-(5-methyl-tetrazol-2-yl)-propyl]-dimethyl-amine
[3-(6-Methoxy-naphthalen-2-yl)-3-(5-methyl-tetrazol-2-yl)-propyl]-dimethyl-amine
Dimethyl-[3-(5-methyl-tetrazol-2-yl)-3-(4-phenoxy-phenyl)-propyl]-amine
[3-(4-Bromo-phenyl)-3-tetrazol-2-yl-propyl]-dimethyl-amine
[3-(4-Bromo-phenyl)-3-(5-methyl-tetrazol-2-yl)-propyl]-dimethyl-amine
Dimethyl-[3-tetrazol-2-yl-3-(4-trifluoromethyl-phenyl)-propyl]-amine
Dimethyl-[3-(5-methyl-tetrazol-2-yl)-3-(4-trifluoromethyl-phenyl)-propyl]-amine
Dimethyl-[3-tetrazol-2-yl-3-(2,3,4-trichloro-phenyl)-propyl]-amine
Dimethyl-[3-(5-methyl-tetrazol-2-yl)-3-(2,3,4-trichloro-phenyl)-propyl]-amine
[3-(3-Chloro-4-methoxy-phenyl)-3-tetrazol-2-yl-propyl]-dimethyl-amine
[3-(3-Chloro-4-methoxy-phenyl)-3-(5-methyl-tetrazol-2-yl)-propyl]-dimethyl-amine
[3-(4-tert-Butyl-phenyl)-3-(5-methyl-tetrazol-2-yl)-propyl]-dimethyl-amine
[3-(2,5-Difluoro-phenyl)-3-(5-methyl-tetrazol-2-yl)-propyl]-dimethyl-amine
[3-(2,4-Dichloro-phenyl)-3-(5-methyl-tetrazol-2-yl)-propyl]-dimethyl-amine
[3-(3-Chloro-phenyl)-3-(5-methyl-tetrazol-2-yl)-propyl]-dimethyl-amine
[3-(2-Chloro-phenyl)-3-(5-methyl-tetrazol-2-yl)-propyl]-dimethyl-amine
[3-(2,5-Difluoro-phenyl)-3-tetrazol-2-yl-propyl]-dimethyl-amine
[3-(2,4-Dichloro-phenyl)-3-tetrazol-2-yl-propyl]-dimethyl-amine
[3-(3-Chloro-phenyl)-3-tetrazol-2-yl-propyl]-dimethyl-amine
Dimethyl-[3-(5-methyl-tetrazol-2-yl)-3-(4-trifluoromethoxy-phenyl)-propyl]-amine
[3-(3-Bromo-4-methyl-phenyl)-3-(5-methyl-tetrazol-2-yl)-propyl]-dimethyl-amine
Dimethyl-[3-tetrazol-2-yl-3-(4-trifluoromethoxy-phenyl)-propyl]-amine
[3-(4-Bromo-3-methyl-phenyl)-3-tetrazol-2-yl-propyl]-dimethyl-amine
[3-(4-Methanesulfonyl-phenyl)-3-(5-methyl-tetrazol-2-yl)-propyl]-dimethyl-amine
[3-(4-Isopropyl-phenyl)-3-(5-methyl-tetrazol-2-yl)-propyl]-dimethyl-amine
[3-(9H-Fluoren-2-yl)-3-(5-methyl-tetrazol-2-yl)-propyl]-dimethyl-amine
Methyl-(3-naphthalen-2-yl-3-[1,2,3]triazol-2-yl-propyl)-amine
[3-Benzotriazol-2-yl-3-(3,4-dichloro-phenyl)-propyl]-methyl-amine
Methyl-[3-(5-methyl-tetrazol-2-yl)-3-naphthalen-2-yl-propyl]-amine
(3-Benzotriazol-2-yl-3-naphthalen-2-yl-propyl)-methyl-amine
(3-Biphenyl-4-yl-3-tetrazol-2-yl-propyl)-methyl-amine
[3-(3,4-Dichloro-phenyl)-3-(5-methyl-tetrazol-2-yl)-propyl]-methyl-amine
[3-(4-Chloro-phenyl)-3-tetrazol-2-yl-propyl]-methyl-amine
[3-(3,4-Dichloro-phenyl)-3-[1,2,3]triazol-2-yl-propyl]-methyl-amine
[3-(3,4-Dichloro-phenyl)-3-tetrazol-2-yl-propyl]-methyl-amine
Methyl-(3-naphthalen-2-yl-3-tetrazol-2-yl-propyl)-amine
[3-(6-Chloro-naphthalen-2-yl)-3-tetrazol-2-yl-propyl]-methyl-amine
Methyl-(3-naphthalen-1-yl-3-tetrazol-2-yl-propyl)-amine
Methyl-[3-(6-methyl-naphthalen-2-yl)-3-tetrazol-2-yl-propyl]-amine
Methyl-[3-naphthalen-2-yl-3-(5-phenyl-tetrazol-2-yl)-propyl]-amine
(3*S*)-3-(3,4-dichlorophenyl)-*N-*methyl-3-(2*H*-tetrazol-2-yl)propan-1-amine
(3*R*)-3-(3,4-dichlorophenyl)-*N*-methyl-3-(2*H*-tetrazol-2-yl)propan-1-amine
(3*S*)-3-(2*H*-benzotriazol-2-yl)-3-(3,4-dichlorophenyl)-*N*-methylpropan-1-amine
(3*R*)-3-(2*H*-benzotriazol-2-yl)-3-(3,4-dichlorophenyl)-*N*-methylpropan-1-amine
(3*S*)-3-(3,4-dichlorophenyl)-*N*-methyl-3-(2*H*-1,2,3-triazol-2-yl)propan-1-amine
(3*S*)-3-(3,4-dichlorophenyl)-*N*-methyl-3-(5-methyl-2*H*-tetrazol-2-yl)propan-1-amine
(3*R*)-3-(3,4-dichlorophenyl)-*N*-methyl-3-(5-methyl-2*H*-tetrazol-2-yl)propan-1-amine
(3*R*)-3-(3,4-dichlorophenyl)-*N*-methyl-3-(2*H*-1,2,3-triazol-2-yl)propan-1-amine
(3*R*)-*N*-methyl-3-(2-naphthyl)-3-(2*H*-tetrazol-2-yl)propan-1-amine
(3S)-*N*-methyl-3-(2-naphthyl)-3-(2*H*-tetrazol-2-yl)propan-1-amine
[3-(3-Methoxy-naphthalen-2-yl)-3-tetrazol-2-yl-propyl]-methyl-amine
[3-(3-Methoxy-naphthalen-2-yl)-3-(5-methyl-tetrazol-2-yl)-propyl]-methyl-amine
[3-(6-Fluoro-naphthalen-2-yl)-3-tetrazol-2-yl-propyl]-methyl-amine
[3-(6-Fluoro-naphthalen-2-yl)-3-(5-methyl-tetrazol-2-yl)-propyl]-methyl-amine
[3-(1,4-Dimethoxy-naphthalen-2-yl)-3-tetrazol-2-yl-propyl]-methyl-amine
[3-(1,4-Dimethoxy-naphthalen-2-yl)-3-(5-methyl-tetrazol-2-yl)-propyl]-methyl-amin e
[3-(3,5-Dimethoxy-naphthalen-2-yl)-3-tetrazol-2-yl-propyl]-methyl-amine
[3-(3,5-Dimethoxy-naphthalen-2-yl)-3-(5-methyl-tetrazol-2-yl)-propyl]-methyl-amin e
[3-(3,5-Dimethoxy-naphthalen-2-yl)-3-tetrazol-1-yl-propyl]-methyl-amine
Methyl-[3-(4-methyl-naphthalen-1-yl)-3-tetrazol-2-yl-propyl]-amine
Methyl-[3-(4-methyl-naphthalen-1-yl)-3-(5-methyl-tetrazol-2-yl)-propyl]-amine
[3-(4-Fluoro-naphthalen-1-yl)-3-tetrazol-2-yl-propyl]-methyl-amine
[3-(4-Fluoro-naphthalen-1-yl)-3-(5-methyl-tetrazol-2-yl)-propyl]-methyl-amine
(3-Benzo[1,3]dioxol-5-yl-3-tetrazol-2-yl-propyl)-methyl-amine
[3-Benzo[1,3]dioxol-5-yl-3-(5-methyl-tetrazol-2-yl)-propyl]-methyl-amine
[3-Benzo[1,3]dioxol-5-yl-3-(5-methyl-tetrazol-1-yl)-propyl]-methyl-amine
[3-(3,4-Dimethoxy-phenyl)-3-tetrazol-2-yl-propyl]-methyl-amine
[3-(2,3-Dihydro-benzo[1,4]dioxin-2-yl)-3-tetrazol-2-yl-propyl]-methyl-amine
[3-(2,3-Dihydro-benzo[1,4]dioxin-2-yl)-3-(5-methyl-tetrazol-2-yl)-propyl]-methyl-a mine
Methyl-[3-(1,2,3,4-tetrahydro-naphthalen-2-yl)-3-tetrazol-2-yl-propyl]-amine
[3-(6-Chloro-pyridin-3-yl)-3-tetrazol-2-yl-propyl]-methyl-amine
(3-Benzo[b]thiophen-2-yl-3-tetrazol-2-yl-propyl)-methyl-amine
[3-Benzo[b]thiophen-2-yl-3-(5-methyl-tetrazol-2-yl)-propyl]-methyl-amine
(3-Benzofuran-2-yl-3-tetrazol-2-yl-propyl)-methyl-amine
[3-Benzofuran-2-yl-3-(5-methyl-tetrazol-2-yl)-propyl]-methyl-amine
(3-Benzofuran-2-yl-3-tetrazol-1-yl-propyl)-methyl-amine
[3-(3,4-Difluoro-phenyl)-3-tetrazol-2-yl-propyl]-methyl-amine
[3-(3,4-Difluoro-phenyl)-3-(5-methyl-tetrazol-2-yl)-propyl]-methyl-amine
Methyl-[3-tetrazol-2-yl-3-(2,4,5-trifluoro-phenyl)-propyl]-amine
Methyl-[3-(5-methyl-tetrazol-2-yl)-3-(2,4,5-trifluoro-phenyl)-propyl]-amine
[3-(3-Bromo-4-fluoro-phenyl)-3-tetrazol-2-yl-propyl]-methyl-amine
[3-(3-Bromo-4-fluoro-phenyl)-3-(5-methyl-tetrazol-2-yl)-propyl]-methyl-amine
[3-(6-Methoxy-naphthalen-2-yl)-3-tetrazol-2-yl-propyl]-methyl-amine
[3-(6-Methoxy-naphthalen-2-yl)-3-(5-methyl-tetrazol-2-yl)-propyl]-methyl-amine
Methyl-[3-(4-phenoxy-phenyl)-3-tetrazol-2-yl-propyl]-amine
Methyl-[3-(5-methyl-tetrazol-2-yl)-3-(4-phenoxy-phenyl)-propyl]-amine
[3-(4-Bromo-phenyl)-3-tetrazol-2-yl-propyl]-methyl-amine
[3-(4-Bromo-phenyl)-3-(5-methyl-tetrazol-2-yl)-propyl]-methyl-amine
Methyl-[3-tetrazol-2-yl-3-(4-trifluoromethyl-phenyl)-propyl]-amine
Methyl-[3-(5-methyl-tetrazol-2-yl)-3-(4-trifluoromethyl-phenyl)-propyl]-amine
Methyl-[3-tetrazol-2-yl-3-(2,3,4-trichloro-phenyl)-propyl]-amine
Methyl-[3-(5-methyl-tetrazol-2-yl)-3-(2,3,4-trichloro-phenyl)-propyl]-amine
[3-(3-Chloro-4-methoxy-phenyl)-3-tetrazol-2-yl-propyl]-methyl-amine
[3-(3-Chloro-4-methoxy-phenyl)-3-(5-methyl-tetrazol-2-yl)-propyl]-methyl-amine
[3-(2-Chloro-phenyl)-3-(5-methyl-tetrazol-2-yl)-propyl]-methyl-amine
[3-(3-Chloro-phenyl)-3-(5-methyl-tetrazol-2-yl)-propyl]-methyl-amine
[3-(2,4-Dichloro-phenyl)-3-(5-methyl-tetrazol-2-yl)-propyl]-methyl-amine
[3-(2,5-Difluoro-phenyl)-3-(5-methyl-tetrazol-2-yl)-propyl]-methyl-amine
[3-(3-Chloro-phenyl)-3-tetrazol-2-yl-propyl]-methyl-amine
[3-(2,4-Dichloro-phenyl)-3-tetrazol-2-yl-propyl]-methyl-amine
[3-(2,5-Difluoro-phenyl)-3-tetrazol-2-yl-propyl]-methyl-amine
[3-(4-Methanesulfonyl-phenyl)-3-(5-methyl-tetrazol-2-yl)-propyl]-methyl-amine
[3-(4-Isopropyl-phenyl)-3-(5-methyl-tetrazol-2-yl)-propyl]-methyl-amine
[3-(9H-Fluoren-2-yl)-3-(5-methyl-tetrazol-2-yl)-propyl]-methyl-amine
(3S)-Methyl-[3-(5-methyl-tetrazol-2-yl)-3-naphthalen-2-yl-propyl]-amine
(3S)-Methyl-[3-naphthalen-2-yl-3-(5-phenyl-tetrazol-2-yl)-propyl]-amine
(3R)-Methyl-[3-(5-methyl-tetrazol-2-yl)-3-naphthalen-2-yl-propyl]-amine
(3R)-Methyl-[3-naphthalen-2-yl-3-(5-phenyl-tetrazol-2-yl)-propyl] -amine
(3R)-[3-(6-Fluoro-naphthalen-2-yl)-3-(5-methyl-tetrazol-2-yl)-propyl]-methyl-amine
(3S)-[3-(6-Fluoro-naphthalen-2-yl)-3-(5-methyl-tetrazol-2-yl)-propyl]-methyl-amine

The compound or pharmaceutically acceptable salt thereof according to the present invention can be used for treating CNS disorders such as depression, anxiety and pain disorder.

In therapeutic use as agents for various CNS disorders such as depression, anxiety and pain disorder, the compounds of the present invention, alone or in combination with pharmaceutically acceptable carrier, are administered to patients at a dosage of from 0.7 to 7,000mg per day. For a normal human adult with a body weight of approximately 70kg, the administration amount is translated into a daily dose of 0.01 to 100mg per kg of body weight. The specific dosage employed, however, may vary depending upon the requirements of the patient, the severity of patient's condition and the activity of the compound. The determination of optimum dosages for a particular situation must clinically be done and is within the skill of the art.

In utilizing the compounds of the present invention for the central nervous system, the compounds represented by general structural formula (I), (III), (IV) and (V) can be administered in any form or mode which makes the compound bioavailable in effective amounts, including the oral, rectal, transdermal, subcutaneous, intravenous, intramuscular or intranasal routs. However it is preferred to administer the compounds orally. Since the compounds absorb well orally, it usually will not be necessary to resort to parenteral administration. For oral administration, the compounds having the general formula (I), (III), (IV) and (V) is preferably combined with a pharmaceutical carrier. The ratio of the carrier to the compound of structural formula (I), (III), (IV) and (V) is not critical to express the effects of the medicine on the central nervous system, and they can vary considerably depending on whether the composition is to be filled into capsules or formed into tablets. In tableting, various edible pharmaceutical carriers or the mixture thereof can be used. A suitable carriers, for example, are a mixture of lactose, diabasic calcium phosphate and/or corn starch. Other pharmaceutically acceptable ingredients can be further added, including lubricants such as magnesium stearate.

The present invention includes compositions of the compounds of structural formula (I), (III), (IV) and (V) for use in methods of treating depression, anxiety and pain disorder in a mammal which comprises administering the composition to a mammal in need of therapy

### Mode for the Invention

A better understanding of the present invention may be obtained in light of following examples which are set forth to illustrate, but are not to be construed to limit, the present invention.

### Example 1

### [3-(3,4-Dichloro-phenyl)-3-tetrazol-2-yl-propyl]-dimethyl-amine

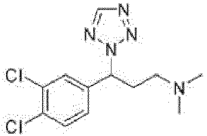

To a solution of 3,4-dichloroacetophenone(2mmol) in ethanol was added dimethylamine (5 equiv.), paraformaldehyde (5 equiv.) and aq. HCl. After 12h, the solvent was removed and ethyl acetate and 1N NaOH were added and organic layer was extracted 3 times. It was dried over MgSO₄, filtered, evaporated and the filtrate was concentrated at reduced pressure. The residue was purified by column chromatography eluting with ethyl acetate and hexane. The product was dissolved in methanol and added NaBH₄ (2 equiv.). After 2h, sat. NaHCO₃ and ethyl acetate were added and the organic layer was extracted 3 times with ethyl acetate. It was dried over MgSO₄, filtered, evaporated and the filtrate was concentrated at reduced pressure. The residue was purified by column chromatography eluting with ethyl acetate and methanol. The resulting residue was dissolved in THF and was added 1*H*-tetrazole (1.5equiv.), triphenylphosphine (1.5equiv.) and diisopropyl azodicarboxylate (1.5equiv.) dropwise at 0°C and warmed to room temperature. After 2h, the solvent was removed and the residue was purified by column chromatography eluting with ethyl acetate and methanol. : Yield 35%

1H-NMR(CDC13, 200MHz) δ2.2(m, 7H), 2.6(m, 2H), 6.0(t, 1H), 7.2(dd, 1H), 7.4(dd, 1H), 7.5(d, 1H), 8.5(s, 1H)

### Example 2

### [3-(3,4-Dichloro-phenyl)-3-tetrazol-2-yl-propyl]-methyl-amine

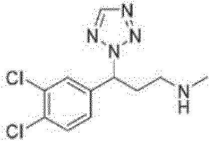

To a solution of [3-(3,4-Dichloro-phenyl)-3-tetrazol-2-yl-propyl]-dimethyl-amine(2mmol) in chloroform was added ethylchloroformate(>10 equiv.) and NaHCO₃(>20 equiv.) and this solution was refluxed for 3h. After that, chloroform was evaporated and ethyl acetate was added and then this organic layer was washed with water 3 times. It was dried over MgSO₄, filtered, evaporated and the filtrate was concentrated at reduced pressure. The residue was dissolved in ethanol and potassium hydroxide(>20 equiv.) dissolved in water was added and refluxed for 3 days. After that, ethyl acetate and sat. NaHCO3 were added and the organic layer was extracted. It was dried over MgSO₄, filtered, evaporated and the filtrate was concentrated at reduced pressure. The residue was purified by column chromatography eluting with ethyl acetate and methanol. : Yield 80%

1H-NMR(CDC13, 200MHz) δ2.4(m, 3H), 2.5(m, 3H), 2.7(m, 1H), 6.2(m, 1H), 7.2(m, 1H), 7.4(m, 1H), 7.5(s, 1H), 8.5(s, 1H)

### Example 3

### S-[3-(3,4-Dichloro-phenyl)-3-tetrazol-2-yl-propyl]-methyl-amine

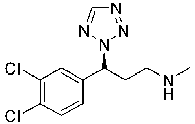

S-[3-(3,4-Dichloro-phenyl)-3-tetrazol-2-yl-propyl]-methyl-amine was obtained from the [3-(3,4-Dichloro-phenyl)-3-tetrazol-2-yl-propyl]-methyl-amine using a CHIRALPACK OD-H column(manufactured by Daicel Chemical Industries, Ltd.) as the Prep-LC column, at a column temperature of 25°C, with n-hexane/isopropylalcohol including 0.1% triethylamine(90:10) as the eluent.

1H-NMR(CDC13, 200MHz) δ2.4(m, 3H), 2.5(m, 3H), 2.7(m, 1H), 6.2(m, 1H), 7.2(m, 1H), 7.4(m, 1H), 7.5(s, 1H), 8.5(s, 1H)

### Example 4

### [3-Benzotriazol-2-yl-3-(3,4-dichloro-phenyl)-propyl]-dimethyl-amine

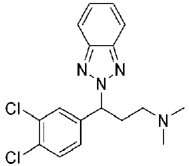

The procedure given in Example 1 was followed using benzotriazole as a reactant, instead of tetrazole, to give [3-Benzotriazol-2-yl-3-(3,4-dichloro-phenyl)-propyl]-dimethyl-amine.

1H-NMR(CDC13, 200MHz) δ2.2(s, 8H), 2.5(m, 1H), 2.8(m, 1H), 6.1(m, 1H), 7.4(m, 4H), 7.6(t, 1H), 7.9(m, 2H)

### Example 5

### Dimethyl-(3-naphthalen-2-yl-3-[1,2,3]triazol-2-yl-propyl)-amine

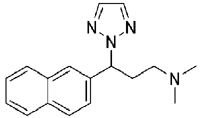

The procedure given in Example 1 was followed using 2-acetonaphthone and 1*H-*1,2,3-triazole as reactants, instead of 3,4-dichloroacetophenone and 1*H*-tetrazole, to give Dimethyl-(3-naphthalen-2-yl-3-[1,2,3]triazol-2-yl-propyl)-amine.

1H-NMR(CDC13, 200MHz) δ2.2(s, 8H), 2.5(m, 1H), 2.8(m, 1H), 6.1(m, 1H), 7.4(m, 3H), 7.6(d, 2H), 7.8(m, 5H)

### Example 6

### Dimethyl-(3-naphthalen-2-yl-3-tetrazol-2-yl-propyl)-amine

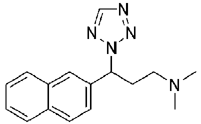

The procedure given in Example 1 was followed using 2-acetonaphthone as a reactant, instead of 3,4-dichloroacetophenone, to give Dimethyl-(3-naphthalen-2-yl-3-tetrazol-2-yl-propyl)-amine.

1H-NMR(CDC13, 200MHz) δ2.2(s, 8H), 2.5(m, 1H), 2.8(m, 1H), 6.1(m, 1H), 7.5(m, 3H), 7.8(m,4H), 8.5(s, 1H)

### Example 7

### (3-Benzotriazol-2-yl-3-naphthalen-2-yl-propyl)-dimethyl-amine

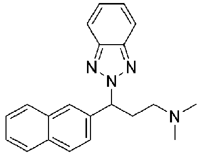

The procedure given in Example 1 was followed using 2-acetonaphthone and benzotriazole as reactants, instead of 3,4-dichloroacetophenone and 1*H*-tetrazole, to give (3-Benzotriazol-2-yl-3-naphthalen-2-yl-propyl)-dimethyl-amine.

1H-NMR(CDC13, 200MHz) δ2.2(s, 8H), 2.5(m, 1H), 2.8(m, 1H), 6.1(m, 1H), 7.4(m, 5H), 7.5(d, 1H), 7.8(m, 6H)

### Example 8

### Dimethyl-[3-(5-methyl-tetrazol-2-yl)-3-naphthalen-2-yl-propyl]-amine

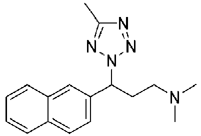

The procedure given in Example 1 was followed using 2-acetonaphthone and 5-methyl-1*H*-tetrazole as reactants, instead of 3,4-dichloroacetophenone and 1*H-*tetrazole, to give Dimethyl-[3-(5-methyl-tetrazol-2-yl)-3-naphthalen-2-yl-propyl]-amine.

1H-NMR(CDC13, 200MHz) δ2.2(s, 8H), 2.5(m, 1H), 2.8(m, 1H), 6.1(m, 4H), 7.5(d, 3H), 7.8(d, 4H)

### Example 9

### [3-(6-Chloro-naphthalen-2-yl)-3-tetrazol-1-yl-propyl]-dimethyl-amine

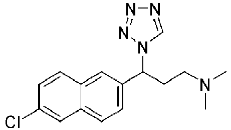

The procedure given in Example 1 was followed using 6-Chloro-2-acetonaphthone as a reactant, instead of 3,4-dichloroacetophenone, to give [3-(6-Chloro-naphthalen-2-yl)-3-tetrazol-1-yl-propyl]-dimethyl-amine.

1H-NMR(CDC13, 200MHz) δ2.2(s, 8H), 2.5(m, 1H), 2.8(m, 1H), 6.7(m, 1H), 7.5(m, 3H), 7.8(dd, 2H), 8.1(s, 1H), 8.6(s, 1H)

### Example 10

### Dimethyl-[3-(4-phenoxy-phenyl)-3-tetrazol-2-yl-propyl]-amine

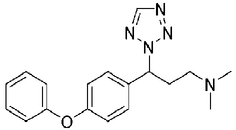

The procedure given in Example 1 was followed using 4-phenoxyacetophenone as a reactant, instead of 3,4-dichloroacetophenone, to give Dimethyl-[3-(4-phenoxy-phenyl)-3-tetrazol-2-yl-propyl]-amine.

1H-NMR(CDC13, 200MHz) δ2.2(s, 8H), 2.5(m, 1H), 2.8(m, 1H), 6.1(m,1H), 6.9(m, 4H), 7.1(m, 1H), 7.4(m, 4H), 8.5(s, 1H)

### Example 11

### Dimethyl-[3-(4-phenoxy-phenyl)-3-tetrazol-1-yl-propyl]-amine

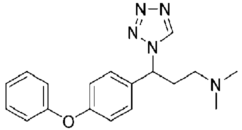

The procedure given in Example 1 was followed using 4-phenoxyacetophenone as a reactant, instead of 3,4-dichloroacetophenone, to give Dimethyl-[3-(4-phenoxy-phenyl)-3-tetrazol-1-yl-propyl]-amine.

1H-NMR(CDC13, 200MHz) δ2.2(s, 8H), 2.5(m, 1H), 2.8(m, 1H), 5.8(m,1H), 6.9(m, 4H), 7.1(m, 1H), 7.4(m, 4H), 8.5(s, 1H)

### Example 12

### [3-(6-Chloro-naphthalen-2-yl)-3-[1,2,3]triazol-2-yl-propyl]-dimethyl-amine

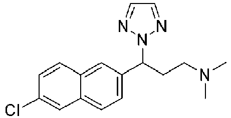

The procedure given in Example 1 was followed using 6-Chloro-2-acetonaphthone and 1*H*-1,2,3-triazole as reactants, instead of 3,4-dichloroacetophenone and 1*H-*tetrazole, to give [3-(6-Chloro-naphthalen-2-yl)-3-[1,2,3]triazol-2-yl-propyl]-dimethyl-amine.

1H-NMR(CDC13, 200MHz) δ2.2(s, 8H), 2.5(m, 1H), 2.8(m, 1H), 6.7(m,1H), 7.4(m, 3H), 7.6(m, 2H), 7.8(m, 2H), 8.4(s, 1H)

### Example 13

### [3-(6-Chloro-naphthalen-2-yl)-3-[1,2,3]triazol-1-yl-propyl]-dimethyl-amine

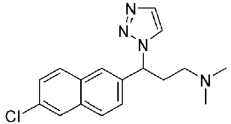

The procedure given in Example 1 was followed using 6-Chloro-2-acetonaphthone and 1*H*-1,2,3-triazole as reactants, instead of 3,4-dichloroacetophenone and *1H-*tetrazole, to give [3-(6-Chloro-naphthalen-2-yl)-3-[1,2,3]triazol-1-yl-propyl]-dimethyl-amine.

1H-NMR(CDC13, 200MHz) δ2.2(s, 8H), 2.5(m, 1H), 2.8(m, 1H), 6.7(m,1H), 7.4(m, 3H), 7.6(m, 2H), 7.8(m, 2H), 8.2(s, 1H)

### Example 14

### [3-(6-Chloro-naphthalen-2-yl)-3-tetrazol-2-yl-propyl]-dimethyl-amine

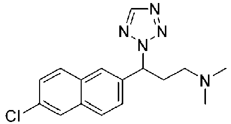

The procedure given in Example 1 was followed using 6-Chloro-2-acetonaphthone as a reactant, instead of 3,4-dichloroacetophenone, to give [3-(6-Chloro-naphthalen-2-yl)-3-tetrazol-2-yl-propyl]-dimethyl-amine.

1H-NMR(CDC13, 200MHz) δ2.2(s, 8H), 2.5(m, 1H), 2.8(m, 1H), 6.7(m, 1H), 7.5(m, 3H), 7.8(dd, 2H), 8.3(s, 1H), 8.5(s, 1H)

### Example 15

### [3-(4-Methoxy-phenyl)-3-[1,2,3]triazol-2-yl-propyl]-dimethyl-amine

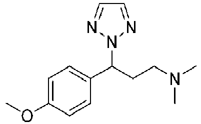

The procedure given in Example 1 was followed using 4-methoxyacetophenone and 1*H*-1,2,3-triazole as reactants, instead of 3,4-dichloroacetophenone and 1*H*-tetrazole, to give [3-(4-Methoxy-phenyl)-3-[1,2,3]triazol-2-yl-propyl]-dimethyl-amine.

1H-NMR(CDC13, 200MHz) δ2.2(s, 8H), 2.5(m, 2H), 2.8(m, 1H), 5.7(m, 1H), 6.8(dd, 2H), 7.3(dd, 2H), 7.6(s, 2H)

### Example 16

### (3-Biphenyl-3-yl-3-tetrazol-2-yl-propyl)-dimethyl-amine

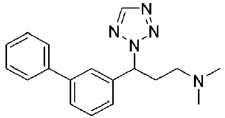

The procedure given in Example 1 was followed using 3-phenylacetophenone as a reactant, instead of 3,4-dichloroacetophenone, to give (3-Biphenyl-3-yl-3-tetrazol-2-yl-propyl)-dimethyl-amine.

1H-NMR(CDCl3, 200MHz) δ2.2(s, 8H), 2.5(m, 1H), 2.8(m, 1H), 6.2(m, 1H), 7.5(m, 9H), 8.5(s, 1H)

### Example 17

### [3-(4-Benzyloxy-phenyl)-3-tetrazol-2-yl-propyl]-dimethyl-amine

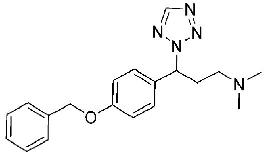

The procedure given in Example 1 was followed using 4-benzyloxyacetophenone as a reactant, instead of 3,4-dichloroacetophenone, to give [3-(4-Benzyloxy-phenyl)-3-tetrazol-2-yl-propyl]-dimethyl-amine.

1H-NMR(CDC13, 200MHz) δ2.2(s, 8H), 2.5(m, 1H), 2.8(m, 1H), 5.0(s, 2H), 6.1(m, 1H), 7.0(m, 2H), 7.4(m, 7H), 8.5(s, 1H)

### Example 18

### (3-Biphenyl-4-yl-3-tetrazol-2-yl-propyl)-dimethyl-amine

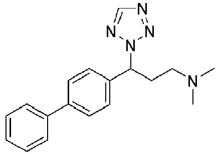

The procedure given in Example 1 was followed using 4-phenylacetophenone as a reactant, instead of 3,4-dichloroacetophenone, to give (3-Biphenyl-4-yl-3-tetrazol-2-yl-propyl)-dimethyl-amine.

1H-NMR(CDCl3, 200MHz) 62.2(s, 8H), 2.5(m, 1H), 2.8(m, 1H), 6.1(m, 1H), 7.4(m, 9H), 8.5(s, 1H)

### Example 19

### Dimethyl-[3-naphthalen-2-yl-3-(5-phenyl-tetrazol-2-yl)-propyl]-amine

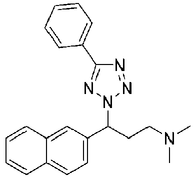

The procedure given in Example 1 was followed using 2-acetonaphthone and 5-Methyl-1*H*-tetrazole as reactants, instead of 3,4-dichloroacetophenone and 1*H-*tetrazole, to give Dimethyl-[3-naphthalen-2-yl-3-(5-phenyl-tetrazol-2-yl)-propyl]-amine.

1H-NMR(CDCl3, 200MHz) δ2.2(s, 8H), 2.5(m, 1H), 2.8(m, 1H), 6.1(m, 1H), 7.5(m, 5H), 7.6(m, 1H), 7.8(m, 3H), 8.0(s, 1H), 8.2(m, 2H)

### Example 20

### Dimethyl-[3-(6-methyl-naphthalen-2-yl)-3-tetrazol-2-yl-propyl]-amine

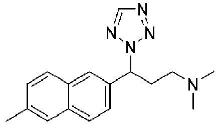

The procedure given in Example 1 was followed using 6-Methylacetonaphthone as a reactant, instead of 3,4-dichloroacetophenone, to give Dimethyl-[3-(6-methyl-naphthalen-2-yl)-3-(5-methyl-tetrazol-2-yl)-propyl]-amine.

1H-NMR(CDCl3, 200MHz) δ2.2(s, 8H), 2.5(m, 3H), 2.8(m, 1H), 6.1(m, 1H), 7.3(dd, 1H), 7.5(m, 2H), 7.7(dd, 2H), 7.9(s, 1H), 8.5(s, 1H)

### Example 21

### [3-(6-Methoxy-naphthalen-2-yl)-3-tetrazol-2-yl-propyl]-dimethyl-amine

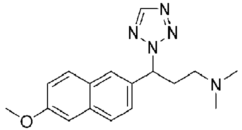

The procedure given in Example 1 was followed using 6-Methoxyacetonaphthone as a reactant, instead of 3,4-dichloroacetophenone, to give [3-(6-Methoxy-naphthalen-2-yl)-3-tetrazol-2-yl-propyl]-dimethyl-amine.

1H-NMR(CDCl3, 200MHz) δ2.2(s, 8H), 2.5(m, 1H), 2.8(m, 1H), 3.9(s, 3H), 6.1(m, 1H), 7.3(dd, 1H), 7.5(m, 2H), 7.7(dd, 2H), 7.9(s, 1H), 8.5(s, 1H)

### Example 22

### [3-(6-Methoxy-naphthalen-2-yl)-3-[1,2,3]triazol-2-yl-propyl]-dimethyl-amine

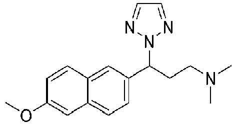

The procedure given in Example 1 was followed using 6-Methoxyacetonaphthone and 1*H*-1,2,3-triazole as reactants, instead of 3,4-dichloroacetophenone and 1*H-*tetrazole, to give [3-(6-Methoxy-naphthalen-2-yl)-3-[1,2,3]triazol-2-yl-propyl]-dimethyl-amine.

1H-NMR(CDCl3, 200MHz) δ2.2(s, 8H), 2.5(m, 1H), 2.8(m, 1H), 3.9(s, 3H), 6.1(m, 1H), 7.3(dd, 1H), 7.5(m, 2H), 7.7(dd, 2H), 7.9(s, 1H), 8.5(s, 2H)

### Example 23

### [3-Benzotriazol-1-yl-3-(3,4-dichloro-phenyl)-propyl]-dimethyl-amine

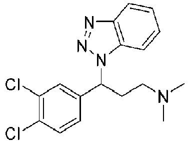

The procedure given in Example 1 was followed using benzotriazole as a reactant, instead of 1*H*-tetrazole, to give [3-Benzotriazol-1-yl-3-(3,4-dichloro-phenyl)-propyl]-dimethyl-amine.

1H-NMR(CDCl3, 200MHz) δ2.2(s, 8H), 2.5(m, 1H), 2.8(m, 1H), 6.0(m, 1H), 7.4(m, 6H), 8.0(t, 1H)

### Example 24

### 2-[1-(3,4-Dichloro-phenyl)-3-pyrrolidin-1-yl-propyl]-2H-[1,2,3]triazole

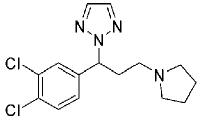

The procedure given in Example 1 was followed using pyrrolidine and 1*H-*1,2,3-triazole as reactants, instead of dimethylamine and 1*H*-tetrazole, to give 2-[1-(3,4-Dichloro-phenyl)-3-pyrrolidin-1-yl-propyl]-2H-[1,2,3]triazole.

1H-NMR(CDC13, 200MHz) δ1.84(m, 4H), 2.2(m, 7H), 2.7(m, 1H), 6.0(m, 1H), 7.2(m, 3H), 7.6(m, 2H)

### Example 25

### [3-(3,4-Dichloro-phenyl)-3-[1,2,3]triazol-2-yl-propyl]-diethyl-amine

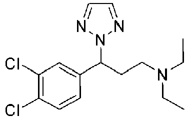

The procedure given in Example 1 was followed using diethylamine and 1*H-*1,2,3-triazole as reactants, instead of dimethylamine and 1*H*-tetrazole, to give [3-(3,4-Dichloro-phenyl)-3-[1,2,3]triazol-2-yl-propyl]-diethyl-amine.

1H-NMR(CDC13, 200MHz) δ1.0(m, 6H), 2.3(m, 7H), 2.6(m, 1H), 6.0(m, 1H), 7.2(m, 3H), 7.6(m, 2H)

### Example 26

### [3-(3,4-Dichloro-phenyl)-3-tetrazol-2-yl-propyl]-diethyl-amine

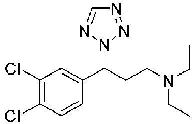

The procedure given in Example 1 was followed using diethylamine as a reactant, instead of dimethylamine, to give [3-(3,4-Dichloro-phenyl)-3-tetrazol-2-yl-propyl]-diethyl-amine.

1H-NMR(CDC13, 200MHz) δ1.0(m, 6H), 2.3(m, 7H), 2.6(m, 1H), 6.0(m, 1H), 7.2(dd, 1H), 7.4(dd, 1H), 7.6(t, 1H), 8.5(s, 1H)

### Example 27

### 2-[1-(3,4-Dichloro-phenyl)-3-pyrrolidin-1-yl-propyl]-2H-tetrazole

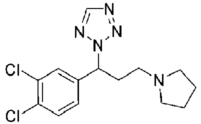

The procedure given in Example 1 was followed using pyrrolidine as a reactant, instead of dimethylamine, to give 2-[1-(3,4-Dichloro-phenyl)-3-pyrrolidin-1-yl-propyl]-2H-tetrazole.

1H-NMR(CDCl3, 200MHz) δ1.8(m, 4H), 2.4(m, 7H), 2.8(m, 1H), 6.1(m, 1H), 7.2(dd, 1H), 7.4(dd, 1H), 7.6(t, 1H), 8.5(s, 1H)

### Example 28

### Dimethyl-(3-naphthalen-1-yl-3-tetrazol-2-yl-propyl)-amine

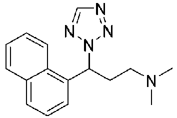

The procedure given in Example 1 was followed using 1-acetonaphthone as a reactant, instead of 3,4-dichloroacetophenone, to give Dimethyl-(3-naphthalen-1-yl-3-tetrazol-2-yl-propyl)-amine.

1H-NMR(CDCl3, 200MHz) δ2.2(m, 8H), 2.6(m, 1H), 2.9(m, 1H), 7.0(m, 1H), 7.5(m, 4H), 7.9(t, 2H), 8.3(d, 1H), 8.5(s, 1H)

### Example 29

### Dimethyl-(3-naphthalen-1-yl-3-[1,2,3]triazol-2-yl-propyl)-amine

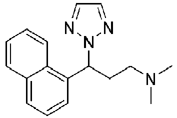

The procedure given in Example 1 was followed using 1-acetonaphthone and 1*H-*1,2,3-triazole as reactants, instead of 3,4-dichloroacetophenone and 1*H*-tetrazole, to give Dimethyl-(3-naphthalen-1-yl-3-[1,2,3]triazol-2-yl-propyl)-amine.

1H-NMR(CDCl3, 200MHz) δ2.2(m, 8H), 2.6(m, 1H), 2.9(m, 1H), 7.0(m, 1H), 7.5(m, 4H), 7.9(t, 2H), 8.3(d, 1H)

### Example 30

### [3-(3,4-Dichloro-phenyl)-3-(5-methyl-tetrazol-2-yl)-propyl]-dimethyl-amine

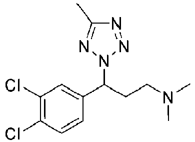

The procedure given in Example 1 was followed using 5-methyl-1*H*-tetrazole e as a reactant, instead of 1*H*-tetrazole, to give [3-(3,4-Dichloro-phenyl)-3-(5-methyl-tetrazol-2-yl)-propyl]-dimethyl-amine.

1H-NMR(CDC13, 200MHz) δ2.2(m, 9H), 2.6(m, 3H), 2.9(m, 3H), 6.0(t, 1H), 7.2(dd, 1H), 7.4(dd, 1H), 7.5(s, 1H)

### Example 31

### [3-(3,4-Dichloro-phenyl)-3-(5-phenyl-tetrazol-2-yl)-propyl]-dimethyl-amine

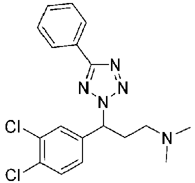

The procedure given in Example 1 was followed using 5-phenyl-1*H*-tetrazole as a reactant, instead of 1*H*-tetrazole, to give [3-(3,4-Dichloro-phenyl)-3-(5-phenyl-tetrazol-2-yl)-propyl]-dimethyl-amine.

1H-NMR(CDC13, 200MHz) δ2.2(m, 9H), 2.6(m, 3H), 2.9(m, 3H), 6.0(t, 1H), 7.4(m, 5H), 8.1(m, 2H)

### Example 32

### [3-(3,4-Difluoro-phenyl)-3-tetrazol-1-yl-propyl]-dimethyl-amine

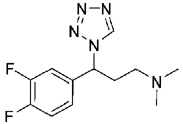

The procedure given in Example 1 was followed using 3,4-difluoroacetophenone as a reactant, instead of 3,4-dichloroacetophenone, to give [3-(3,4-Difluoro-phenyl)-3-tetrazol-1-yl-propyl]-dimethyl-amine.

1H-NMR(CDC13, 200MHz) δ2.2(m, 8H), 2.6(m, 2H), 2.9(m, 1H), 6.0(t, 1H), 7.4(m, 4H), 8.7(s, 1H)

### Example 33

### [3-(3,4-Difluoro-phenyl)-3-tetrazol-2-yl-propyl]-dimethyl-amine

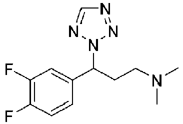

The procedure given in Example 1 was followed using 3,4-difluoroacetophenone as a reactant, instead of 3,4-dichloroacetophenone, to give [3-(3,4-Difluoro-phenyl)-3-tetrazol-2-yl-propyl]-dimethyl-amine.

1H-NMR(CDC13, 200MHz) δ2.2(m, 8H), 2.6(m, 2H), 2.9(m, 1H), 6.0(t, 1H), 7.4(m, 4H), 8.4(s, 1H)

### Example 34

### [3-(3,4-Difluoro-phenyl)-3-[1,2,3]triazol-1-yl-propyl]-dimethyl-amine

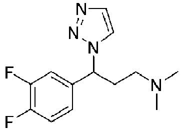

The procedure given in Example 1 was followed using 3,4-difluoroacetophenone and 1*H*-1,2,3-triazole as reactants, instead of 3,4-dichloroacetophenone and 1*H*-tetrazole, to give [3-(3,4-Difluoro-phenyl)-3-[1,2,3]triazol-1-yl-propyl]-dimethyl-amine.

1H-NMR(CDC13, 200MHz) δ2.2(m, 8H), 2.6(m, 2H), 2.9(m, 1H), 6.0(t, 1H), 7.4(m, 3H),7.5(s, 1H), 7.7(s, 1H)

### Example 35

### [3-(3,4-Difluoro-phenyl)-3-[1,2,3]triazol-2-yl-propyl]-dimethyl-amine

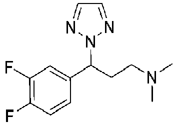

The procedure given in Example 1 was followed using 3,4-difluoroacetophenone and 1*H*-1,2,3-triazole as reactants, instead of 3,4-dichloroacetophenone and 1*H*-tetrazole, to give [3-(3,4-Difluoro-phenyl)-3-[1,2,3]triazol-2-yl-propyl]-dimethyl-amine.

1H-NMR(CDC13, 200MHz) δ2.2(m, 8H), 2.6(m, 2H), 2.9(m, 1H), 6.0(t, 1H), 7.4(m, 3H),7.6(s, 2H)

### Example 36

### [3-(3,4-Dimethyl-phenyl)-3-[1,2,3]triazol-1-yl-propyl]-dimethyl-amine

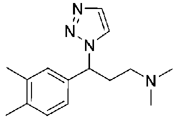

The procedure given in Example 1 was followed using 3,4-dimethylacetophenone and 1*H*-1,2,3-triazole as reactant, instead of 3,4-dichloroacetophenone, and 1*H-*tetrazole to give [3-(3,4-Dimethyl-phenyl)-3-[1,2,3]triazol-1-yl-propyl]-dimethyl-amine.

1H-NMR(CDC13, 200MHz) δ2.2(m, 14H), 2.6(m, 2H), 2.9(m, 2H), 6.0(t, 1H), 7.4(m, 3H), 7.5(s, 1H), 7.7(s, 1H)

### Example 37

### [3-(3,4-Dimethyl-phenyl)-3-[1,2,3]triazol-2-yl-propyl]-dimethyl-amine

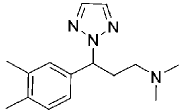

The procedure given in Example 1 was followed using 3,4-dimethylacetophenone and 1*H*-1,2,3-triazole as reactants, instead of 3,4-dichloroacetophenone and 1*H-*tetrazole, to give [3-(3,4-Dimethyl-phenyl)-3-[1,2,3]triazol-2-yl-propyl]-dimethyl-amine.

1H-NMR(CDC13, 200MHz) δ2.2(m, 14H), 2.6(m, 2H), 2.9(m, 2H), 6.0(t, 1H), 7.4(m, 3H), 7.5(t, 2H)

### Example 38

### [3-(3,4-Dimethyl-phenyl)-3-tetrazol-1-yl-propyl]-dimethyl-amine

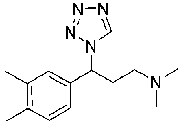

The procedure given in Example 1 was followed using 3,4-dimethylacetophenone as a reactant, instead of 3,4-dichloroacetophenone, to give [3-(3,4-Dimethyl-phenyl)-3-tetrazol-1-yl-propyl]-dimethyl-amine.

1H-NMR(CDC13, 200MHz) δ2.2(m, 14H), 2.6(m, 2H), 2.9(m, 2H), 6.0(t, 1H), 7.4(m, 3H), 8.5(s, 1H)

### Example 39

### [3-(3,4-Dimethyl-phenyl)-3-tetrazol-2-yl-propyl]-dimethyl-amine

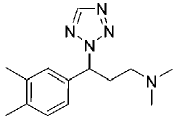

The procedure given in Example 1 was followed using 3,4-dimethylacetophenone as a reactant, instead of 3,4-dichloroacetophenone, to give [3-(3,4-Dimethyl-phenyl)-3-tetrazol-2-yl-propyl]-dimethyl-amine.

1H-NMR(CDC13, 200MHz) δ2.2(m, 14H), 2.6(m, 2H), 2.9(m, 2H), 6.0(t, 1H), 7.4(m, 3H), 8.3(s, 1H)

### Example 40

### [3-(4-Chloro-phenyl)-3-tetrazol-1-yl-propyl]-dimethyl-amine

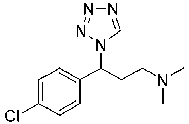

The procedure given in Example 1 was followed using 4-chloroacetophenone as a reactant, instead of 3,4-dichloroacetophenone, to give [3-(4-Chloro-phenyl)-3-tetrazol-1-yl-propyl]-dimethyl-amine.

1H-NMR(CDC13, 200MHz) δ2.2(m, 7H), 2.6(m, 2H), 2.9(m, 2H), 6.0(t, 1H), 7.4(m, 4H), 8.7(s, 1H)

### Example 41

### [3-(4-Chloro-phenyl)-3-tetrazol-2-yl-propyl]-dimethyl-amine

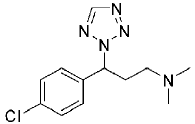

The procedure given in Example 1 was followed using 4-chloroacetophenone as a reactant, instead of 3,4-dichloroacetophenone, to give [3-(4-Chloro-phenyl)-3-tetrazol-2-yl-propyl]-dimethyl-amine.

1H-NMR(CDC13, 200MHz) δ2.2(m, 7H), 2.6(m, 2H), 2.9(m, 2H), 6.0(t, 1H), 7.4(m, 4H), 8.7(s, 1H)

### Example 42

### Dimethyl-(3-phenyl-3-[1,2,3]triazol-1-yl-propyl)-amine

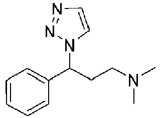

The procedure given in Example 1 was followed using acetophenone and 1*H-*1,2,3-triazole as reactants, instead of 3,4-dichloroacetophenone and 1*H*-tetrazole, to give Dimethyl-(3-phenyl-3-[1,2,3]triazol-1-yl-propyl)-amine.

1H-NMR(CDC13, 200MHz) δ2.2(m, 7H), 2.6(m, 2H), 2.9(m, 2H), 6.0(t, 1H), 7.2(m, 5H), 7.4(s, 1H), 7.6(s, 1H)

### Example 43

### Dimethyl-(3-phenyl-3-[1,2,3]triazol-2-yl-propyl)-amine

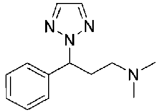

The procedure given in Example 1 was followed using acetophenone and 1*H-*1,2,3-triazole as reactants, instead of 3,4-dichloroacetophenone and 1*H*-tetrazole, to give Dimethyl-(3-phenyl-3-[1,2,3]triazol-2-yl-propyl)-amine.

1H-NMR(CDC13, 200MHz) δ2.2(m, 7H), 2.6(m, 2H), 6.0(t, 1H), 7.2(m, 5H), 7.4(s, 2H)

### Example 44

### [3-(4-Chloro-phenyl)-3-[1,2,3]triazol-1-yl-propyl]-dimethyl-amine

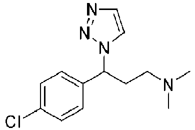

The procedure given in Example 1 was followed using 4-chloroacetophenone and 1*H* -1,2,3-triazole as reactant, instead of 3,4-dichloroacetophenone and 1*H*-tetrazole, to give [3-(4-Chloro-phenyl)-3-[1,2,3]triazol-1-yl-propyl]-dimethyl-amine.

1H-NMR(CDC13, 200MHz) δ2.2(m, 7H), 2.6(m, 2H), 6.0(t, 1H), 7.2(m, 4H), 7.5(s, 1H), 7.7(s, 1H)

### Example 45

### [3-(4-Chloro-phenyl)-3-[1,2,3]triazol-2-yl-propyl]-dimethyl-amine

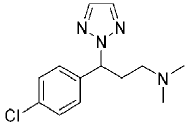

The procedure given in Example 1 was followed using 4-chloroacetophenone and 1*H* -1,2,3-triazole as reactants, instead of 3,4-dichloroacetophenone and 1*H*-tetrazole, to give [3-(4-Chloro-phenyl)-3-[1,2,3]triazol-2-yl-propyl]-dimethyl-amine.

1H-NMR(CDCl3, 200MHz) δ2.2(m, 7H), 2.6(m, 2H), 6.0(t, 1H), 7.2(m, 4H), 7.6(s, 2H)

### Example 46

### [3-(3,4-Dichloro-phenyl)-3-[1,2,3]triazol-2-yl-propyl]-dimethyl-amine

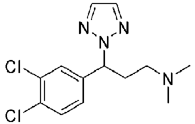

The procedure given in Example 1 was followed using 1*H*-1,2,3-triazole as a reactant, instead of 1*H*-tetrazole, to give [3-(3,4-Dichloro-phenyl)-3-[1,2,3]triazol-2-yl-propyl]-dimethyl-amine.

1H-NMR(CDC13, 200MHz) δ2.2(m, 7H), 2.4(m, 2H), 2.6(m, 2H), 6.0(t, 1H), 7.2(dd, 1H), 7.4(dd, 2H), 7.6(s, 2H)

### Example 47

### [3-(3,4-Dichloro-phenyl)-3-imidazol-1-yl-propyl]-dimethyl-amine

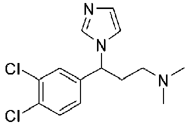

The procedure given in Example 1 was followed using imidazole as a reactant, instead of 1*H*-tetrazole, to give [3-(3,4-Dichloro-phenyl)-3-imidazol-1-yl-propyl]-dimethyl-amine.

1H-NMR(CDC13, 200MHz) δ2.2(br, 10H), 5.3(t, 1H), 7.2(dd, 1H), 7.4(dd, 2H), 7.6(s, 2H)

### Example 48

### [3-(3-Methoxy-naphthalen-2-yl)-3-tetrazol-2-yl-propyl]-dimethyl-amine

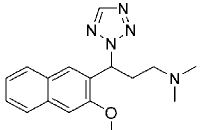

The procedure given in Example 1 was followed using 1-(3-Methoxy-naphthalen-2-yl)-ethanone as reactants, instead of 3,4-dichloroacetophenone, to give [3-(3-Methoxy-naphthalen-2-yl)-3-tetrazol-2-yl-propyl]-dimethyl-amine.

1H-NMR(CDCl3, 200MHz) δ2.4(m, 13H), 6.2(m, 1H), 7.5(m, 3H), 7.8(m, 4H), 8.5(s, 1H)

### Example 49

### [3-(3-Methoxy-naphthalen-2-yl)-3-(5-methyl-tetrazol-2-yl)-propyl]-dimethyl-ami ne

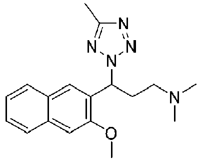

The procedure given in Example 1 was followed using 1-(3-Methoxy-naphthalen-2-yl)-ethanone and 5-Methyl-1*H*-tetrazole as reactants, instead of 3,4-dichloroacetophenone and 1*H*-tetrazole, to give [3-(3-Methoxy-naphthalen-2-yl)-3-(5-methyl-tetrazol-2-yl)-propyl]-dimethyl-amine.

1H-NMR(CDC13, 200MHz) δ2.4(m, 9H), 2.5(m, 5H), 2.8(m, 1H), 4.0(s, 3H), 6.6(m, 1H), 7.5(m, 3H), 7.8(m, 4H)

### Example 50

### [3-(6-Fluoro-naphthalen-2-yl)-3-tetrazol-2-yl-propyl]-dimethyl-amine

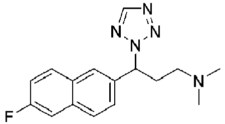

The procedure given in Example 1 was followed using 1-(6-Fluoro-naphthalen-2-yl)-ethanone as reactants, instead of 3,4-dichloroacetophenone, to give [3-(6-Fluoro-naphthalen-2-yl)-3-tetrazol-2-yl-propyl]-dimethyl-amine.

1H-NMR(CDCl3, 200MHz) δ2.4(m, 12H), 2.5(m, 1H), 2.8(m, 1H), 6.2(m, 1H), 7.2(m, 1H), 7.4(m, 1H), 7.5(m, 1H), 7.8(m, 3H), 8.5(s, 1H)

### Example 51

### [3-(6-Fluoro-naphthalen-2-yl)-3-(5-methyl-tetrazol-2-yl)-propyl]-dimethyl-amin e

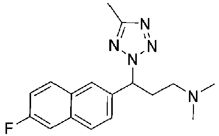

The procedure given in Example 1 was followed using 1-(6-Fluoro-naphthalen-2-yl)-ethanone and 5-Me-1*H*-tetrazole as reactants, instead of 3,4-dichloroacetophenone and 1*H*-tetrazole, to give [3-(6-Fluoro-naphthalen-2-yl)-3-(5-methyl-tetrazol-2-yl)-propyl]-dimethyl-amine.

1H-NMR(CDCl3, 200MHz) δ2.4(m, 9H), 2.5(m, 4H), 2.8(m, 1H), 6.2(t, 1H), 7.2(m, 1H), 7.4(m, 1H), 7.5(m, 1H), 7.8(m, 3H)

### Example 52

### [3-(1,4-Dimethoxy-naphthalen-2-yl)-3-tetrazol-2-yl-propyl]-dimethyl-amine

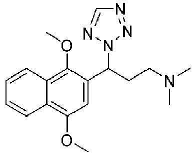

The procedure given in Example 1 was followed using 1-(1,4-Dimethoxy-naphthalen-2-yl)-ethanone as reactants, instead of 3,4-dichloroacetophenone, to give [3-(1,4-Dimethoxy-naphthalen-2-yl)-3-tetrazol-2-yl-propyl]-dimethyl-amine.

1H-NMR(CDC13, 200MHz) δ2.4(m, 12H), 2.5(m, 1H), 2.8(m, 1H), 4.0(d, 6H), 6.2(m, 1H), 7.2(m, 1H), 7.4(m, 1H), 7.5(m, 1H), 7.8(m, 2H), 8.5(s, 1H)

### Example 53

### [3-(1,4-Dimethoxy-naphthalen-2-yl)-3-(5-methyl-tetrazol-2-yl)-propyl]-dimethyl-amine

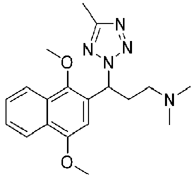

The procedure given in Example 1 was followed using 1-(1,4-Dimethoxy-naphthalen-2-yl)-ethanone and 5-Me-1*H*-tetrazole as reactants, instead of 3,4-dichloroacetophenone and 1*H*-tetrazole, to give [3-(1,4-Dimethoxy-naphthalen-2-yl)-3-(5-methyl-tetrazol-2-yl)-propyl]-dimethyl-amin e.

1H-NMR(CDCl3, 200MHz) δ2.4(m, 12H), 2.5(m, 1H), 2.8(m, 1H), 4.0(d, 6H), 6.2(m, 1H), 6.9(s, 1H), 7.5(m, 2H), 8.0(s, 1H), 8.2(d, 1H)

### Example 54

### [3-(3,5-Dimethoxy-naphthalen-2-yl)-3-tetrazol-2-yl-propyl]-dimethyl-amine

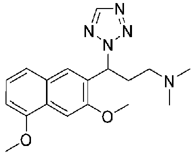

The procedure given in Example 1 was followed using 1-(3,5-Dimethoxy-naphthalen-2-yl)-ethanone as reactants, instead of 3,4-dichloroacetophenone, to give [3-(3,5-Dimethoxy-naphthalen-2-yl)-3-tetrazol-2-yl-propyl]-dimethyl-amine.

1H-NMR(CDCl3, 200MHz) δ2.4(m, 12H), 2.5(m, 1H), 2.8(m, 1H), 4.0(m, 6H), 6.8(m, 1H), 7.3(s, 6H), 7.5(m, 2H), 8.5(s, 1H)

### Example 55

### [3-(3,5-Dimethoxy-naphthalen-2-yl)-3-(5-methyl-tetrazol-2-yl)-propyl]-dimethyl-amine

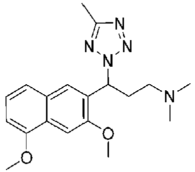

The procedure given in Example 1 was followed using 1-(3,5-Dimethoxy-naphthalen-2-yl)-ethanone and 5-Me-1*H*-tetrazole as reactants, instead of 3,4-dichloroacetophenone and 1*H*-tetrazole, to give [3-(3,5-Dimethoxy-naphthalen-2-yl)-3-(5-methyl-tetrazol-2-yl)-propyl]-dimethyl-amin e.

1H-NMR(CDC13, 200MHz) δ2.4(m, 9H), 2.5(m, 4H), 2.8(m, 1H), 4.0(m, 6H), 6.6(m, 1H), 6.8(m, 1H), 7.2(s, 3H), 7.5(s, 1H), 7.7(s, 1H)

### Example 56

### [3-(3,5-Dimethoxy-naphthalen-2-yl)-3-tetrazol-1-yl-propyl]-dimethyl-amine

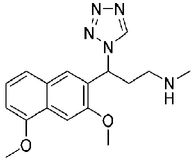

The procedure given in Example 1 was followed using 1-(3,5-Dimethoxy-naphthalen-2-yl)-ethanone as reactants, instead of 3,4-dichloroacetophenone, to give [3-(3,5-Dimethoxy-naphthalen-2-yl)-3-tetrazol-1-yl-propyl]-dimethyl-amine.

1H-NMR(CDCl3, 200MHz) δ2.4(m, 12H), 2.5(m, 1H), 2.8(m, 1H), 4.0(m, 6H), 6.6(m, 1H), 6.8(m, 1H), 7.2(s, 3H), 7.5(s, 1H), 7.7(s, 1H), 8.7(s, 1H)

### Example 57

### Dimethyl-[3-(4-methyl-naphthalen-1-yl)-3-tetrazol-2-yl-propyl]-amine

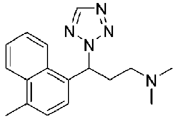

The procedure given in Example 1 was followed using 1-(4-Methyl-naphthalen-1-yl)-ethanone as reactants, instead of 3,4-dichloroacetophenone, to give Dimethyl-[3-(4-methyl-naphthalen-1-yl)-3-tetrazol-2-yl-propyl]-amine.

1H-NMR(CDC13, 200MHz) δ2.2(m, 10H), 2.4(m, 1H), 2.8(m, 4H), 3.0(m, 1H), 7.0(m, 1H), 7.2(m, 1H), 7.5(m, 4H), 8.0(m, 1H), 8.3(m, 1H), 8.5(s, 1H)

### Example 58

### Dimethyl-[3-(4-methyl-naphthalen-1-yl)-3-(5-methyl-tetrazol-2-yl)-propyl]-amin e

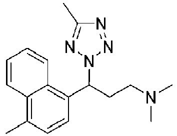

The procedure given in Example 1 was followed using 1-(4-Methyl-naphthalen-1-yl)-ethanone and 5-Me-1*H*-tetrazole as reactants, instead of 3,4-dichloroacetophenone and 1*H*-tetrazole, to give Dimethyl-[3-(4-methyl-naphthalen-1-yl)-3-(5-methyl-tetrazol-2-yl)-propyl]-amine.

1H-NMR(CDC13, 200MHz) δ2.2(m, 9H), 2.4(m, 3H), 2.8(m, 3H), 3.0(m, 1H), 7.0(m, 1H), 7.2(m, 1H), 7.5(m, 4H), 8.0(m, 1H), 8.3(m, 1H)

### Example 59

### [3-(4-Fluoro-naphthalen-1-yl)-3-tetrazol-2-yl-propyl]-dimethyl-amine

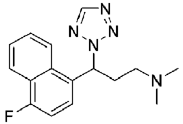

The procedure given in Example 1 was followed using 1-(4-Fluoro-naphthalen-1-yl)-ethanone as reactants, instead of 3,4-dichloroacetophenone, to give [3-(4-Fluoro-naphthalen-1-yl)-3-tetrazol-2-yl-propyl]-dimethyl-amine.

1H-NMR(CDC13, 200MHz) δ2.2(m, 12H), 2.4(m, 1H), 2.8(m, 1H), 7.0(m, 1H), 7.2(m, 1H), 7.5(m, 4H), 8.0(m, 1H), 8.3(m, 1H), 8.5(s, 1H)

### Example 60

### [3-(4-Fluoro-naphthalen-1-yl)-3-(5-methyl-tetrazol-2-yl)-propyl]-dimethyl-amin e

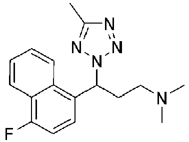

The procedure given in Example 1 was followed using 1-(4-Methyl-naphthalen-1-yl)-ethanone and 5-Me-1*H*-tetrazole as reactants, instead of 3,4-dichloroacetophenone and 1*H*-tetrazole, to give [3-(4-Fluoro-naphthalen-1-yl)-3-(5-methyl-tetrazol-2-yl)-propyl]-dimethyl-amine.

1H-NMR(CDC13, 200MHz) δ2.2(m, 9H), 2.4(m, 4H), 2.8(m, 1H), 7.0(m, 1H), 7.2(m, 1H), 7.5(m, 3H), 8.1(m, 1H), 8.3(m, 1H)

### Example 61

### (3-Isoquinolin-1-yl-3-tetrazol-2-yl-propyl)-dimethyl-amine

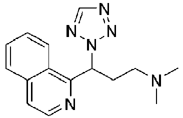

The procedure given in Example 1 was followed using 1-Isoquinolin-1-yl-ethanone as reactants, instead of 3,4-dichloroacetophenone, to give (3-Isoquinolin-1-yl-3-tetrazol-2-yl-propyl)-dimethyl-amine.

1H-NMR(CDC13, 200MHz) δ2.2(m, 9H), 2.4(m, 1H), 2.8(m, 1H), 7.2(m, 1H), 7.6(m, 2H), 7.8(m, 2H), 8.4(m, 1H), 8.5(m, 1H), 9.1(s, 1H)

### Example 62

### [3-Isoquinolin-1-yl-3-(5-methyl-tetrazol-2-yl)-propyl]-dimethyl-amine

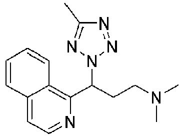

The procedure given in Example 1 was followed using 1-Isoquinolin-1-yl-ethanone and 5-Me-1*H*-tetrazole as reactants, instead of 3,4-dichloroacetophenone and 1*H-*tetrazole, to give [3-Isoquinolin-1-yl-3-(5-methyl-tetrazol-2-yl)-propyl]-dimethyl-amine.

1H-NMR(CDC13, 200MHz) δ2.2(m, 9H), 2.5(m, 3H), 2.8(m, 3H), 7.2(m, 1H), 7.6(m, 2H), 7.8(m, 2H), 8.4(m, 1H), 8.5(m, 1H)

### Example 63

### (3-Benzo[1,3]dioxol-5-yl-3-tetrazol-2-yl-propyl)-dimethyl-amine

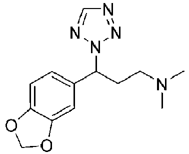

The procedure given in Example 1 was followed using 1-Benzo[1,3]dioxol-5-yl-ethanone as reactants, instead of 3,4-dichloroacetophenone, to give (3-Benzo[1,3]dioxol-5-yl-3-tetrazol-2-yl-propyl)-dimethyl-amine.

1H-NMR(CDC13, 200MHz) 52.2(m, 9H), 2.5(m, 1H), 2.8(m, 1H), 6.0(s, 2H), 6.1(m, 1H), 6.8(d, 1H), 7.0(d, 2H), 8.5(m, 1H)

### Example 64

### [3-Benzo[1,3]dioxol-5-yl-3-(5-methyl-tetrazol-2-yl)-propyl]-dimethyl-amine

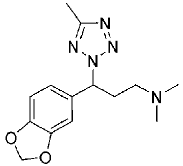

The procedure given in Example 1 was followed using l-Benzo[1,3]dioxol-5-yl-ethanone and 5-Me-1*H*-tetrazole as reactants, instead of 3,4-dichloroacetophenone and 1*H*-tetrazole, to give [3-Benzo[1,3]dioxol-5-yl-3-(5-methyl-tetrazol-2-yl)-propyl]-dimethyl-amine.

1H-NMR(CDC13, 200MHz) 62.2(m, 15H), 2.8(m, 1H), 6.0(s, 2H), 6.1(m, 1H), 6.8(d, 1H), 7.0(d, 2H), 8.5(m, 1H)

### Example 65

### [3-Benzo[1,3]dioxol-5-yl-3-(5-methyl-tetrazol-1-yl)-propyl]-dimethyl-amine

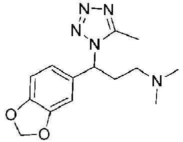

The procedure given in Example 1 was followed using 1-Benzo[1,3]dioxol-5-yl-ethanone and 5-Me-1*H*-tetrazole as reactants, instead of 3,4-dichloroacetophenone and 1*H*-tetrazole, to give [3-Benzo[1,3]dioxol-5-yl-3-(5-methyl-tetrazol-1-yl)-propyl]-dimethyl-amine.

1H-NMR(CDC13, 200MHz) δ2.2(m, 9H), 2.4(m, 1H), 2.5(s, 3H), 2.8(m, 1H), 5.5(t, 2H), 6.1(s, 2H), 6.8(m, 3H)

### Example 66

### [3-(3,4-Dimethoxy-phenyl)-3-tetrazol-2-yl-propyl]-dimethyl-amine

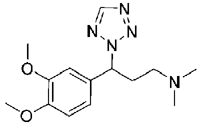

The procedure given in Example 1 was followed using 1-(3,4-Dimethoxy-phenyl)-ethanone as reactants, instead of 3,4-dichloroacetophenone, to give [3-(3,4-Dimethoxy-phenyl)-3-tetrazol-2-yl-propyl]-dimethyl-amine.

1H-NMR(CDC13, 200MHz) δ2.2(s, 12H), 2.4(m, 1H), 2.8(m, 1H), 4.0(s, 6H), 6.1(s, 2H), 6.8(d, 1H), 7.0(d, 2H), 8.5(s, 1H)

### Example 67

### [3-(2,3-Dihydro-benzo[1,4]dioxin-2-yl)-3-tetrazol-2-yl-propyl]-dimethyl-amine

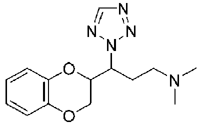

The procedure given in Example 1 was followed using 1-(2,3-Dihydro-benzo[1,4]dioxin-2-yl)-ethanone as reactants, instead of 3,4-dichloroacetophenone, to give [3-(2,3-Dihydro-benzo[1,4]dioxin-2-yl)-3-tetrazol-2-yl-propyl]-dimethyl-amine.

1H-NMR(CDC13, 200MHz) δ2.2(s, 12H), 2.4(m, 3H), 3.8(m, 1H), 4.6(m, 1H), 5.4(m, 1H), 7.0(s, 5H), 8.5(s, 1H)

### Example 68

### [3-(2,3-Dihydro-benzo[1,4]dioxin-2-yl)-3-(5-methyl-tetrazol-2-yl)-propyl]-dimet hyl-amine

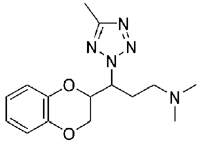

The procedure given in Example 1 was followed using 1-(2,3-Dihydro-benzo[1,4]dioxin-2-yl)-ethanone and 5-Me-1*H*-tetrazole as reactants, instead of 3,4-dichloroacetophenone and 1*H*-tetrazole, to give [3-(2,3-Dihydro-benzo[1,4]dioxin-2-yl)-3-(5-methyl-tetrazol-2-yl)-propyl]-dimethyl-a mine.

1H-NMR(CDC13, 200MHz) δ2.2(s, 10H), 2.4(m, 3H), 2.8(m, 4H), 3.8(m, 1H), 4.0(m, 1H), 4.8(m, 1H), 5.3(m, 1H), 7.0(s, 4H)

### Example 69

### Dimethyl-(3-quinolin-2-yl-3-tetrazol-2-yl-propyl)-amine

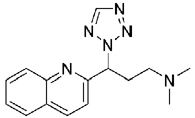

The procedure given in Example 1 was followed using 1-Quinolin-2-yl-ethanone as reactants, instead of 3,4-dichloroacetophenone, to give Dimethyl-(3-quinolin-2-yl-3-tetrazol-2-yl-propyl)-amine.

1H-NMR(CDC13, 200MHz) δ2.2(s, 10H), 2.8(m, 2H), 6.5(t, 1H), 7.3(m, 2H), 7.5(m, 1H), 7.8(m, 2H), 8.0(t, 2H), 8.5(s, 1H)

### Example 70

### Dimethyl-[3-(5-methyl-tetrazol-2-yl)-3-quinolin-2-yl-propyl]-amine

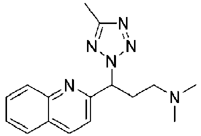

The procedure given in Example 1 was followed using 1-Quinolin-2-yl-ethanone and 5-Me-1*H*-tetrazole as reactants, instead of 3,4-dichloroacetophenone and 1*H*-tetrazole, to give Dimethyl-[3-(5-methyl-tetrazol-2-yl)-3-quinolin-2-yl-propyl]-amine.

1H-NMR(CDC13, 200MHz) δ2.2(s, 10H), 2.5(s, 3H), 2.8(m, 2H), 6.5(t, 1H), 7.3(m, 2H), 7.5(m, 1H), 7.8(m, 2H), 8.0(t, 2H), 8.5(s, 1H)

### Example 71

### Dimethyl-[3-(1-methyl-1H-indol-2-yl)-3-tetrazol-2-yl-propyl]-amine

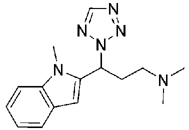

The procedure given in Example 1 was followed using 1-(1-Methyl-1H-indol-2-yl)-ethanone as reactants, instead of 3,4-dichloroacetophenone, to give Dimethyl-[3-(1-methyl-1H-indol-2-yl)-3-tetrazol-2-yl-propyl]-amine.

1H-NMR(CDC13, 200MHz) δ2.2(s, 10H), 2.5(s, 3H), 2.8(m, 2H), 6.5(t, 1H), 7.3(m, 2H), 7.5(m, 1H), 7.8(m, 2H), 8.0(t, 2H), 8.5(s, 1H)

### Example 72

### Dimethyl-[3-(1-methyl-1H-indol-2-yl)-3-(5-methyl-tetrazol-2-yl)-propyl]-amine

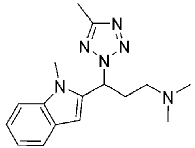

The procedure given in Example 1 was followed using 1-(1-Methyl-1H-indol-2-yl)-ethanone and 5-Me-1*H*-tetrazole as reactants, instead of 3,4-dichloroacetophenone and 1*H*-tetrazole, to give Dimethyl-[3-(1-methyl-1H-indol-2-yl)-3-(5-methyl-tetrazol-2-yl)-propyl]-amine.

1H-NMR(CDC13, 200MHz) δ2.2(s, 8H), 2.5(s, 3H), 2.8(m, 2H), 3.8(s, 2H), 6.5(t, 1H), 6.8(s, 1H), 7.3(m, 1H), 7.5(m, 2H), 7.8(m, 2H)

### Example 73

### Dimethyl-[3-(1,2,3,4-tetrahydro-naphthalen-2-yl)-3-tetrazol-2-yl-propyl]-amine

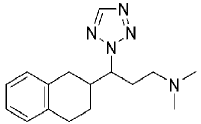

The procedure given in Example 1 was followed using 1-(1,2,3,4-Tetrahydro-naphthalen-2-yl)-ethanone as reactants, instead of 3,4-dichloroacetophenone, to give Dimethyl-[3-(1,2,3,4-tetrahydro-naphthalen-2-yl)-3-tetrazol-2-yl-propyl]-amine.

1H-NMR(CDC13, 200MHz) δ2.2(s, 11H), 2.8(m, 8H), 5.0(m, 1H), 7.0(m, 4H), 8.5(s, 1H)

### Example 74

### [3-(6-Chloro-pyridin-3-yl)-3-tetrazol-2-yl-propyl]-dimethyl-amine

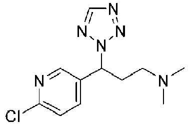

The procedure given in Example 1 was followed using 1-(6-Chloro-pyridin-3-yl)-ethanone as reactants, instead of 3,4-dichloroacetophenone, to give [3-(6-Chloro-pyridin-3-yl)-3-tetrazol-2-yl-propyl]-dimethyl-amine.

1H-NMR(CDC13, 200MHz) δ2.2(m, 10H), 2.5(m, 1H), 2.8(m, 1H), 6.2(m, 1H), 7.2(m, 1H), 7.8(m, 1H), 8.5(s, 1H)

### Example 75

### (3-Benzo[b]thiophen-2-yl-3-tetrazol-2-yl-propyl)-dimethyl-amine

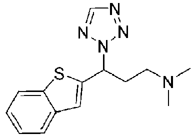

The procedure given in Example 1 was followed using 1-Benzo[b]thiophen-2-yl-ethanone as reactants, instead of 3,4-dichloroacetophenone, to give (3-Benzo[b]thiophen-2-yl-3-tetrazol-2-yl-propyl)-dimethyl-amine.

1H-NMR(CDC13, 200MHz) δ2.2(m, 10H), 2.8(m, 1H), 6.5(m, 1H), 7.4(m, 3H), 7.8(m, 2H), 8.5(s, 1H)

### Example 76

### [3-Benzo[b]thiophen-2-yl-3-(5-methyl-tetrazol-2-yl)-propyl]-dimethyl-amine

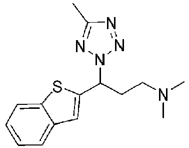

The procedure given in Example 1 was followed using 1-Benzo[b]thiophen-2-yl-ethanone and 5-Me-1*H*-tetrazole as reactants, instead of 3,4-dichloroacetophenone and 1*H*-tetrazole, to give [3-Benzo[b]thiophen-2-yl-3-(5-methyl-tetrazol-2-yl)-propyl]-dimethyl-amine.

1H-NMR(CDC13, 200MHz) δ2.2(m, 9H), 2.8(m, 5H), 6.5(m, 1H), 7.4(m, 3H), 7.8(m, 2H)

### Example 77

### (3-Benzofuran-2-yl-3-tetrazol-2-yl-propyl)-dimethyl-amine

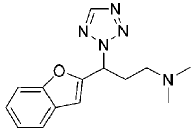

The procedure given in Example 1 was followed using 1-Benzofuran-2-yl-ethanone as reactants, instead of 3,4-dichloroacetophenone, to give (3-Benzofuran-2-yl-3-tetrazol-2-yl-propyl)-dimethyl-amine.

1H-NMR(CDC13, 200MHz) δ2.2(m, 10H), 2.8(m, 2H), 6.5(m, 1H), 7.4(m, 3H), 7.8(m, 2H), 8.5(s, 1H)

### Example 78

### [3-Benzofuran-2-yl-3-(5-methyl-tetrazol-2-yl)-propyl]-dimethyl-amine

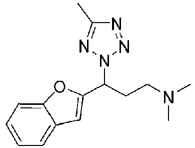

The procedure given in Example 1 was followed using 1-Benzofuran-2-yl-ethanone and 5-Me-1*H*-tetrazole as reactants, instead of 3,4-dichloroacetophenone and 1*H-*tetrazole, to give [3-Benzofuran-2-yl-3-(5-methyl-tetrazol-2-yl)-propyl]-dimethyl-amine.

1H-NMR(CDC13, 200MHz) δ2.2(m, 10H), 2.8(m, 2H), 6.5(m, 1H), 7.4(m, 3H), 7.8(m, 2H)

### Example 79

### (3-Benzofuran-2-yl-3-tetrazol-1-yl-propyl)-dimethyl-amine

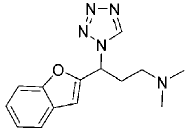

The procedure given in Example 1 was followed using 1-Benzofuran-2-yl-ethanone as reactants, instead of 3,4-dichloroacetophenone, to give (3-Benzofuran-2-yl-3-tetrazol-1-yl-propyl)-dimethyl-amine.

1H-NMR(CDC13, 200MHz) δ2.2(m, 10H), 2.8(m, 2H), 6.2(m, 1H), 7.4(m, 3H), 7.8(m, 2H)

### Example 80

### [3-(3,4-Difluoro-phenyl)-3-(5-methyl-tetrazol-2-yl)-propyl]-dimethyl-amine

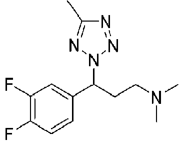

The procedure given in Example 1 was followed using 1-Benzofuran-2-yl-ethanone and 5-Me-1*H*-tetrazole as reactants, instead of 3,4-dichloroacetophenone and 1*H-*tetrazole, to give [3-(3,4-Difluoro-phenyl)-3-(5-methyl-tetrazol-2-yl)-propyl]-dimethyl-amine.

1H-NMR(CDC13, 200MHz) δ2.2(m, 10H), 2.8(m, 4H), 6.2(m, 1H), 7.4(m, 4H)

### Example 81

### Dimethyl-[3-tetrazol-2-yl-3-(2,4,5-trifluoro-phenyl)-propyl]-amine

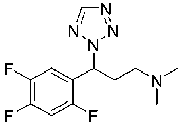

The procedure given in Example 1 was followed using 1-(2,4,5-Trifluoro-phenyl)-ethanone as reactants, instead of 3,4-dichloroacetophenone, to give Dimethyl-[3-tetrazol-2-yl-3-(2,4,5-trifluoro-phenyl)-propyl]-amine.

1H-NMR(CDCl3, 200MHz) δ2.2(m, 12H), 2.8(m, 2H), 6.5(m, 1H), 7.0(m, 1H), 7.4(m, 1H), 8.6(s, 1H)

### Example 82

### Dimethyl-[3-(5-methyl-tetrazol-2-yl)-3-(2,4,5-trifluoro-phenyl)-propyl]-amine

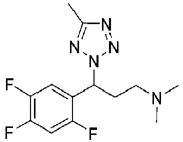

The procedure given in Example 1 was followed using 1-(2,4,5-Trifluoro-phenyl)-ethanone and 5-Me-1*H*-tetrazole as reactants, instead of 3,4-dichloroacetophenone and 1*H*-tetrazole, to give Dimethyl-[3-(5-methyl-tetrazol-2-yl)-3-(2,4,5-trifluoro-phenyl)-propyl]-amine.

1H-NMR(CDC13, 200MHz) δ2.2(m, 12H), 2.8(m, 4H), 6.5(m, 1H), 7.0(m, 1H), 7.4(m, 1H)

### Example 83

### [3-(3-Bromo-4-fluoro-phenyl)-3-tetrazol-2-yl-propyl]-dimethyl-amine

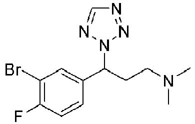

The procedure given in Example 1 was followed using 1-(3-Bromo-4-fluoro-phenyl)-ethanone as reactants, instead of 3,4-dichloroacetophenone, to give [3-(3-Bromo-4-fluoro-phenyl)-3-tetrazol-2-yl-propyl]-dimethyl-amine.

1H-NMR(CDC13, 200MHz) δ2.2(m, 10H), 2.4(m, 2H), 2.8(m, 1H), 6.5(m, 1H), 7.0(m, 1H), 7.4(m, 1H), 7.8(m, 1H), 8.5(s, 1H)

### Example 84

### [3-(3-Bromo-4-fluoro-phenyl)-3-(5-methyl-tetrazol-2-yl)-propyl]-dimethyl-amine

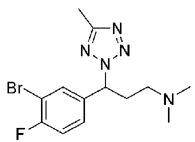

The procedure given in Example 1 was followed using 1-(3-Bromo-4-fluoro-phenyl)-ethanone and 5-Me-1*H*-tetrazole as reactants, instead of 3,4-dichloroacetophenone and 1*H*-tetrazole, to give [3-(3-Bromo-4-fluoro-phenyl)-3-(5-methyl-tetrazol-2-yl)-propyl]-dimethyl-amine.

1H-NMR(CDC13, 200MHz) δ2.2(m, 10H), 2.4(m, 2H), 2.8(m, 1H), 6.5(m, 1H), 7.0(m, 1H), 7.4(m, 1H), 7.8(m, 1H)

### Example 85

### [3-(6-Methoxy-naphthalen-2-yl)-3-(5-methyl-tetrazol-2-yl)-propyl]-dimethyl-ami ne

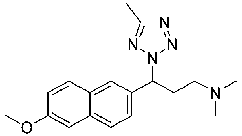

The procedure given in Example 1 was followed using 1-(6-Methoxy-naphthalen-2-yl)-ethanone and 5-Me-1*H*-tetrazole as reactants, instead of 3,4-dichloroacetophenone and 1*H*-tetrazole, to give [3-(6-Methoxy-naphthalen-2-yl)-3-(5-methyl-tetrazol-2-yl)-propyl]-dimethyl-amine.

1H-NMR(CDC13, 200MHz) 62.2(m, 11H), 2.4(m, 2H), 2.8(m, 1H), 4.0(s, 3H), 6.3(m, 1H), 7.1(m, 3H), 7.4(m, 1H), 7.8(m, 1H)

### Example 86

### Dimethyl-[3-(5-methyl-tetrazol-2-yl)-3-(4-phenoxy-phenyl)-propyl]-amine

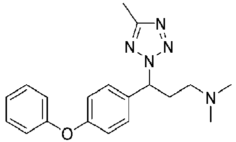

The procedure given in Example 1 was followed using 1-(4-Phenoxy-phenyl)-ethanone and 5-Me-1*H*-tetrazole as reactants, instead of 3,4-dichloroacetophenone and 1*H*-tetrazole, to give Dimethyl-[3-(5-methyl-tetrazol-2-yl)-3-(4-phenoxy-phenyl)-propyl]-amine.

1H-NMR(CDC13, 200MHz) δ2.2(m, 8H), 2.4(m, 6H), 2.8(m, 1H), 6.2(m, 1H), 7.1(m, 4H), 7.4(m, 4H)

### Example 87

### [3-(4-Bromo-phenyl)-3-tetrazol-2-yl-propyl]-dimethyl-amine

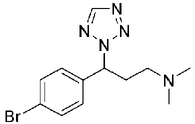

The procedure given in Example 1 was followed using 1-(4-Bromo-phenyl)-ethanone as reactants, instead of 3,4-dichloroacetophenone, to give [3-(4-Bromo-phenyl)-3-tetrazol-2-yl-propyl]-dimethyl-amine.

1H-NMR(CDC13, 200MHz) δ2.2(m, 10H), 2.4(m, 1H), 2.8(m, 1H), 6.2(m, 1H), 7.3(m, 2H), 7.5(m, 2H), 8.5(s, 1H)

### Example 88

### [3-(4-Bromo-phenyl)-3-(5-methyl-tetrazol-2-yl)-propyl]-dimethyl-amine

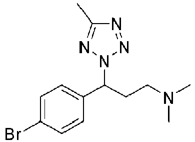

The procedure given in Example 1 was followed using 1-(4-Bromo-phenyl)-ethanone and 5-Me-1*H*-tetrazole as reactants, instead of 3,4-dichloroacetophenone and 1*H -* tetrazole, to give [3-(4-Bromo-phenyl)-3-(5-methyl-tetrazol-2-yl)-propyl]-dimethyl-amine.

1H-NMR(CDC13, 200MHz) 52.2(m, 9H), 2.3(m, 1H), 2.5(s, 3H), 2.8(m, 1H), 6.0(m, 1H), 7.3(m, 2H), 7.5(m, 2H)

### Example 89

### Dimethyl-[3-tetrazol-2-yl-3-(4-trifluoromethyl-phenyl)-propyl]-amine

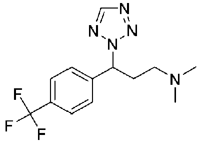

The procedure given in Example 1 was followed using 1-(4-Trifluoromethyl-phenyl)-ethanone as reactants, instead of 3,4-dichloroacetophenone, to give Dimethyl-[3-tetrazol-2-yl-3-(4-trifluoromethyl-phenyl)-propyl]-amine.

1H-NMR(CDC13, 200MHz) 62.2(m, 9H), 2.3(m, 1H), 2.8(m, 1H), 6.2(m, 1H), 7.3(m, 2H), 7.5(m, 2H), 8.5(s, 1H)

### Example 90

### Dimethyl-[3-(5-methyl-tetrazol-2-yl)-3-(4-trifluoromethyl-phenyl)-propyl]-amin e

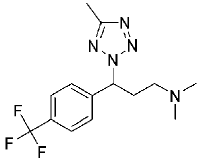

The procedure given in Example 1 was followed using 1-(4-Trifluoromethyl-phenyl)-ethanone and 5-Me-1*H*-tetrazole as reactants, instead of 3,4-dichloroacetophenone and 1*H*-tetrazole, to give Dimethyl-[3-(5-methyl-tetrazol-2-yl)-3-(4-trifluoromethyl-phenyl)-propyl]-amine.

1H-NMR(CDC13, 200MHz) δ2.2(m, 9H), 2.3(m, 1H), 2.5(s, 3H), 2.8(m, 1H), 6.0(m, 1H), 7.6(m, 4H)

### Example 91

### Dimethyl-[3-tetrazol-2-yl-3-(2,3,4-trichloro-phenyl)-propyl]-amine

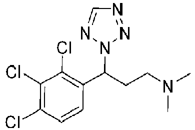

The procedure given in Example 1 was followed using 1-(2,3,4-Trichloro-phenyl)-ethanone as reactants, instead of 3,4-dichloroacetophenone, to give Dimethyl-[3-tetrazol-2-yl-3-(2,3,4-trichloro-phenyl)-propyl]-amine.

1H-NMR(CDC13, 200MHz) 62.2(m, 9H), 2.3(m, 1H), 2.8(m, 1H), 6.2(m, 1H), 7.3(m, 3H), 8.5(s, 1H)

### Example 92

### Dimethyl-[3-(5-methyl-tetrazol-2-yl)-3-(2,3,4-trichloro-phenyl)-propyl]-amine

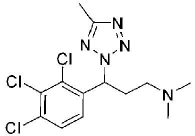

The procedure given in Example 1 was followed using 1-(2,3,4-Trichloro-phenyl)-ethanone and 5-Me-1*H*-tetrazole as reactants, instead of 3,4-dichloroacetophenone and 1*H*-tetrazole, to give Dimethyl-[3-(5-methyl-tetrazol-2-yl)-3-(2,3,4-trichloro-phenyl)-propyl]-amine.

1H-NMR(CDC13, 200MHz) δ2.2(m, 9H), 2.3(m, 1H), 2.5(s, 3H), 2.8(m, 1H), 6.0(m, 1H), 7.2(m, 2H)

### Example 93

### [3-(3-Chloro-4-methoxy-phenyl)-3-tetrazol-2-yl-propyl]-dimethyl-amine

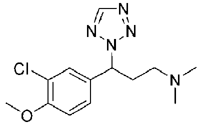

The procedure given in Example 1 was followed using 1-(3-Chloro-4-methoxy-phenyl)-ethanone as reactants, instead of 3,4-dichloroacetophenone, to give [3-(3-Chloro-4-methoxy-phenyl)-3-tetrazol-2-yl-propyl]-dimethyl-amine.

1H-NMR(CDC13, 200MHz) δ2.2(m, 10H), 2.3(m, 1H), 2.8(m, 1H), 4.0(s, 3H), 6.1(m, 1H), 6.9(d, 1H), 7.3(d, 1H), 7.5(s, 1H), 8.5(s, 1H)

### Example 94

### [3-(3-Chloro-4-methoxy-phenyl)-3-(5-methyl-tetrazol-2-yl)-propyl]-dimethyl-am ine

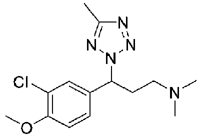

The procedure given in Example 1 was followed using 1-(3-Chloro-4-methoxy-phenyl)-ethanone and 5-Me-1*H*-tetrazole as reactants, instead of 3,4-dichloroacetophenone and 1*H*-tetrazole, to give [3-(3-Chloro-4-methoxy-phenyl)-3-(5-methyl-tetrazol-2-yl)-propyl]-dimethyl-amine.

1H-NMR(CDC13, 200MHz) δ2.2(m, 10H), 2.3(m, 1H), 2.8(m, 1H), 4.0(s, 3H), 6.1(m, 1H), 6.9(d, 1H), 7.3(d, 1H), 7.5(s, 1H)

### Example 95

### [3-(4-tert-Butyl-phenyl)-3-(5-methyl-tetrazol-2-yl)-propyl]-dimethyl-amine

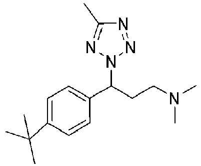

The procedure given in Example 1 was followed using 1-(4-tert-Butyl-phenyl)-ethanone and 5-Me-1*H*-tetrazole as reactants, instead of 3,4-dichloroacetophenone and 1*H*-tetrazole, to give [3-(4-tert-Butyl-phenyl)-3-(5-methyl-tetrazol-2-yl)-propyl]-dimethyl-amine.

1H-NMR(CDC13, 200MHz) δ1.2(s, 9H), 2.4(m, 3H), 2.5(m, 6H), 2.7(m, 1H), 6.2(m, 1H), 7.4(m, 4H)

### Example 96

### [3-(2,5-Difluoro-phenyl)-3-(5-methyl-tetrazol-2-yl)-propyl]-dimethyl-amine

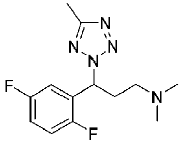

The procedure given in Example 1 was followed using 1-(2,5-Difluoro-phenyl)-ethanone and 5-Me-1*H*-tetrazole as reactants, instead of 3,4-dichloroacetophenone and 1*H*-tetrazole, to give [3-(2,5-Difluoro-phenyl)-3-(5-methyl-tetrazol-2-yl)-propyl]-dimethyl-amine.

1H-NMR(CDC13, 200MHz) δ2.4(m, 10H), 2.5(m, 3H), 2.7(m, 1H), 6.4(m, 1H), 7.4(m, 3H)

### Example 97

### [3-(2,4-Dichloro-phenyl)-3-(5-methyl-tetrazol-2-yl)-propyl]-dimethyl-amine

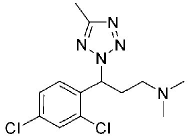

The procedure given in Example 1 was followed using 1-(2,4-Dichloro-phenyl)-ethanone and 5-Me-1*H*-tetrazole as reactants, instead of 3,4-dichloroacetophenone and 1*H*-tetrazole, to give [3-(2,4-Dichloro-phenyl)-3-(5-methyl-tetrazol-2-yl)-propyl]-dimethyl-amine.

1H-NMR(CDC13, 200MHz) δ2.4(m, 10H), 2.5(m, 3H), 2.7(m, 1H), 6.4(m, 1H), 7.4(m, 3H)

### Example 98

### [3-(3-Chloro-phenyl)-3-(5-methyl-tetrazol-2-yl)-propyl]-dimethyl-amine

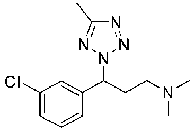

The procedure given in Example 1 was followed using 1-(3-Chloro-phenyl)-ethanone and 5-Me-1*H*-tetrazole as reactants, instead of 3,4-dichloroacetophenone and 1*H-*tetrazole, to give [3-(3-Chloro-phenyl)-3-(5-methyl-tetrazol-2-yl)-propyl]-dimethyl-amine.

1H-NMR(CDC13, 200MHz) δ2.4(m, 10H), 2.5(m, 3H), 2.7(m, 1H), 6.4(m, 1H), 7.4(m, 4H)

### Example 99

### [3-(2-Chloro-phenyl)-3-(5-methyl-tetrazol-2-yl)-propyl]-dimethyl-amine

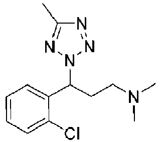

The procedure given in Example 1 was followed using 1-(2-Chloro-phenyl)-ethanone and 5-Me-1*H*-tetrazole as reactants, instead of 3,4-dichloroacetophenone and 1*H-*tetrazole, to give [3-(2-Chloro-phenyl)-3-(5-methyl-tetrazol-2-yl)-propyl]-dimethyl-amine.

1H-NMR(CDC13, 200MHz) δ2.4(m, 10H), 2.5(m, 3H), 2.7(m, 1H), 6.4(m, 1H), 7.4(m, 4H)

### Example 100

### [3-(2,5-Difluoro-phenyl)-3-tetrazol-2-yl-propyl]-dimethyl-amine

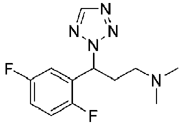

The procedure given in Example 1 was followed using 1-(2,5-Difluoro-phenyl)-ethanone as reactants, instead of 3,4-dichloroacetophenone, to give [3-(2-5-difluoro-phenyl)-3-tetrazol-2-yl-propyl]-dimethyl-amine.

1H-NMR(CDC13, 200MHz) δ2.4(m, 10H), 2.5(m, 1H), 2.7(m, 1H), 6.4(m, 1H), 7.4(m, 3H), 8.5(s, 1H)

### Example 101

### [3-(2,4-Dichloro-phenyl)-3-tetrazol-2-yl-propyl]-dimethyl-amine

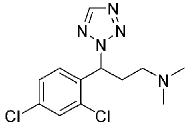

The procedure given in Example 1 was followed using 1-(2,4-Dichloro-phenyl)-ethanone as reactants, instead of 3,4-dichloroacetophenone, to give [3-(2,4-dichloro-phenyl)-3-tetrazol-2-yl-propyl]-dimethyl-amine.

1H-NMR(CDC13, 200MHz) δ2.4(m, 10H), 2.5(m, 1H), 2.7(m, 1H), 6.4(m, 1H), 7.4(m, 3H), 8.5(s, 1H)

### Example 102

### [3-(3-Chloro-phenyl)-3-tetrazol-2-yl-propyl]-dimethyl-amine

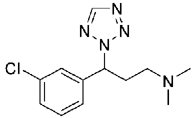

The procedure given in Example 1 was followed using 1-(3-Chloro-phenyl)-ethanone as reactants, instead of 3,4-dichloroacetophenone, to give [3-(3-Chloro-phenyl)-3-tetrazol-2-yl-propyl]-dimethyl-amine.

1H-NMR(CDC13, 200MHz) δ2.4(m, 10H), 2.5(m, 1H), 2.7(m, 1H), 6.4(m, 1H), 7.4(m, 4H), 8.5(s, 1H)

### Example 103

### Dimethyl-[3-(5-methyl-tetrazol-2-yl)-3-(4-trifluoromethoxy-phenyl)-propyl]-ami ne

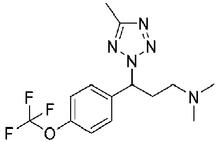

The procedure given in Example 1 was followed using 1-(4-Trifluoromethoxy-phenyl)-ethanone and 5-Me-1*H*-tetrazole as reactants, instead of 3,4-dichloroacetophenone and 1*H*-tetrazole, to give Dimethyl-[3-(5-methyl-tetrazol-2-yl)-3-(4-trifluoromethoxy-phenyl)-propyl]-amine.

1H-NMR(CDC13, 200MHz) δ2.4(m, 10H), 2.5(m, 3H), 2.7(m, 1H), 6.4(m, 1H), 7.2(m, 2H),7.4(m, 2H)

### Example 104

### [3-(3-Bromo-4-methyl-phenyl)-3-(5-methyl-tetrazol-2-yl)-propyl]-dimethyl-amin e

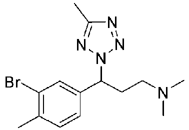

The procedure given in Example 1 was followed using 1-(3-Bromo-4-methyl-phenyl)-ethanone and 5-Me-1*H*-tetrazole as reactants, instead of 3,4-dichloroacetophenone and 1*H*-tetrazole, to give [3-(3-Bromo-4-methyl-phenyl)-3-(5-methyl-tetrazol-2-yl)-propyl]-dimethyl-amine.

1H-NMR(CDC13, 200MHz) δ2.4(m, 10H), 2.5(m, 3H), 2.7(m, 1H), 6.4(m, 1H), 7.4(m, 3H)

### Example 105

### Dimethyl-[3-tetrazol-2-yl-3-(4-trifluoromethoxy-phenyl)-propyl]-amine

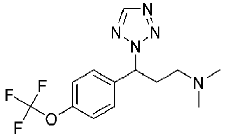

The procedure given in Example 1 was followed using 1-(4-Trifluoromethoxy-phenyl)-ethanone as reactants, instead of 3,4-dichloroacetophenone, to give Dimethyl-[3-tetrazol-2-yl-3-(4-trifluoromethoxy-phenyl)-propyl]-amine.

1H-NMR(CDC13, 200MHz) δ2.4(m, 10H), 2.5(m, 1H), 2.7(m, 1H), 6.4(m, 1H), 7.2(m, 2H),7.4(m, 2H), 8.5(s, 1H)

### Example 106

### [3-(4-Bromo-3-methyl-phenyl)-3-tetrazol-2-yl-propyl]-dimethyl-amine

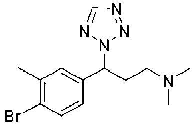

The procedure given in Example 1 was followed using 1-(3-Bromo-4-methyl-phenyl)-ethanone as reactants, instead of 3,4-dichloroacetophenone, to give [3-(4-Bromo-3-methyl-phenyl)-3-tetrazol-2-yl-propyl]-dimethyl-amine.

1H-NMR(CDC13, 200MHz) δ2.4(m, 10H), 2.5(m, 1H), 2.7(m, 1H), 6.4(m, 1H), 7.4(m, 3H), 8.5(s, 1H)

### Example 107

### [3-(4-Methanesulfonyl-phenyl)-3-(5-methyl-tetrazol-2-yl)-propyl]-dimethyl-ami ne

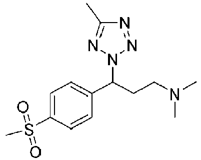

The procedure given in Example 1 was followed using 1-(4-Methanesulfonyl-phenyl)-ethanone and 5-Me-1*H*-tetrazole as reactants, instead of 3,4-dichloroacetophenone and 1*H*-tetrazole, to give [3-(4-Methanesulfonyl-phenyl)-3-(5-methyl-tetrazol-2-yl)-propyl]-dimethyl-amine.

1H-NMR(CDC13, 200MHz) δ2.4(m, 10H), 2.5(m, 3H), 2.7(m, 1H), 3.0(m, 3H), 6.4(m, 1H), 7.2(m, 2H),7.4(m, 2H)

### Example 108

### [3-(4-Isopropyl-phenyl)-3-(5-methyl-tetrazol-2-yl)-propyl]-dimethyl-amine

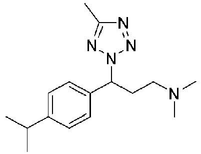

The procedure given in Example 1 was followed using 1-(4-Isopropyl-phenyl)-ethanone and 5-Me-1*H*-tetrazole as reactants, instead of 3,4-dichloroacetophenone and 1*H*-tetrazole, to give [3-(4-Isopropyl-phenyl)-3-(5-methyl-tetrazol-2-yl)-propyl]-dimethyl-amine.

1H-NMR(CDC13, 200MHz) δ1.0(s, 6H), 2.4(m, 10H), 2.7(m, 1H), 3.0(m, 3H), 6.4(m, 1H), 7.2(m, 2H),7.4(m, 2H)

### Example 109

### [3-(9H-Fluoren-2-yl)-3-(5-methyl-tetrazol-2-yl)-propyl]-dimethyl-amine

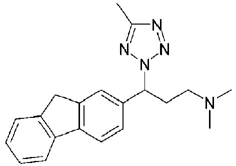

The procedure given in Example 1 was followed using 1-(9H-Fluoren-2-yl)-ethanone and 5-Me-1*H*-tetrazole as reactants, instead of 3,4-dichloroacetophenone and 1*H* - tetrazole, to give [3-(9H-Fluoren-2-yl)-3-(5-methyl-tetrazol-2-yl)-propyl]-dimethyl-amine.

1H-NMR(CDC13, 200MHz) δ2.4(m, 10H), 2.5(m, 3H), 2.7(m, 1H), 4.0(m, 3H), 6.4(m, 1H), 7.2(m, 2H),7.4(m, 2H)

### Example 110

### Methyl-(3-naphthalen-2-yl-3-[1,2,3]triazol-2-yl-propyl)-amine

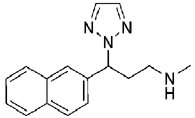

The procedure given in Example 2 was followed using dimethyl-(3-naphthalen-2-yl-3-[1,2,3]triazol-2-yl-propyl)-amine as a reactant, instead of [3-(3,4-Dichloro-phenyl)-3-tetrazol-2-yl-propyl]-dimethyl-amine, to give Methyl-(3-naphthalen-2-yl-3-[1,2,3]triazol-2-yl-propyl)-amine.

1H-NMR(CDC13, 200MHz) δ2.4(m, 3H), 2.5(m, 6H), 2.7(m, 1H), 6.2(m, 1H), 7.2(m, 3H), 7.6(d, 2H), 7.8(m, 4H)

### Example 111

### [3-Benzotriazol-2-yl-3-(3,4-dichloro-phenyl)-propyl]-methyl-amine

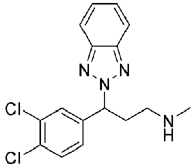

The procedure given in Example 2 was followed using [3-benzotriazol-2-yl-3-(3,4-dichloro-phenyl)-propyl]-dimethyl-amineas a reactant, instead of [3-(3,4-Dichloro-phenyl)-3-tetrazol-2-yl-propyl]-dimethyl-amine, to give [3-Benzotriazol-2-yl-3-(3,4-dichloro-phenyl)-propyl]-methyl-amine.

1H-NMR(CDC13, 200MHz) δ2.4(m, 3H), 2.5(m, 3H), 2.7(m, 1H), 6.2(m, 1H), 7.2(m, 4H), 7.6(s, 1H), 7.9(m, 2H)

### Example 112

### Methyl-[3-(5-methyl-tetrazol-2-yl)-3-naphthalen-2-yl-propyl]-amine

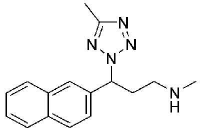

The procedure given in Example 2 was followed using [dimethyl-[3-(5-methyl-tetrazol-2-yl)-3-naphthalen-2-yl-propyl]-amine a reactant, instead of [3-(3,4-dichloro-phenyl)-3-tetrazol-2-yl-propyl]-dimethyl-amine, to give methyl-[3-(5-methyl-tetrazol-2-yl)-3-naphthalen-2-yl-propyl]-amine.

1H-NMR(CDC13, 200MHz) δ2.4(s, 1H), 2.6(m, 6H), 2.8(m, 1H), 6.2(m, 1H), 7.5(m, 3H), 7.8(m, 4H)

### Example 113

### (3-Benzotriazol-2-yl-3-naphthalen-2-yl-propyl)-methyl-amine

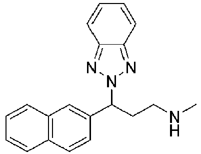

The procedure given in Example 2 was followed using (3-benzotriazol-2-yl-3-naphthalen-2-yl-propyl)-dimethyl-amine as a reactant, instead of [3-(3,4-dichloro-phenyl)-3-tetrazol-2-yl-propyl]-dimethyl-amine, to give (3-benzotriazol-2-yl-3-naphthalen-2-yl-propyl)-methyl-amine.

1H-NMR(CDC13, 200MHz) δ2.4(s, 1H), 2.6(m, 6H), 2.8(m, 1H), 6.2(m, 1H), 7.4(m, 4H), 7.6(d, 1H), 7.9(m, 6H)

### Example 114

### (3-Biphenyl-4-yl-3-tetrazol-2-yl-propyl)-methyl-amine

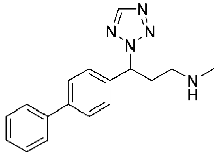

The procedure given in Example 2 was followed using (3-biphenyl-4-yl-3-tetrazol-2-yl-propyl)-dimethyl-amine as a reactant, instead of [3-(3,4-dichloro-phenyl)-3-tetrazol-2-yl-propyl]-dimethyl-amine, to give (3-Biphenyl-4-yl-3-tetrazol-2-yl-propyl)-methyl-amine.

1H-NMR(CDC13, 200MHz) δ2.4(s, 1H), 2.6(m, 6H), 2.8(m, 1H), 6.2(m, 1H), 7.4(m 9H), 8.5(s, 1H)

### Example 115

### [3-(3,4-Dichloro-phenyl)-3-(5-methyl-tetrazol-2-yl)-propyl]-methyl-amine

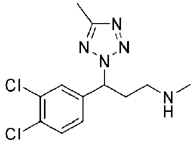

The procedure given in Example 2 was followed using [3-(3,4-dichloro-phenyl)-3-(5-methyl-tetrazol-2-yl)-propyl]-dimethyl-amine as a reactant, instead of [3-(3,4-dichloro-phenyl)-3-tetrazol-2-yl-propyl]-dimethyl-amine, to give [3-(3,4-dichloro-phenyl)-3-(5-methyl-tetrazol-2-yl)-propyl]-methyl-amine.

1H-NMR(CDC13, 200MHz) δ2.4(m, 3H), 2.5(m, 6H), 2.7(m, 1H), 6.2(m, 1H), 7.2(m, 1H), 7.4(m, 1H), 7.5(s, 1H)

### Example 116

### [3-(4-Chloro-phenyl)-3-tetrazol-2-yl-propyl]-methyl-amine

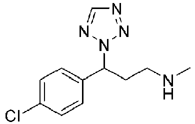

The procedure given in Example 2 was followed using [3-(4-chloro-phenyl)-3-tetrazol-2-yl-propyl]-dimethyl-amine as a reactant, instead of [3-(3,4-dichloro-phenyl)-3-tetrazol-2-yl-propyl]-dimethyl-amine, to give [3-(4-chloro-phenyl)-3-tetrazol-2-yl-propyl]-methyl-amine.

1H-NMR(CDC13, 200MHz) δ2.4(m, 3H), 2.5(m, 6H), 2.7(m, 1H), 6.2(m, 1H), 7.3(m, 4H), 8.5(s, 1H)

### Example 117

### [3-(3,4-Dichloro-phenyl)-3-[1,2,3]triazol-2-yl-propyl]-methyl-amine

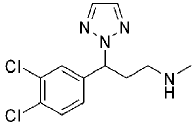

The procedure given in Example 2 was followed using [3-(3,4-dichloro-phenyl)-3-[1,2,3]triazol-2-yl-propyl]-dimethyl-amine as a reactant, instead of [3-(3,4-dichloro-phenyl)-3-tetrazol-2-yl-propyl]-dimethyl-amine, to give [3-(3,4-dichloro-phenyl)-3-[1,2,3]triazol-2-yl-propyl]-methyl-amine.

1H-NMR(CDC13, 200MHz) δ2.4(m, 3H), 2.5(m, 3H), 2.7(m, 1H), 6.2(m, 1H), 7.2(m, 1H), 7.4(m, 1H), 7.5(s, 1H), 7.6(d, 2H)

### Example 118

### [3-(3,4-Dichloro-phenyl)-3-tetrazol-2-yl-propyl]-methyl-amine

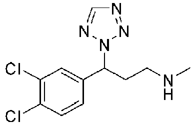

The procedure given in Example 2 was followed using [3-(3,4-dichloro-phenyl)-3-tetrazol-2-yl-propyl]-dimethyl-amine as a reactant, instead of [3-(3,4-dichloro-phenyl)-3-tetrazol-2-yl-propyl]-dimethyl-amine, to give [3-(3,4-dichloro-phenyl)-3-tetrazol-2-yl-propyl]-methyl-amine.

1H-NMR(CDC13, 200MHz) δ2.4(m, 3H), 2.5(m, 3H), 2.7(m, 1H), 6.2(m, 1H), 7.2(m, 1H), 7.4(m, 1H), 7.5(s, 1H), 8.5(s, 1H)

### Example 119

### Methyl-(3-naphthalen-2-yl-3-tetrazol-2-yl-propyl)-amine

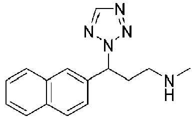

The procedure given in Example 2 was followed using dimethyl-(3-naphthalen-2-yl-3-tetrazol-2-yl-propyl)-amine as a reactant, instead of [3-(3,4-dichloro-phenyl)-3-tetrazol-2-yl-propyl]-dimethyl-amine, to give methyl-(3-naphthalen-2-yl-3-tetrazol-2-yl-propyl)-amine.

1H-NMR(CDC13, 200MHz) δ2.4(m, 3H), 2.5(m, 3H), 2.7(m, 1H), 6.2(m, 1H), 7.5(m, 3H), 7.8(m, 4H), 8.5(s, 1H)

### Example 120

### [3-(6-Chloro-naphthalen-2-yl)-3-tetrazol-2-yl-propyl]-methyl-amine

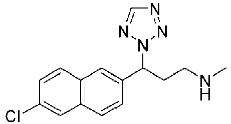

The procedure given in Example 2 was followed using [3-(6-chloro-naphthalen-2-yl)-3-tetrazol-2-yl-propyl]-dimethyl-amine as a reactant, instead of [3-(3,4-dichloro-phenyl)-3-tetrazol-2-yl-propyl]-dimethyl-amine, to give [3-(6-Chloro-naphthalen-2-yl)-3-tetrazol-2-yl-propyl]-methyl-amine.

1H-NMR(CDC13, 200MHz) δ2.4(m, 3H), 2.5(m, 3H), 2.7(m, 1H), 7.0(m, 1H), 7.5(m, 3H), 7.8(m, 2H), 8.3(s, 1H), 8.5(s, 1H)

### Example 121

### Methyl-(3-naphthalen-1-yl-3-tetrazol-2-yl-propyl)-amine

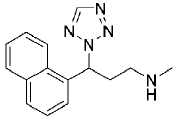

The procedure given in Example 2 was followed using Dimethyl-(3-naphthalen-1-yl-3-tetrazol-2-yl-propyl)-amine as a reactant, instead of [3-(3,4-dichloro-phenyl)-3-tetrazol-2-yl-propyl]-dimethyl-amine, to give methyl-(3-naphthalen-1-yl-3-tetrazol-2-yl-propyl)-amine.

1H-NMR(CDC13, 200MHz) δ2.4(m, 3H), 2.5(m, 3H), 2.7(m, 1H), 7.5(m, 4H), 7.9(t, 2H), 8.3(d, 1H), 8.5(s, 1H)

### Example 122

### Methyl-[3-(6-methyl-naphthalen-2-yl)-3-tetrazol-2-yl-propyl]-amine

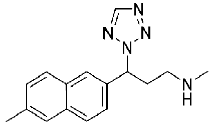

The procedure given in Example 2 was followed using Dimethyl-[3-(6-methyl-naphthalen-2-yl)-3-tetrazol-2-yl-propyl]-amine as a reactant, instead of [3-(3,4-dichloro-phenyl)-3-tetrazol-2-yl-propyl]-dimethyl-amine, to give methyl-[3-(6-methyl-naphthalen-2-yl)-3-tetrazol-2-yl-propyl]-amine.

1H-NMR(CDC13, 200MHz) δ2.4(s, 3H), 2.5(m, 6H), 2.8(m, 1H), 6.3(m,1H), 7.3(dd, 1H), 7.5(m, 2H), 7.7(dd, 2H), 7.8(s, 1H), 8.5(s, 1H)

### Example 123

### Methyl-[3-naphthalen-2-yl-3-(5-phenyl-tetrazol-2-yl)-propyl]-amine

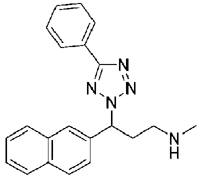

The procedure given in Example 2 was followed using Dimethyl-[3-naphthalen-2-yl-3-(5-phenyl-tetrazol-2-yl)-propyl]-amine as a reactant, instead of [3-(3,4-dichloro-phenyl)-3-tetrazol-2-yl-propyl]-dimethyl-amine, to give methyl-[3-naphthalen-2-yl-3-(5-phenyl-tetrazol-2-yl)-propyl]-amine.

1H-NMR(CDC13, 200MHz) δ2.4(m, 3H), 2.5(m, 3H), 2.7(m, 1H), 6.3(m, 1H), 7.5(m, 5H), 7.6(dd, 1H), 7.8(m, 3H), 7.9(s, 1H), 8.2(m, 2H)

### Example 124

### (3,S)-3-(3,4-dichlorophenyl)-N-methyl-3-(2H-tetrazol-2-yl)propan-1-amine

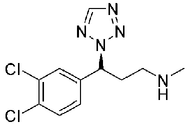

The procedure given in Example 3 was followed using [3-(3,4-Dichloro-phenyl)-3-tetrazol-2-yl-propyl]-methyl-amine, instead of [3-(3,4-Dichloro-phenyl)-3-tetrazol-2-yl-propyl]-methyl-amine, to give (3S)-3-(3,4-Dichloro-phenyl)-3-tetrazol-2-yl-propyl]-methyl-amine.

1H-NMR(CDC13, 200MHz) δ2.4(m, 3H), 2.5(m, 3H), 2.7(m, 1H), 6.2(m, 1H), 7.2(m, 1H), 7.4(m, 1H), 7.5(s, 1H), 8.5(s, 1H)

### Example 125

### (3R)-3-(3,4-dichlorophenyl)-N-methyl-3-(2H-tetrazol-2-yl)propan-1-amine

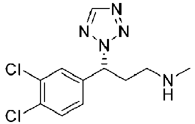

The procedure given in Example 3 was followed using [3-(3,4-Dichloro-phenyl)-3-tetrazol-2-yl-propyl]-methyl-amine, instead of [3-(3,4-Dichloro-phenyl)-3-tetrazol-2-yl-propyl]-methyl-amine, to give 3R-3-(3,4-Dichloro-phenyl)-3-tetrazol-2-yl-propyl]-methyl-amine.

1H-NMR(CDC13, 200MHz) δ2.4(m, 3H), 2.5(m, 3H), 2.7(m, 1H), 6.2(m, 1H), 7.2(m, 1H), 7.4(m, 1H), 7.5(s, 1H), 8.5(s, 1H)

### Example 126

### (3S)-3-(2H-benzotriazol-2-yl)-3-(3,4-dichlorophenyl)-N - methylpropan-1-amine

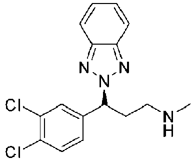

The procedure given in Example 3 was followed using [3-Benzotriazol-2-yl-3-(3,4-dichloro-phenyl)-propyl]-methyl-amine, instead of [3-(3,4-Dichloro-phenyl)-3-tetrazol-2-yl-propyl]-methyl-amine, to give S-[3-Benzotriazol-2-yl-3-(3,4-dichloro-phenyl)-propyl]-methyl-amine.

1H-NMR(CDC13, 200MHz) δ2.4(m, 3H), 2.5(m, 3H), 2.7(m, 1H), 6.2(m, 1H), 7.2(m, 4H), 7.6(s, 1H), 7.9(m, 2H)

### Example 127

### (3R)-3-(2H-benzotriazol-2-yl)-3-(3,4-dichlorophenyl)-N - methylpropan-1-amine

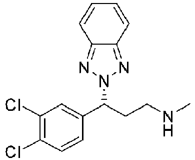

The procedure given in Example 3 was followed using [3-Benzotriazol-2-yl-3-(3,4-dichloro-phenyl)-propyl]-methyl-amine, instead of [3-(3,4-Dichloro-phenyl)-3-tetrazol-2-yl-propyl]-methyl-amine, to give R-[3-Benzotriazol-2-yl-3-(3,4-dichloro-phenyl)-propyl]-methyl-amine.

1H-NMR(CDC13, 200MHz) δ2.4(m, 3H), 2.5(m, 3H), 2.7(m, 1H), 6.2(m, 1H), 7.2(m, 4H), 7.6(s, 1H), 7.9(m, 2H)

### Example 128

### (3S)-3-(3,4-dichlorophenyl)-N-methyl-3-(2H-1,2,3-triazol-2-yl)propan-1-amine

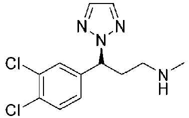

The procedure given in Example 3 was followed using [3-(3,4-Dichloro-phenyl)-3-[1,2,3]triazol-2-yl-propyl]-methyl-amine, instead of [3-(3,4-Dichloro-phenyl)-3-tetrazol-2-yl-propyl]-methyl-amine, to give S-[3-(3,4-Dichloro-phenyl)-3-[1,2,3]triazol-2-yl-propyl]-methyl-amine.

1H-NMR(CDC13, 200MHz) δ2.4(m, 3H), 2.5(m, 3H), 2.7(m, 1H), 6.2(m, 1H), 7.2(m, 1H), 7.4(m, 1H), 7.5(s, 1H), 7.6(d, 2H)

### Example 129

### (3S)-3-(3,4-dichlorophenyl)-N-methyl-3-(5-methyl-2H-tetrazol-2-yl)propan-1-amine

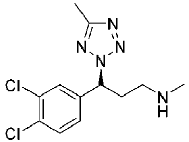

The procedure given in Example 3 was followed using [3-(3,4-Dichloro-phenyl)-3-(4-methyl-[1,2,3]triazol-2-yl)-propyl]-methyl-amine, instead of [3-(3,4-Dichloro-phenyl)-3-tetrazol-2-yl-propyl]-methyl-amine, to give S-[3-(3,4-Dichloro-phenyl)-3-(4-methyl-[1,2,3]triazol-2-yl)-propyl]-methyl-amine.

1H-NMR(CDC13, 200MHz) δ2.4(m, 3H), 2.5(m, 6H), 2.7(m, 1H), 6.2(m, 1H), 7.2(m, 1H), 7.4(m, 1H), 7.5(s, 1H)

### Example 130

### (3R)-3-(3,4-dichlorophenyl)-N-methyl-3-(5-methyl-2H-tetrazol-2-yl)propan-1-amine

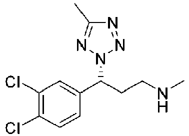

The procedure given in Example 3 was followed using [3-(3,4-Dichloro-phenyl)-3-(4-methyl-[1,2,3] triazol-2-yl)-propyl]-methyl-amine, instead of [3-(3,4-Dichloro-phenyl)-3-tetrazol-2-yl-propyl]-methyl-amine, to give R-[3-(3,4-Dichloro-phenyl)-3-(4-methyl-[1,2,3]triazol-2-yl)-propyl]-methyl-amine.

1H-NMR(CDC13, 200MHz) δ2.4(m, 3H), 2.5(m, 6H), 2.7(m, 1H), 6.2(m, 1H), 7.2(m, 1H), 7.4(m, 1H), 7.5(s, 1H)

### Example 131

### (3R)-3-(3,4-dichlorophenyl)-N-methyl-3-(2H-1,2,3-triazol-2-yl)propan-1-amine

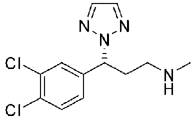

The procedure given in Example 3 was followed using [3-(3,4-Dichloro-phenyl)-3-[1,2,3]triazol-2-yl-propyl]-methyl-amine, instead of [3-(3,4-Dichloro-phenyl)-3-tetrazol-2-yl-propyl]-methyl-amine, to give R-[3-(3,4-Dichloro-phenyl)-3-[1,2,3]triazol-2-yl-propyl]-methyl-amine.

1H-NMR(CDC13, 200MHz) δ2.4(m, 3H), 2.5(m, 3H), 2.7(m, 1H), 6.2(m, 1H), 7.2(m, 1H), 7.4(m, 1H), 7.5(s, 1H), 7.6(d, 2H)

### Example 132

### (3R)-N-methyl-3-(2-naphthyl)-3-(2H-tetrazol-2-yl)propan-1-amine

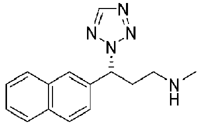

The procedure given in Example 3 was followed using Methyl-(3-naphthalen-2-yl-3-tetrazol-2-yl-propyl)-amine, instead of [3-(3,4-Dichloro-phenyl)-3-tetrazol-2-yl-propyl]-methyl-amine, to give R-Methyl-(3-naphthalen-2-yl-3-tetrazol-2-yl-propyl)-amine.

1H-NMR(CDC13, 200MHz) δ2.4(m, 3H), 2.5(m, 3H), 2.7(m, 1H), 6.2(m, 1H), 7.5(m, 3H), 7.8(m, 4H), 8.5(s, 1H)

### Example 133

### (3S)-N-methyl-3-(2-naphthyl)-3-(2H-tetrazol-2-yl)propan-1-amine

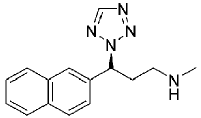

The procedure given in Example 3 was followed using Methyl-(3-naphthalen-2-yl-3-tetrazol-2-yl-propyl)-amine, instead of [3-(3,4-Dichloro-phenyl)-3-tetrazol-2-yl-propyl]-methyl-amine, to give S-Methyl-(3-naphthalen-2-yl-3-tetrazol-2-yl-propyl)-amine.

1H-NMR(CDC13, 200MHz) δ2.4(m, 3H), 2.5(m, 3H), 2.7(m, 1H), 6.2(m, 1H), 7.5(m, 3H), 7.8(m, 4H), 8.5(s, 1H)

### Example 134

### [3-(3-Methoxy-naphthalen-2-yl)-3-tetrazol-2-yl-propyl]-methyl-amine

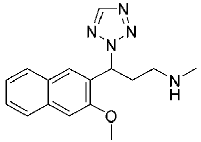

The procedure given in Example 2 was followed using [3-(3-Methoxy-naphthalen-2-yl)-3-tetrazol-2-yl-propyl]-dimethyl-amine as a reactant, instead of [3-(3,4-Dichloro-phenyl)-3-tetrazol-2-yl-propyl]-dimethyl-amine, to give [3-(3-Methoxy-naphthalen-2-yl)-3-tetrazol-2-yl-propyl]-methyl-amine.

1H-NMR(CDC13, 200MHz) δ2.4(m, 10),6.2(m, 1H), 7.5(m, 3H), 7.8(m, 4H), 8.5(s, 1H)

### Example 135

### [3-(3-Methoxy-naphthalen-2-yl)-3-(5-methyl-tetrazol-2-yl)-propyl]-methyl-amin e

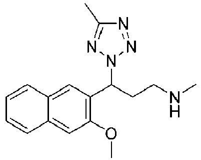

The procedure given in Example 2 was followed using [3-(3-Methoxy-naphthalen-2-yl)-3-(5-methyl-tetrazol-2-yl)-propyl]-dimethyl-amine as a reactant, instead of [3-(3,4-Dichloro-phenyl)-3-tetrazol-2-yl-propyl]-dimethyl-amine, to give [3-(3-Methoxy-naphthalen-2-yl)-3-(5-methyl-tetrazol-2-yl)-propyl]-methyl-amine.

1H-NMR(CDC13, 200MHz) δ2.4(m, 9H), 2.5(m, 5H), 2.8(m, 1H), 4.0(s, 3H), 6.6(m, 1H), 7.5(m, 3H), 7.8(m, 4H)

### Example 136

### [3-(6-Fluoro-naphthalen-2-yl)-3-tetrazol-2-yl-propyl]-methyl-amine

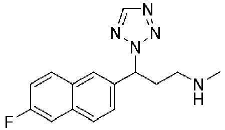

The procedure given in Example 2 was followed using [3-(6-Fluoro-naphthalen-2-yl)-3-tetrazol-2-yl-propyl]-dimethyl-amine as a reactant, instead of [3-(3,4-Dichloro-phenyl)-3-tetrazol-2-yl-propyl]-dimethyl-amine, to give [3-(6-Fluoro-naphthalen-2-yl)-3-tetrazol-2-yl-propyl]-methyl-amine.

1H-NMR(CDC13, 200MHz) δ2.4(m, 9H), 2.5(m, 1H), 2.8(m, 1H), 6.2(m, 1H), 7.2(m, 1H), 7.4(m, 1H), 7.5(m, 1H), 7.8(m, 3H), 8.5(s, 1H)

### Example 137

### [3-(6-Fluoro-naphthalen-2-yl)-3-(5-methyl-tetrazol-2-yl)-propyl]-methyl-amine

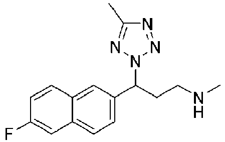

The procedure given in Example 2 was followed using [3-(6-Fluoro-naphthalen-2-yl)-3-(5-methyl-tetrazol-2-yl)-propyl]-dimethyl-amine as a reactant, instead of [3-(3,4-Dichloro-phenyl)-3-tetrazol-2-yl-propyl]-dimethyl-amine, to give [3-(6-Fluoro-naphthalen-2-yl)-3-(5-methyl-tetrazol-2-yl)-propyl]-methyl-amine.

1H-NMR(CDC13, 200MHz) δ2.4(m, 6H), 2.5(m, 2H), 2.8(m, 1H), 6.2(t, 1H), 7.2(m, 1H), 7.4(m, 1H), 7.5(m, 1H), 7.8(m, 3H)

### Example 138

### [3-(1,4-Dimethoxy-naphthalen-2-yl)-3-tetrazol-2-yl-propyl]-methyl-amine

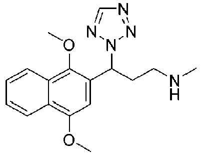

The procedure given in Example 2 was followed using [3-(1,4-Dimethoxy-naphthalen-2-yl)-3-tetrazol-2-yl-propyl]-dimethyl-amine as a reactant, instead of [3-(3,4-Dichloro-phenyl)-3-tetrazol-2-yl-propyl]-dimethyl-amine, to give [3-(1,4-Dimethoxy-naphthalen-2-yl)-3-tetrazol-2-yl-propyl]-methyl-amine.

1H-NMR(CDC13, 200MHz) δ2.4(m, 9H), 2.5(m, 1H), 2.8(m, 1H), 4.0(d, 6H), 6.2(m, 1H), 7.2(m, 1H), 7.4(m, 1H), 7.5(m, 1H), 7.8(m, 2H), 8.5(s, 1H)

### Example 139

### [3-(1,4-Dimethoxy-naphthalen-2-yl)-3-(5-methyl-tetrazol-2-yl)-propyl]-methyl-a mine

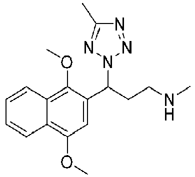

The procedure given in Example 2 was followed using [3-(1,4-Dimethoxy-naphthalen-2-yl)-3-(5-methyl-tetrazol-2-yl)-propyl]-dimethyl-amin e as a reactant, instead of [3-(3,4-Dichloro-phenyl)-3-tetrazol-2-yl-propyl]-dimethyl-amine, to give [3-(1,4-Dimethoxy-naphthalen-2-yl)-3-(5-methyl-tetrazol-2-yl)-propyl]-methyl-amine.

1H-NMR(CDC13, 200MHz) δ2.4(m, 9H), 2.5(m, 1H), 2.8(m, 1H), 4.0(d, 6H), 6.2(m, 1H), 6.9(s, 1H), 7.5(m, 2H), 8.0(s, 1H), 8.2(d, 1H)

### Example 140

### [3-(3,5-Dimethoxy-naphthalen-2-yl)-3-tetrazol-2-yl-propyl]-methyl-amine

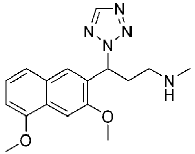

The procedure given in Example 2 was followed using [3-(3,5-Dimethoxy-naphthalen-2-yl)-3-tetrazol-2-yl-propyl]-dimethyl-amineas a reactant, instead of [3-(3,4-Dichloro-phenyl)-3-tetrazol-2-yl-propyl]-dimethyl-amine, to give [3-(3,5-Dimethoxy-naphthalen-2-yl)-3-tetrazol-2-yl-propyl]-methyl-amine.

1H-NMR(CDC13, 200MHz) δ2.4(m, 9H), 2.5(m, 1H), 2.8(m, 1H), 4.0(m, 6H), 6.8(m, 1H), 7.3(s, 6H), 7.5(m, 2H), 8.5(s, 1H)

### Example 141

### [3-(3,5-Dimethoxy-naphthalen-2-yl)-3-(5-methyl-tetrazol-2-yl)-propyl]-methyl-a mine

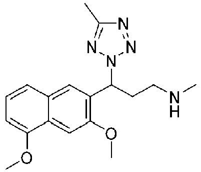

The procedure given in Example 2 was followed using [3-(3,5-Dimethoxy-naphthalen-2-yl)-3-(5-methyl-tetrazol-2-yl)-propyl]-dimethyl-amin e as a reactant, instead of [3-(3,4-Dichloro-phenyl)-3-tetrazol-2-yl-propyl]-dimethyl-amine, to give [3-(3,5-Dimethoxy-naphthalen-2-yl)-3-(5-methyl-tetrazol-2-yl)-propyl]-methyl-amine.

1H-NMR(CDC13, 200MHz) δ2.0(s, 6H), 2.4(m, 6H), 2.5(m, 2H), 2.8(m, 1H), 4.0(m, 6H), 6.6(m, 1H), 6.8(m, 1H), 7.2(s, 3H), 7.7(d, 2H)

### Example 142

### [3-(3,5-Dimethoxy-naphthalen-2-yl)-3-tetrazol-1-yl-propyl]-methyl-amine

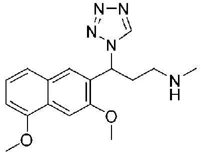

The procedure given in Example 2 was followed [3-(3,5-Dimethoxy-naphthalen-2-yl)-3-tetrazol-1-yl-propyl]-dimethyl-amine as a reactant, instead of [3-(3,4-Dichloro-phenyl)-3-tetrazol-2-yl-propyl]-dimethyl-amine, to give [3-(3,5-Dimethoxy-naphthalen-2-yl)-3-tetrazol-1-yl-propyl]-methyl-amine.

1H-NMR(CDC13, 200MHz) δ2.4(m, 9H), 2.5(m, 4H), 2.8(m, 1H), 4.0(m, 6H), 6.6(m, 1H), 6.8(m, 1H), 7.2(s, 3H), 7.5(s, 1H), 7.7(s, 1H), 8.7(s, 1H)

### Example 143

### Methyl-[3-(4-methyl-naphthalen-1-yl)-3-tetrazol-2-yl-propyl]-amine

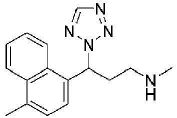

The procedure given in Example 2 was followed Dimethyl-[3-(4-methyl-naphthalen-1-yl)-3-tetrazol-2-yl-propyl]-amine as a reactant, instead of [3-(3,4-Dichloro-phenyl)-3-tetrazol-2-yl-propyl]-dimethyl-amine, to give Methyl-[3-(4-methyl-naphthalen-1-yl)-3-tetrazol-2-yl-propyl]-amine.

1H-NMR(CDC13, 200MHz) δ2.2(m, 9H), 2.4(m, 1H), 2.8(m, 4H), 3.0(m, 1H), 7.0(m, 1H), 7.2(m, 1H), 7.5(m, 4H), 8.0(m, 1H), 8.3(m, 1H), 8.5(s, 1H)

### Example 144

### Methyl-[3-(4-methyl-naphthalen-1-yl)-3-(5-methyl-tetrazol-2-yl)-propyl]-amine

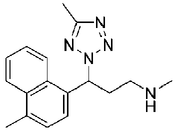

The procedure given in Example 2 was followed Dimethyl-[3-(4-methyl-naphthalen-1-yl)-3-(5-methyl-tetrazol-2-yl)-propyl]-amine as a reactant, instead of [3-(3,4-Dichloro-phenyl)-3-tetrazol-2-yl-propyl]-dimethyl-amine, to give Methyl-[3-(4-methyl-naphthalen-1-yl)-3-(5-methyl-tetrazol-2-yl)-propyl]-amine.

1H-NMR(CDC13, 200MHz) δ2.2(m, 6H), 2.4(m, 3H), 2.8(m, 3H), 3.0(m, 1H), 7.0(m, 1H), 7.2(m, 1H), 7.5(m, 4H), 8.0(m, 1H), 8.3(m, 1H)

### Example 145

### [3-(4-Fluoro-naphthalen-1-yl)-3-tetrazol-2-yl-propyl]-methyl-amine

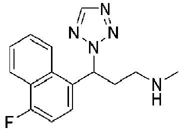

The procedure given in Example 2 was followed [3-(4-Fluoro-naphthalen-1-yl)-3-tetrazol-2-yl-propyl]-dimethyl-amine as a reactant, instead of [3-(3,4-Dichloro-phenyl)-3-tetrazol-2-yl-propyl]-dimethyl-amine, to give [3-(4-Fluoro-naphthalen-1-yl)-3-tetrazol-2-yl-propyl]-methyl-amine.

1H-NMR(CDC13, 200MHz) δ2.2(m, 9H), 2.4(m, 1H), 2.8(m, 1H), 7.0(m, 1H), 7.2(m, 1H), 7.5(m, 4H), 8.0(m, 1H), 8.3(m, 1H), 8.5(s, 1H)

### Example 146

### [3-(4-Fluoro-naphthalen-1-yl)-3-(5-methyl-tetrazol-2-yl)-propyl]-methyl-amine

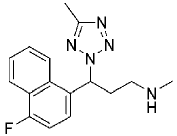

The procedure given in Example 2 was followed [3-(4-Fluoro-naphthalen-1-yl)-3-(5-methyl-tetrazol-2-yl)-propyl]-dimethyl-amine as a reactant, instead of [3-(3,4-Dichloro-phenyl)-3-tetrazol-2-yl-propyl]-dimethyl-amine, to give [3-(4-Fluoro-naphthalen-1-yl)-3-(5-methyl-tetrazol-2-yl)-propyl]-methyl-amine.

1H-NMR(CDC13, 200MHz) δ2.2(m, 4H), 2.4(m, 6H), 2.8(m, 1H), 7.0(m, 1H), 7.2(m, 1H), 7.5(m, 3H), 8.1(m, 1H), 8.3(m, 1H)

### Example 147

### (3-Benzo[1,3]dioxol-5-yl-3-tetrazol-2-yl-propyl)-methyl-amine

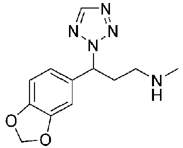

The procedure given in Example 2 was followed (3-Benzo[1,3]dioxol-5-yl-3-tetrazol-2-yl-propyl)-dimethyl-amine as a reactant, instead of [3-(3,4-Dichloro-phenyl)-3-tetrazol-2-yl-propyl]-dimethyl-amine, to give (3-Benzo[1,3]dioxol-5-yl-3-tetrazol-2-yl-propyl)-methyl-amine.

1H-NMR(CDC13, 200MHz) δ2.2(m, 6H), 2.5(m, 1H), 2.8(m, 1H), 6.0(s, 2H), 6.1(m, 1H), 6.8(d, 1H), 7.0(d, 2H), 8.5(m, 1H)

### Example 148

### [3-Benzo[1,3]dioxol-5-yl-3-(5-methyl-tetrazol-2-yl)-propyl]-methyl-amine

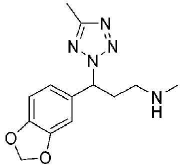

The procedure given in Example 2 was followed [3-Benzo[1,3]dioxol-5-yl-3-(5-methyl-tetrazol-2-yl)-propyl]-dimethyl-amine as a reactant, instead of [3-(3,4-Dichloro-phenyl)-3-tetrazol-2-yl-propyl]-dimethyl-amine, to give [3-Benzo[1,3]dioxol-5-yl-3-(5-methyl-tetrazol-2-yl)-propyl]-methyl-amine.

1H-NMR(CDC13, 200MHz) δ2.2(m, 12H), 2.8(m, 1H), 6.0(s, 2H), 6.1(m, 1H), 6.8(d, 1H), 7.0(d, 2H), 8.5(m, 1H)

### Example 149

### [3-Benzo[1,3]dioxol-5-yl-3-(5-methyl-tetrazol-1-yl)-propyl]-methyl-amine

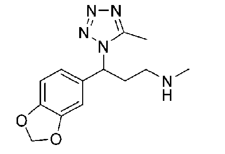

The procedure given in Example 2 was followed [3-Benzo[1,3]dioxol-5-yl-3-(5-methyl-tetrazol-1-yl)-propyl]-dimethyl-amine as a reactant, instead of [3-(3,4-Dichloro-phenyl)-3-tetrazol-2-yl-propyl]-dimethyl-amine, to give [3-Benzo[1,3]dioxol-5-yl-3-(5-methyl-tetrazol-1-yl)-propyl]-methyl-amine.

1H-NMR(CDC13, 200MHz) δ2.2(m, 6H), 2.4(m, 1H), 2.5(s, 3H), 2.8(m, 1H), 5.5(t, 2H), 6.1(s, 2H), 6.8(m, 3H)

### Example 150

### [3-(3,4-Dimethoxy-phenyl)-3-tetrazol-2-yl-propyl]-methyl-amine

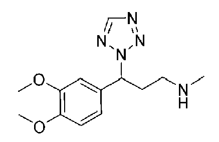

The procedure given in Example 2 was followed [3-(3,4-Dimethoxy-phenyl)-3-tetrazol-2-yl-propyl]-dimethyl-amine as a reactant, instead of [3-(3,4-Dichloro-phenyl)-3-tetrazol-2-yl-propyl]-dimethyl-amine, to give [3-(3,4-Dimethoxy-phenyl)-3-tetrazol-2-yl-propyl]-methyl-amine.

1H-NMR(CDC13, 200MHz) δ2.2(s, 9H), 2.4(m, 1H), 2.8(m, 1H), 4.0(s, 6H), 6.1(s, 2H), 6.8(d, 1H), 7.0(d, 2H), 8.5(s, 1H)

### Example 151

### [3-(2,3-Dihydro-benzo[1,4]dioxin-2-yl)-3-tetrazol-2-yl-propyl]-methyl-amine

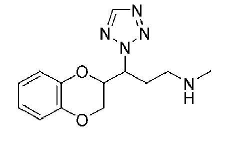

The procedure given in Example 2 was followed [3-(2,3-Dihydro-benzo[1,4]dioxin-2-yl)-3-tetrazol-2-yl-propyl]-dimethyl-amine as a reactant, instead of [3-(3,4-Dichloro-phenyl)-3-tetrazol-2-yl-propyl]-dimethyl-amine, to give [3-(2,3-Dihydro-benzo[1,4]dioxin-2-yl)-3-tetrazol-2-yl-propyl]-methyl-amine.

1H-NMR(CDC13, 200MHz) δ2.2(s, 9H), 2.4(m, 3H), 3.8(m, 1H), 4.6(m, 1H), 5.4(m, 1H), 7.0(s, 5H), 8.5(s, 1H)

### Example 152

### [3-(2,3-Dihydro-benzo[1,4]dioxin-2-yl)-3-(5-methyl-tetrazol-2-yl)-propyl]-methyl -amine

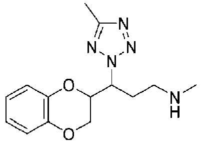

The procedure given in Example 2 was followed [3-(2,3-Dihydro-benzo[1,4]dioxin-2-yl)-3-(5-methyl-tetrazol-2-yl)-propyl]-dimethyl-a mine as a reactant, instead of [3-(3,4-Dichloro-phenyl)-3-tetrazol-2-yl-propyl]-dimethyl-amine, to give [3-(2,3-Dihydro-benzo[1,4]dioxin-2-yl)-3-(5-methyl-tetrazol-2-yl)-propyl]-methyl-ami ne.

1H-NMR(CDC13, 200MHz) δ2.2(s, 7H), 2.4(m, 3H), 2.8(m, 4H), 3.8(m, 1H), 4.0(m, 1H), 4.8(m, 1H), 5.3(m, 1H), 7.0(s, 4H)

### Example 153

### Methyl-[3-(1,2,3,4-tetrahydro-naphthalen-2-yl)-3-tetrazol-2-yl-propyl]-amine

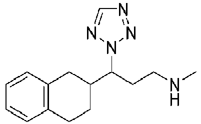

The procedure given in Example 2 was followed Dimethyl-[3-(1,2,3,4-tetrahydro-naphthalen-2-yl)-3-tetrazol-2-yl-propyl]-amine as a reactant, instead of [3-(3,4-Dichloro-phenyl)-3-tetrazol-2-yl-propyl]-dimethyl-amine, to give Methyl-[3-(1,2,3,4-tetrahydro-naphthalen-2-yl)-3-tetrazol-2-yl-propyl]-amine.

1H-NMR(CDC13, 200MHz) δ2.2(s, 8H), 2.8(m, 8H), 5.0(m, 1H), 7.0(m, 4H), 8.5(s, 1H)

### Example 154

### [3-(6-Chloro-pyridin-3-yl)-3-tetrazol-2-yl-propyl]-methyl-amine

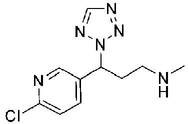

The procedure given in Example 2 was followed [3-(6-Chloro-pyridin-3-yl)-3-tetrazol-2-yl-propyl]-dimethyl-amine as a reactant, instead of [3-(3,4-Dichloro-phenyl)-3-tetrazol-2-yl-propyl]-dimethyl-amine, to give [3-(6-Chloro-pyridin-3-yl)-3-tetrazol-2-yl-propyl]-methyl-amine.

1H-NMR(CDC13, 200MHz) δ2.2(m, 7H), 2.5(m, 1H), 2.8(m, 1H), 6.2(m, 1H), 7.2(m, 1H), 7.8(m, 1H), 8.5(s, 1H)

### Example 155

### (3-Benzo[b]thiophen-2-yl-3-tetrazol-2-yl-propyl)-methyl-amine

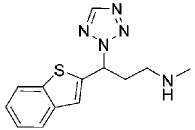

The procedure given in Example 2 was (3-Benzo[b]thiophen-2-yl-3-tetrazol-2-yl-propyl)-dimethyl-amine as a reactant, instead of [3-(3,4-Dichloro-phenyl)-3-tetrazol-2-yl-propyl]-dimethyl-amine, to give (3-Benzo[b]thiophen-2-yl-3-tetrazol-2-yl-propyl)-methyl-amine.

1H-NMR(CDC13, 200MHz) δ2.2(m, 7H), 2.8(m, 1H), 6.5(m, 1H), 7.4(m, 3H), 7.8(m, 2H), 8.5(s, 1H)

### Example 156

### [3-Benzo[b]thiophen-2-yl-3-(5-methyl-tetrazol-2-yl)-propyl]-methyl-amine

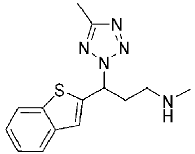

The procedure given in Example 2 was [3-Benzo[b]thiophen-2-yl-3-(5-methyl-tetrazol-2-yl)-propyl]-dimethyl-amine as a reactant, instead of [3-(3,4-Dichloro-phenyl)-3-tetrazol-2-yl-propyl]-dimethyl-amine, to give [3-Benzo[b]thiophen-2-yl-3-(5-methyl-tetrazol-2-yl)-propyl]-methyl-amine.

1H-NMR(CDC13, 200MHz) δ2.2(m, 6H), 2.8(m, 5H), 6.5(m, 1H), 7.4(m, 3H), 7.8(m, 2H)

### Example 157

### (3-Benzofuran-2-yl-3-tetrazol-2-yl-propyl)-methyl-amine

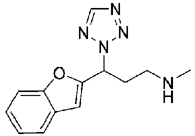

The procedure given in Example 2 was (3-Benzofuran-2-yl-3-tetrazol-2-yl-propyl)-dimethyl-amine as a reactant, instead of [3-(3,4-Dichloro-phenyl)-3-tetrazol-2-yl-propyl]-dimethyl-amine, to give (3-Benzofuran-2-yl-3-tetrazol-2-yl-propyl)-methyl-amine.

1H-NMR(CDC13, 200MHz) δ2.2(m, 7H), 2.8(m, 2H), 6.5(m, 1H), 7.4(m, 3H), 7.8(m, 2H), 8.5(s, 1H)

### Example 158

### [3-Benzofuran-2-yl-3-(5-methyl-tetrazol-2-yl)-propyl]-methyl-amine

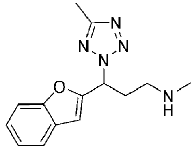

The procedure given in Example 2 was [3-Benzofuran-2-yl-3-(5-methyl-tetrazol-2-yl)-propyl]-dimethyl-amine as a reactant, instead of [3-(3,4-Dichloro-phenyl)-3-tetrazol-2-yl-propyl]-dimethyl-amine, to give [3-Benzofuran-2-yl-3-(5-methyl-tetrazol-2-yl)-propyl]-methyl-amine.

1H-NMR(CDC13, 200MHz) δ2.2(m, 7H), 2.8(m, 2H), 6.5(m, 1H), 7.4(m, 3H), 7.8(m, 2H)

### Example 159

### (3-Benzofuran-2-yl-3-tetrazol-1-yl-propyl)-methyl-amine

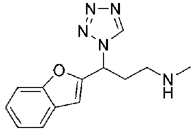

The procedure given in Example 2 was (3-Benzofuran-2-yl-3-tetrazol-1-yl-propyl)-dimethyl-amine as a reactant, instead of [3-(3,4-Dichloro-phenyl)-3-tetrazol-2-yl-propyl]-dimethyl-amine, to give (3-Benzofuran-2-yl-3-tetrazol-1-yl-propyl)-methyl-amine.

1H-NMR(CDC13, 200MHz) δ2.2(m, 7H), 2.8(m, 2H), 6.2(m, 1H), 7.4(m, 3H), 7.8(m, 2H)

### Example 160

### [3-(3,4-Difluoro-phenyl)-3-tetrazol-2-yl-propyl]-methyl-amine

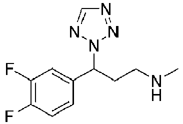

The procedure given in Example 2 was [3-(3,4-Difluoro-phenyl)-3-tetrazol-2-yl-propyl]-dimethyl-amine as a reactant, instead of [3-(3,4-Dichloro-phenyl)-3-tetrazol-2-yl-propyl]-dimethyl-amine, to give [3-(3,4-Difluoro-phenyl)-3-tetrazol-2-yl-propyl]-methyl-amine.

1H-NMR(CDC13, 200MHz) δ2.2(m, 12H), 6.2(m, 1H), 7.4(m, 4H), 8.5(s, 1H)

### Example 161

### [3-(3,4-Difluoro-phenyl)-3-(5-methyl-tetrazol-2-yl)-propyl]-methyl-amine

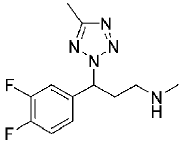

The procedure given in Example 2 was [3-(3,4-Difluoro-phenyl)-3-(5-methyl-tetrazol-2-yl)-propyl]-dimethyl-amine as a reactant, instead of [3-(3,4-Dichloro-phenyl)-3-tetrazol-2-yl-propyl]-dimethyl-amine, to give [3-(3,4-Difluoro-phenyl)-3-(5-methyl-tetrazol-2-yl)-propyl]-methyl-amine.

1H-NMR(CDC13, 200MHz) δ2.2(m, 7H), 2.8(m, 4H), 6.2(m, 1H), 7.4(m, 4H)

### Example 162

### Methyl-[3-tetrazol-2-yl-3-(2,4,5-trifluoro-phenyl)-propyl]-amine

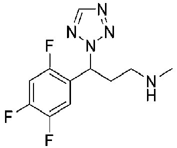

The procedure given in Example 2 was Dimethyl-[3-tetrazol-2-yl-3-(2,4,5-trifluoro-phenyl)-propyl]-amine as a reactant, instead of [3-(3,4-Dichloro-phenyl)-3-tetrazol-2-yl-propyl]-dimethyl-amine, to give Methyl-[3-tetrazol-2-yl-3-(2,4,5-trifluoro-phenyl)-propyl]-amine.

1H-NMR(CDC13, 200MHz) δ2.2(m, 9H), 2.8(m, 2H), 6.5(m, 1H), 7.0(m, 1H), 7.4(m, 1H), 8.6(s, 1H)

### Example 163

### Methyl-[3-(5-methyl-tetrazol-2-yl)-3-(2,4,5-trifluoro-phenyl)-propyl]-amine

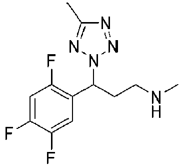

The procedure given in Example 2 was Dimethyl-[3-(5-methyl-tetrazol-2-yl)-3-(2,4,5-trifluoro-phenyl)-propyl]-amineas a reactant, instead of [3-(3,4-Dichloro-phenyl)-3-tetrazol-2-yl-propyl]-dimethyl-amine, to give Methyl-[3-(5-methyl-tetrazol-2-yl)-3-(2,4,5-trifluoro-phenyl)-propyl]-amine.

1H-NMR(CDC13, 200MHz) δ2.2(m, 12H), 6.4(m, 1H), 7.0(m, 1H), 7.4(m, 1H)

### Example 164

### [3-(3-Bromo-4-fluoro-phenyl)-3-tetrazol-2-yl-propyl]-methyl-amine

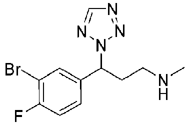

The procedure given in Example 2 was [3-(3-Bromo-4-fluoro-phenyl)-3-tetrazol-2-yl-propyl]-dimethyl-amine as a reactant, instead of [3-(3,4-Dichloro-phenyl)-3-tetrazol-2-yl-propyl]-dimethyl-amine, to give [3-(3-Bromo-4-fluoro-phenyl)-3-tetrazol-2-yl-propyl]-methyl-amine.

1H-NMR(CDC13, 200MHz) δ2.2(m, 7H), 2.4(m, 2H), 2.8(m, 1H), 6.5(m, 1H), 7.0(m, 1H), 7.4(m, 1H), 7.8(m, 1H), 8.5(s, 1H)

### Example 165

### [3-(3-Bromo-4-fluoro-phenyl)-3-(5-methyl-tetrazol-2-yl)-propyl]-methyl-amine

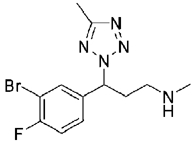

The procedure given in Example 2 was [3-(3-Bromo-4-fluoro-phenyl)-3-(5-methyl-tetrazol-2-yl)-propyl]-dimethyl-amine as a reactant, instead of [3-(3,4-Dichloro-phenyl)-3-tetrazol-2-yl-propyl]-dimethyl-amine, to give [3-(3-Bromo-4-fluoro-phenyl)-3-(5-methyl-tetrazol-2-yl)-propyl]-methyl-amine.

1H-NMR(CDC13, 200MHz) δ2.2(m, 7H), 2.4(m, 2H), 2.8(m, 1H), 6.5(m, 1H), 7.0(m, 1H), 7.4(m, 1H), 7.8(m, 1H)

### Example 166

### [3-(6-Methoxy-naphthalen-2-yl)-3-tetrazol-2-yl-propyl]-methyl-amine

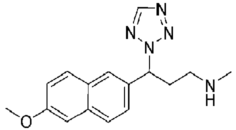

The procedure given in Example 2 was [3-(6-Methoxy-naphthalen-2-yl)-3-tetrazol-2-yl-propyl]-dimethyl-amine as a reactant, instead of [3-(3,4-Dichloro-phenyl)-3-tetrazol-2-yl-propyl]-dimethyl-amine, to give [3-(6-Methoxy-naphthalen-2-yl)-3-tetrazol-2-yl-propyl]-methyl-amine.

1H-NMR(CDC13, 200MHz) δ2.2(m, 5H), 2.4(m, 2H), 2.8(m, 1H), 4.0(s, 3H), 6.3(m, 1H), 7.1(m, 3H), 7.4(m, 1H), 7.8(m, 1H), 8.5(s, 1H)

### Example 167

### [3-(6-Methoxy-naphthalen-2-yl)-3-(5-methyl-tetrazol-2-yl)-propyl]-methyl-amin e

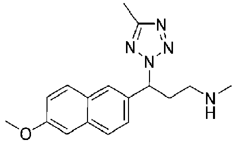

The procedure given in Example 2 was [3-(6-Methoxy-naphthalen-2-yl)-3-(5-methyl-tetrazol-2-yl)-propyl]-dimethyl-amine as a reactant, instead of [3-(3,4-Dichloro-phenyl)-3-tetrazol-2-yl-propyl]-dimethyl-amine, to give [3-(6-Methoxy-naphthalen-2-yl)-3-(5-methyl-tetrazol-2-yl)-propyl]-methyl-amine.

1H-NMR(CDC13, 200MHz) δ2.2(m, 8H), 2.4(m, 2H), 2.8(m, 1H), 4.0(s, 3H), 6.3(m, 1H), 7.1(m, 3H), 7.4(m, 1H), 7.8(m, 1H)

### Example 168

### Methyl-[3-(4-phenoxy-phenyl)-3-tetrazol-2-yl-propyl]-amine

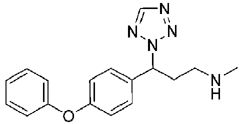

The procedure given in Example 2 was Dimethyl-[3-(4-phenoxy-phenyl)-3-tetrazol-2-yl-propyl]-amine as a reactant, instead of [3-(3,4-Dichloro-phenyl)-3-tetrazol-2-yl-propyl]-dimethyl-amine, to give Methyl-[3-(4-phenoxy-phenyl)-3-tetrazol-2-yl-propyl]-amine.

1H-NMR(CDCl3, 200MHz) δ2.2(m, 7H), 2.4(m, 1H), 2.8(m, 1H), 6.2(m, 1H), 7.1(m, 3H), 7.4(m, 1H), 7.6(m, 4H), 8.5(s, 1H)

### Example 169

### Methyl-[3-(5-methyl-tetrazol-2-yl)-3-(4-phenoxy-phenyl)-propyl]-amine

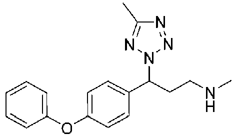

The procedure given in Example 2 was Dimethyl-[3-(5-methyl-tetrazol-2-yl)-3-(4-phenoxy-phenyl)-propyl]-amine as a reactant, instead of [3-(3,4-Dichloro-phenyl)-3-tetrazol-2-yl-propyl]-dimethyl-amine, to give Methyl-[3-(5-methyl-tetrazol-2-yl)-3-(4-phenoxy-phenyl)-propyl]-amine.

1H-NMR(CDC13, 200MHz) δ2.2(m, 5H), 2.4(m, 6H), 2.8(m, 1H), 6.2(m, 1H), 7.1(m, 4H), 7.4(m, 4H)

### Example 170

### [3-(4-Bromo-phenyl)-3-tetrazol-2-yl-propyl]-methyl-amine

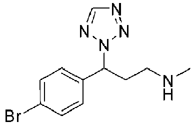

The procedure given in Example 2 was [3-(4-Bromo-phenyl)-3-tetrazol-2-yl-propyl]-dimethyl-amine as a reactant, instead of [3-(3,4-Dichloro-phenyl)-3-tetrazol-2-yl-propyl]-dimethyl-amine, to give [3-(4-Bromo-phenyl)-3-tetrazol-2-yl-propyl]-methyl-amine.

1H-NMR(CDCl3, 200MHz) δ2.2(m, 7H), 2.4(m, 1H), 2.8(m, 1H), 6.2(m, 1H), 7.3(m, 2H), 7.5(m, 2H), 8.5(s, 1H)

### Example 171

### [3-(4-Bromo-phenyl)-3-(5-methyl-tetrazol-2-yl)-propyl]-methyl-amine

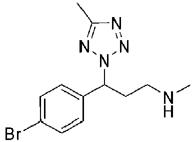

The procedure given in Example 2 was [3-(4-Bromo-phenyl)-3-(5-methyl-tetrazol-2-yl)-propyl]-dimethyl-amine as a reactant, instead of [3-(3,4-Dichloro-phenyl)-3-tetrazol-2-yl-propyl]-dimethyl-amine, to give [3-(4-Bromo-phenyl)-3-(5-methyl-tetrazol-2-yl)-propyl]-methyl-amine.

1H-NMR(CDC13, 200MHz) δ2.2(m, 6H), 2.3(m, 1H), 2.5(s, 3H), 2.8(m, 1H), 6.0(m, 1H), 7.3(m, 2H), 7.5(m, 2H)

### Example 172

### Methyl-[3-tetrazol-2-yl-3-(4-trifluoromethyl-phenyl)-propyl]-amine

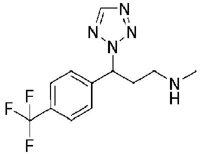

The procedure given in Example 2 was Dimethyl-[3-tetrazol-2-yl-3-(4-trifluoromethyl-phenyl)-propyl]-amine as a reactant, instead of [3-(3,4-Dichloro-phenyl)-3-tetrazol-2-yl-propyl]-dimethyl-amine, to give Methyl-[3-tetrazol-2-yl-3-(4-trifluoromethyl-phenyl)-propyl]-amine.

1H-NMR(CDCl3, 200MHz) δ2.2(m, 6H), 2.3(m, 1H), 2.8(m, 1H), 6.2(m, 1H), 7.3(m, 2H), 7.5(m, 2H), 8.5(s, 1H)

### Example 173

### Methyl-[3-(5-methyl-tetrazol-2-yl)-3-(4-trifluoromethyl-phenyl)-propyl]-amine

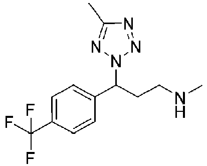

The procedure given in Example 2 was Dimethyl-[3-(5-methyl-tetrazol-2-yl)-3-(4-trifluoromethyl-phenyl)-propyl]-amine as a reactant, instead of [3-(3,4-Dichloro-phenyl)-3-tetrazol-2-yl-propyl]-dimethyl-amine, to give Methyl-[3-(5-methyl-tetrazol-2-yl)-3-(4-trifluoromethyl-phenyl)-propyl]-amine.

1H-NMR(CDC13, 200MHz) δ2.2(m, 6H), 2.3(m, 1H), 2.5(s, 3H), 2.8(m, 1H), 6.0(m, 1H), 7.6(m, 4H)

### Example 174

### Methyl-[3-tetrazol-2-yl-3-(2,3,4-trichloro-phenyl)-propyl]-amine

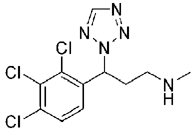

The procedure given in Example 2 was Dimethyl-[3-tetrazol-2-yl-3-(2,3,4-trichloro-phenyl)-propyl]-amine as a reactant, instead of [3-(3,4-Dichloro-phenyl)-3-tetrazol-2-yl-propyl]-dimethyl-amine, to give Methyl-[3-tetrazol-2-yl-3-(2,3,4-trichloro-phenyl)-propyl]-amine.

1H-NMR(CDCl3, 200MHz) δ2.2(m, 6H), 2.3(m, 1H), 2.8(m, 1H), 6.2(m, 1H), 7.3(m, 2H), 8.5(s, 1H)

### Example 175

### Methyl-[3-(5-methyl-tetrazol-2-yl)-3-(2,3,4-trichloro-phenyl)-propyl]-amine

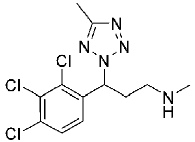

The procedure given in Example 2 was Dimethyl-[3-(5-methyl-tetrazol-2-yl)-3-(2,3,4-trichloro-phenyl)-propyl]-amine as a reactant, instead of [3-(3,4-Dichloro-phenyl)-3-tetrazol-2-yl-propyl]-dimethyl-amine, to give Methyl-[3-(5-methyl-tetrazol-2-yl)-3-(2,3,4-trichloro-phenyl)-propyl]-amine.

1H-NMR(CDC13, 200MHz) δ2.2(m, 6H), 2.3(m, 1H), 2.5(s, 3H), 2.8(m, 1H), 6.0(m, 1H), 7.2(m, 2H)

### Example 176

### [3-(3-Chloro-4-methoxy-phenyl)-3-tetrazol-2-yl-propyl]-methyl-amine

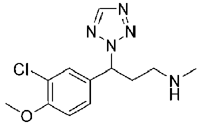

The procedure given in Example 2 was [3-(3-Chloro-4-methoxy-phenyl)-3-tetrazol-2-yl-propyl]-dimethyl-amine as a reactant, instead of [3-(3,4-Dichloro-phenyl)-3-tetrazol-2-yl-propyl]-dimethyl-amine, to give [3-(3-Chloro-4-methoxy-phenyl)-3-tetrazol-2-yl-propyl]-methyl-amine.

1H-NMR(CDCl3, 200MHz) δ2.2(m, 7H), 2.3(m, 1H), 2.8(m, 1H), 4.0(s, 3H), 6.1(m, 1H), 6.9(d, 1H), 7.3(d, 1H), 7.5(s, 1H), 8.5(s, 1H)

### Example 177

### [3-(3-Chloro-4-methoxy-phenyl)-3-(5-methyl-tetrazol-2-yl)-propyl]-methyl-amin e

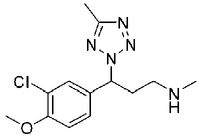

The procedure given in Example 2 was [3-(3-Chloro-4-methoxy-phenyl)-3-(5-methyl-tetrazol-2-yl)-propyl]-dimethyl-amine as a reactant, instead of [3-(3,4-Dichloro-phenyl)-3-tetrazol-2-yl-propyl]-dimethyl-amine, to give [3-(3-Chloro-4-methoxy-phenyl)-3-(5-methyl-tetrazol-2-yl)-propyl]-methyl-amine.

1H-NMR(CDCl3, 200MHz) δ2.2(m, 7H), 2.3(m, 1H), 2.8(m, 1H), 4.0(s, 3H), 6.1(m, 1H), 6.9(d, 1H), 7.3(d, 1H), 7.5(s, 1H)

### Example 178

### [3-(2-Chloro-phenyl)-3-(5-methyl-tetrazol-2-yl)-propyl]-methyl-amine

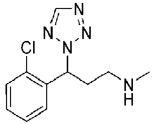

The procedure given in Example 2 was [3-(2-Chloro-phenyl)-3-(5-methyl-tetrazol-2-yl)-propyl]-dimethyl-amine as a reactant, instead of [3-(3,4-Dichloro-phenyl)-3-tetrazol-2-yl-propyl]-dimethyl-amine, to give [3-(2-Chloro-phenyl)-3-(5-methyl-tetrazol-2-yl)-propyl]-methyl-amine.

1H-NMR(CDC13, 200MHz) δ2.2(m, 6H), 2.3(m, 3H), 2.5(m, 1H), 2.8(m, 1H), 7.2(m, 2H), 7.4(m, 2H)

### Example 179

### [3-(3-Chloro-phenyl)-3-(5-methyl-tetrazol-2-yl)-propyl]-methyl-amine

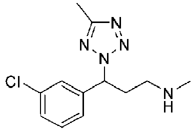

The procedure given in Example 2 was [3-(3-Chloro-phenyl)-3-(5-methyl-tetrazol-2-yl)-propyl]-dimethyl-amine as a reactant, instead of [3-(3,4-Dichloro-phenyl)-3-tetrazol-2-yl-propyl]-dimethyl-amine, to give [3-(3-Chloro-phenyl)-3-(5-methyl-tetrazol-2-yl)-propyl]-methyl-amine.

1H-NMR(CDC13, 200MHz) δ2.2(m, 6H), 2.3(m, 3H), 2.5(m, 1H), 2.8(m, 1H), 7.2(m, 2H), 7.4(m, 2H)

### Example 180

### [3-(2,4-Dichloro-phenyl)-3-(5-methyl-tetrazol-2-yl)-propyl]-methyl-amine

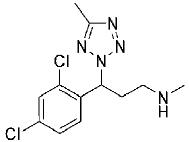

The procedure given in Example 2 was [3-(2,4-Dichloro-phenyl)-3-(5-methyl-tetrazol-2-yl)-propyl]-dimethyl-amine as a reactant, instead of [3-(3,4-Dichloro-phenyl)-3-tetrazol-2-yl-propyl]-dimethyl-amine, to give [3-(2,4-Dichloro-phenyl)-3-(5-methyl-tetrazol-2-yl)-propyl]-methyl-amine.

1H-NMR(CDC13, 200MHz) δ2.2(m, 6H), 2.3(m, 3H), 2.5(m, 1H), 2.8(m, 1H), 7.2(m, 3H)

### Example 181

### [3-(2,5-Difluoro-phenyl)-3-(5-methyl-tetrazol-2-yl)-propyl]-methyl-amine

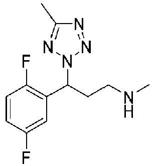

The procedure given in Example 2 was [3-(2,5-Difluoro-phenyl)-3-(5-methyl-tetrazol-2-yl)-propyl]-dimethyl-amine as a reactant, instead of [3-(3,4-Dichloro-phenyl)-3-tetrazol-2-yl-propyl]-dimethyl-amine, to give [3-(2,5-Difluoro-phenyl)-3-(5-methyl-tetrazol-2-yl)-propyl]-methyl-amine.

1H-NMR(CDC13, 200MHz) δ2.2(m, 6H), 2.3(m, 3H), 2.5(m, 1H), 2.8(m, 1H), 7.2(m, 3H)

### Example 182

### [3-(3-Chloro-phenyl)-3-tetrazol-2-yl-propyl]-methyl-amine

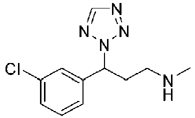

The procedure given in Example 2 was [3-(3-Chloro-phenyl)-3-tetrazol-2-yl-propyl]-dimethyl-amine as a reactant, instead of [3-(3,4-Dichloro-phenyl)-3-tetrazol-2-yl-propyl]-dimethyl-amine, to give [3-(3-Chloro-phenyl)-3-tetrazol-2-yl-propyl]-methyl-amine.

1H-NMR(CDC13, 200MHz) δ2.2(m, 6H), 2.5(m, 1H), 2.8(m, 1H), 7.2(m, 2H), 7.4(m, 2H)

### Example 183

### [3-(2,4-Dichloro-phenyl)-3-tetrazol-2-yl-propyl]-methyl-amine

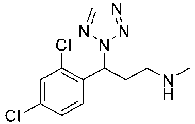

The procedure given in Example 2 was [3-(2,4-Dichloro-phenyl)-3-tetrazol-2-yl-propyl]-dimethyl-amine as a reactant, instead of [3-(3,4-Dichloro-phenyl)-3-tetrazol-2-yl-propyl]-dimethyl-amine, to give [3-(2,4-Dichloro-phenyl)-3-tetrazol-2-yl-propyl]-methyl-amine.

1H-NMR(CDC13, 200MHz) δ2.2(m, 6H), 2.3(m, 3H), 2.5(m, 1H), 2.8(m, 1H), 7.2(m, 3H)

### Example 184

### [3-(2,5-Difluoro-phenyl)-3-tetrazol-2-yl-propyl]-methyl-amine

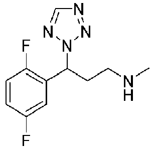

The procedure given in Example 2 was [3-(2-Chloro-phenyl)-3-tetrazol-2-yl-propyl]-dimethyl-amine as a reactant, instead of [3-(3,4-Dichloro-phenyl)-3-tetrazol-2-yl-propyl]-dimethyl-amine, to give [3-(2,5-Difluoro-phenyl)-3-tetrazol-2-yl-propyl]-methyl-amine.

1H-NMR(CDC13, 200MHz) δ2.2(m, 6H), 2.3(m, 3H), 2.5(m, 1H), 2.8(m, 1H), 7.2(m, 3H)

### Example 185

### [3-(4-Methanesulfonyl-phenyl)-3-(5-methyl-tetrazol-2-yl)-propyl]-methyl-amine

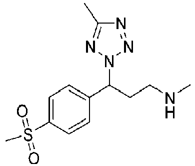

The procedure given in Example 2 was [3-(4-Methanesulfonyl-phenyl)-3-(5-methyl-tetrazol-2-yl)-propyl]-dimethyl-amine as a reactant, instead of [3-(3,4-Dichloro-phenyl)-3-tetrazol-2-yl-propyl]-dimethyl-amine, to give [3-(4-Methanesulfonyl-phenyl)-3-(5-methyl-tetrazol-2-yl)-propyl]-methyl-amine.

1H-NMR(CDC13, 200MHz) δ2.2(m, 6H), 2.3(m, 3H), 2.5(m, 1H), 2.8(m, 1H), 3.0(s, 3H), 7.2(m, 3H)

### Example 186

### [3-(4-Isopropyl-phenyl)-3-(5-methyl-tetrazol-2-yl)-propyl]-methyl-amine

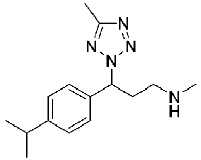

The procedure given in Example 2 was [3-(4-Isopropyl-phenyl)-3-(5-methyl-tetrazol-2-yl)-propyl]-dimethyl-amine as a reactant, instead of [3-(3,4-Dichloro-phenyl)-3-tetrazol-2-yl-propyl]-dimethyl-amine, to give [3-(4-Isopropyl-phenyl)-3-(5-methyl-tetrazol-2-yl)-propyl]-methyl-amine.

1H-NMR(CDC13, 200MHz) δ2.2(m, 6H), 2.3(m, 3H), 2.5(m, 1H), 2.8(m, 1H), 4.0(s, 6H), 7.2(m, 3H)

### Example 187

### [3-(9H-Fluoren-2-yl)-3-(5-methyl-tetrazol-2-yl)-propyl]-methyl-amine

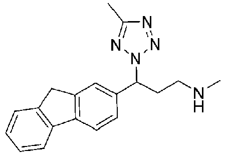

The procedure given in Example 2 was [3-(9H-Fluoren-2-yl)-3-(5-methyl-tetrazol-2-yl)-propyl]-dimethyl-amine as a reactant, instead of [3-(3,4-Dichloro-phenyl)-3-tetrazol-2-yl-propyl]-dimethyl-amine, to give [3-(9H-Fluoren-2-yl)-3-(5-methyl-tetrazol-2-yl)-propyl]-methyl-amine.

1H-NMR(CDC13, 200MHz) δ2.2(m, 6H), 2.3(m, 3H), 2.5(m, 1H), 2.8(m, 1H), 4.2(s, 2H), 7.2(m, 3H)

### Example 188

### (3S)-Methyl-[3-(5-methyl-tetrazol-2-yl)-3-naphthalen-2-yl-propyl]-amine

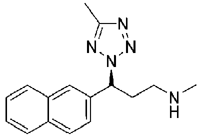

The procedure given in Example 3 was followed using Methyl-[3-(5-methyl-tetrazol-2-yl)-3-naphthalen-2-yl-propyl]-amine, instead of [3-(3,4-Dichloro-phenyl)-3-tetrazol-2-yl-propyl]-methyl-amine, to give (3S)-Methyl-[3-(5-methyl-tetrazol-2-yl)-3-naphthalen-2-yl-propyl]-amine.

1H-NMR(CDC13, 200MHz) δ2.2(m, 9H), 2.3(m, 3H), 2.5(m, 1H), 2.8(m, 1H), 4.2(s, 2H), 7.4(m, 3H), 7.8(m, 4H)

### Example 189

### (3S)-Methyl-[3-naphthalen-2-yl-3-(5-phenyl-tetrazol-2-yl)-propyl]-amine

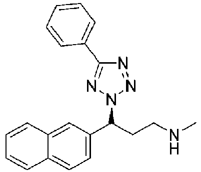

The procedure given in Example 3 was followed using Methyl-[3-naphthalen-2-yl-3-(5-phenyl-tetrazol-2-yl)-propyl]-amine, instead of [3-(3,4-Dichloro-phenyl)-3-tetrazol-2-yl-propyl]-methyl-amine, to give (3S)-Methyl-[3-naphthalen-2-yl-3-(5-phenyl-tetrazol-2-yl)-propyl]-amine.

1H-NMR(CDCl3, 200MHz) δ2.2(m, 9H), 2.3(m, 3H), 2.5(m, 1H), 2.8(m, 1H), 4.2(s, 2H), 7.4(m, 10H), 7.8(m, 4H)

### Example 190

### (3R)-Methyl-[3-(5-methyl-tetrazol-2-yl)-3-naphthalen-2-yl-propyl]-amine

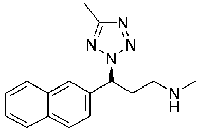

The procedure given in Example 3 was followed using Methyl-[3-(5-methyl-tetrazol-2-yl)-3-naphthalen-2-yl-propyl]-amine, instead of [3-(3,4-Dichloro-phenyl)-3-tetrazol-2-yl-propyl]-methyl-amine, to give (3R)-Methyl-[3-(5-methyl-tetrazol-2-yl)-3-naphthalen-2-yl-propyl]-amine.

1H-NMR(CDC13, 200MHz) δ2.2(m, 9H), 2.3(m, 3H), 2.5(m, 1H), 2.8(m, 1H), 4.2(s, 2H), 7.4(m, 3H), 7.8(m, 4H)

### Example 191

### (3R)-Methyl-[3-naphthalen-2-yl-3-(5-phenyl-tetrazol-2-yl)-propyl]-amine

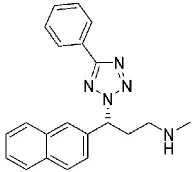

The procedure given in Example 3 was followed using Methyl-[3-naphthalen-2-yl-3-(5-phenyl-tetrazol-2-yl)-propyl]-amine, instead of [3-(3,4-Dichloro-phenyl)-3-tetrazol-2-yl-propyl]-methyl-amine, to give (3R)-Methyl-[3-naphthalen-2-yl-3-(5-phenyl-tetrazol-2-yl)-propyl]-amine.

1H-NMR(CDCl3, 200MHz) δ2.2(m, 9H), 2.3(m, 3H), 2.5(m, 1H), 2.8(m, 1H), 4.2(s, 2H), 7.4(m, 10H), 7.8(m, 4H)

### Example 192

### (3R)-[3-(6-Fluoro-naphthalen-2-yl)-3-(5-methyl-tetrazol-2-yl)-propyl]-methyl-a mine

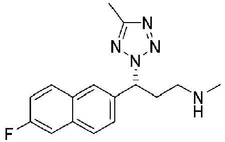

The procedure given in Example 3 was followed using [3-(6-Fluoro-naphthalen-2-yl)-3-(5-methyl-tetrazol-2-yl)-propyl]-methyl-amine, instead of [3-(3,4-Dichloro-phenyl)-3-tetrazol-2-yl-propyl]-methyl-amine, to give (3R)-[3-(6-Fluoro-naphthalen-2-yl)-3-(5-methyl-tetrazol-2-yl)-propyl]-methyl-amine.

1H-NMR(CDCl3, 200MHz) δ2.2(m, 9H), 2.3(m, 3H), 2.5(m, 1H), 2.8(m, 1H), 4.2(s, 2H), 7.4(m, 3H), 7.8(m, 4H)

### Example 193

### (3S)-[3-(6-Fluoro-naphthalen-2-yl)-3-(5-methyl-tetrazol-2-yl)-propyl]-methyl-a mine

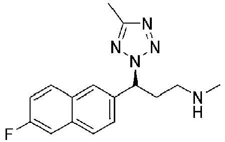

The procedure given in Example 3 was followed using [3-(6-Fluoro-naphthalen-2-yl)-3-(5-methyl-tetrazol-2-yl)-propyl]-methyl-amine, instead of [3-(3,4-Dichloro-phenyl)-3-tetrazol-2-yl-propyl]-methyl-amine, to give (3S)-[3-(6-Fluoro-naphthalen-2-yl)-3-(5-methyl-tetrazol-2-yl)-propyl]-methyl-amine.

1H-NMR(CDC13, 200MHz) δ2.2(m, 9H), 2.3(m, 3H), 2.5(m, 1H), 2.8(m, 1H), 4.2(s, 2H), 7.4(m, 3H), 7.8(m, 4H)

### Serotonin transporter reuptake inhibition assay

The method to test the ability of compounds to inhibit transporters from reuptake of serotonin followed Gu H. et al., J Biol Chem., 1994, 269, p7214∼7130.

The recombinant HEK-293 cells with human serotonin transporter were plated. Test compounds described in table 1 were pre-incubated with cells (2 x 10⁵/ml) in modified Tris-HEPES buffer pH 7.1 for 20 minutes at 25°C and then they were incubated for additional 10 minutes after 65 nM of [³H]Serotonin was added. Bound cells were filtered and counted to determine [³H]Serotonin uptake. Reduction of [³H] Serotonin uptake by 50 percent or more (≥50%) relative to 10µM fluoxetine indicates significant inhibitory activity. Compounds were screened at 10, 1, 0.1, 0.01 and 0.001µM for IC50s.

### Norepinephrine transporter reuptake inhibition assay

Norepinephrine transporter reuptake inhibition assay used the method described by Galli A. et al., J Exp Biol.,1995, 198, p2197∼2212.

MDCK cells with stably expressed human recombinant norepinephrine transporter were plated one day. Test compounds were pre-incubated with cells (2 x 10⁵/ml) in modified Tris-HEPES buffer pH 7.1 for 20 minutes at 25°C and then they were incubated for additional 10 minutes after 25 nM of [³H]Norepinephrine was added. A lysate was obtained from solubilized cells and the filtered lysate was counted to determine [³H]Norepinephrine uptake. Reduction of [³H]Norepinephrine uptake by 50 percent or more (≥50%) relative to 10µM desipramine indicates significant inhibitory activity. Compounds were screened at 10, 1, 0.1, 0.01 and 0.001µM to determine their IC50s.

### Dopamine transporter reuptake inhibition assay

The assay followed the method modified from Pristupa Z.B. et al., Mol Pharmacol., 1994, p125∼135.

CHO-K1 cells with human recombinant dopamine transporter were plated. Test compounds were pre-incubated with cells (4 x 10⁵/ml) in modified Tris-HEPES buffer pH 7.1 for 20 minutes at 25°C and then they were incubated for additional 10 minutes after 50 nM of [³H]Dopamine was added. A lysate is obtained from solubilized cells and counted to determine [³H]Dopamine uptake. Reduction of [³H]Dopamine uptake by 50 percent or more (≥50%) relative to 10 mM nomifensine indicates significant inhibitory activity. Compounds were tested at 10, 1, 0.1, 0.01 and 0.001µM for IC50s.

The results obtained by testing compounds of the invention are given in the following table 1:

**[Table 1]**

| Test Compound | Inhibition % at 100nM | | |
|---|---|---|---|
| | 5-HT reuptake | NE reuptake | DA reuptake |
| Example 1 | 27% | 37% | 58% |
| Example 2 | 35% | 56% | 48% |
| Example 6 | 41% | 30% | 20% |
| Example 14 | 46% | 32% | 18% |
| Example 21 | 44% | 39% | 36% |
| Example 50 | 61% | 12% | 7% |
| Example 51 | 61% | 10% | 6% |
| Example 119 | 78% | 84% | 10% |
| Example 122 | 93% | 55% | 3% |
| Example 129 | 43% | 84% | 62% |
| Example 135 | 47% | 17% | -1% |
| Example 136 | 59% | 25% | 6% |
| Example 137 | 58% | 39% | 10% |
| Example 166 | 88% | 70% | 4% |
| Example 167 | 82% | 84% | 3% |
| Example 188 | 82% | 79% | 7% |

The data in Table 1 show that racemic or enantiomerically enriched 3-substituted propanamine derivatives, the compounds of the invention have a significantly high inhibition potency of the serotonin, norepinephrine, dopamine transporter reuptake. This inhibition of serotonin, norepinephrine, dopamine transporter reuptake has been associated with the treatment of one or more of the CNS disorders such as depression, anxiety and pain disorder.

### Forced swimming test in mice(FST)

The Forced swimming test is an animal model based on the rodent's behavioral repertoire for screening drugs with potential antidepressant activity. As in several other models used for this goal, an uncontrollable stress stimulus produces behavioral changes that are sensitive to antidepressant treatment.

The mice were intraperitoneally treated with the test compound with an injection volume of 10 mg/kg. The group treated with 30% PEG400 served as a control group. Thirty minutes following administration, mice were individually forced to swim in a transparent glass vessel (14 cm high, 11.5 cm in diameter) filled with 10 cm of water at 25°C. The total duration of immobility (second) was measured during the last 4 minutes of a single 6-min test session. Mice were considered immobile when they made no further attempts to escape other than the movements necessary to keep their heads above the water. The potent ability of the compounds was determined as percent value of reduction in the duration of immobility comparing to the control group.

The results obtained by testing compounds of the invention are given in the following table 2 :

**[Table 2]**

| Test Compound | Reduction % at 30ip |
|---|---|
| | FST |
| Example 1 | 249% |
| Example 2 | 82.4% |
| Example 6 | 41.4% |
| Example 50 | 35.0% |
| Example 51 | 24.5% |
| Example 119 | 43.2% |
| Example 122 | 43.7% |
| Example 129 | 36.7% |
| Example 166 | 28.9% |
| Example 167 | 17.9% |
| Example 188 | 36.7% |

Specially the results of forced swimming test(FST) in mice as noted in Table 2 show the compounds of the invention are related to the treatment of depression.

### Marble burying test

The Marble burying test is a screening tool for putative anxiolytics. In this test, control mice naturally bury glass marbles in the cage bedding and the administration of anxiolytic compounds, including Diazepam, reduces the number of buried marbles. Positive compounds in the marble burying test including selective serotonin reuptake inhibitor may be especially beneficial to obsessive-compulsive disorder.

A group of mice was intraperitoneally treated with test compound dissolved in 30% PEG400 with an injection volume of 10 ml/kg. The group treated with only 30% PEG400 served as a control group. Thirty minutes after the treatment, the animals were individually placed in a polycarbonate cage which was same as used for animal housing with an open top located within a quiet room. Each cage consisted of 1/8 inch corn bedding 5 cm deep. Twenty four clean glass marbles (15 mm diameter) were evenly spaced in four rows of six on top of the bedding. Each mouse was left in the cage for 30 minutes and the number of marbles buried (buried more than 1/2 or 2/3) was counted. The potent ability of the compounds was determined as percent value of reduction in the number of marbles buried comparing to the control group.

The results obtained by testing compounds of the invention are given in the following table 3 :

**[Table 3]**

| Test Compound | Reduction % at 30ip |
|---|---|
| Example 1 | 54.8% |
| Example 2 | 100% |
| Example 6 | 87.7% |
| Example 50 | 86.2% |
| Example 51 | 72.4% |
| Example 129 | 94.3% |
| Example 167 | 56.9% (at 10ip) |
| Example 188 | 70.7% |

Specially the results of Marble burying test(MB) in mice as noted in Table 3 show the compounds of the invention are related to the treatment of anxiety.

### Acetic acid induced writhing test (AA writhing test)

The Acetic acid-induced writhing test is a well-established nociceptive test using a chemical stimulus. Although several animal models of nociceptive tests have been developed to examine and compare the anti-nociceptive effects of different drugs, the anti-nociceptive effects of antidepressants appear to be test-dependent. Indeed, the acetic acid-induced writhing test is more sensitive to antidepressants than other tests using thermal, mechanical or electrical stimuli.

The animals were subcutaneously treated with the test compound with an injection volume of 10 ml/kg. The group treated with 30% PEG400 or saline served as a control group. Thirty minutes later, the mice were intraperitoneally treated with 0.8 %(v/v) acetic acid. Each mouse was then placed in a cage for individual observation. The writhing numbers for 10 minutes were counted. The writhe is operationally defined as a contraction of the abdomen followed by stretching of the hind limbs. The potent ability of the compounds was determined as percent value of reduction in the number of writhing comparing to the control group.

The results obtained by testing compounds of the invention are given in the following table 4 :

**[Table 4]**

| Test Compound | Reduction % at 30sc |
|---|---|
| Example 1 | 55.3% |
| Example 2 | 50.3% |
| Example 6 | 60.2% |
| Example 50 | 53.8% |
| Example 51 | 51.4% |
| Example 129 | 94.4% |
| Example 167 | 52.1% |
| Example 188 | 39.5%, |

Specially the results of Acetic acid induced writhing test(AA writhing test) in mice as noted in Table 4 show the compounds of the invention are related to the treatment of pain.

## Claims

1. A racemic or enantiomerically enriched 3-substituted propanamine compound represented by the following structural formula (I): 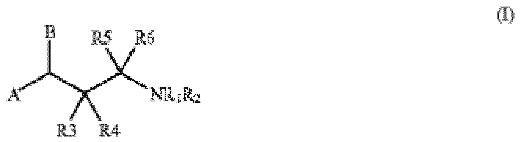 or a pharmaceutically acceptable salt thereof, wherein;
A is selected from the group consisting of phenyl, naphthyl, benzothienyl, pyridyl, quinolyl, isoquinolyl, benzodioxolyl, benzodioxinyl, indolyl, fluorenyl and benzofuranyl, which can be substituted with one or more identical or different substituents selected from the group consisting of hydrogen, halogen, straight- or branched-chain C₁₋₄alkyl, straight- or branched-chain C₁₋₃alkoxy, phenyl, phenyloxy, nitro, cyano, trifluoromethyl, trifluoromethoxy, methanesulfonyl and thienyl;
R₁ and R₂ are same or different and independently selected from the group consisting of hydrogen, lower alkyl of from 1 to 4 carbon atoms, substituted or unsubstituted phenyl-C₁₋₄ alkyl or R₁ and R₂ are fused with nitrogen atom to form a cyclic group which has 4 to 7 carbon atoms;
R₃, R₄, R₅ and R₆ are same or different and independently selected from the group consisting of hydrogen, C₁₋₄alkyl and substituted or unsubstituted phenyl;
B is an azole selected from the group consisting of triazole, benzotriazole, tetrazole, 5-methyl tetrazole and 5-phenyl tetrazole which are linked by nitrogen as represented by the following structural formulae (II):

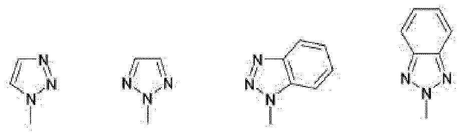

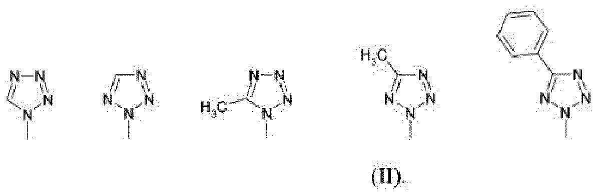

2. A racemic or enantiomerically enriched 3-substituted propanamine compound in accordance with claim 1 wherein A, B, R₁ and R₂ are as defined therein and each of R₃ through R₆ is hydrogen, said compound being represented by the following structural formula (III):

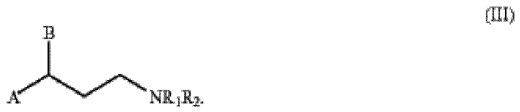

3. A racemic or enantiomerically enriched 3-substituted propanamine compound in accordance with claim 1 wherein A, B are as defined therein, each of R₃ through R₆ is hydrogen and one of R₁ and R₂ is hydrogen and the other is methyl, said compound being represented by the following structural formula (XI):

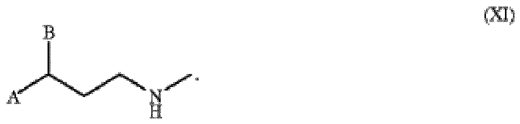

4. 3-Substituted propanamine compounds in accordance with claim 2 wherein said compounds are selected from the group consisting of:
[3-(3,4-Dichloro-phenyl)-3-tetrazol-2-yl-propyl]-methyl-amine
S-[3-(3,4-Dichloro-phenyl)-3-tetrazol-2-yl-propyl]-methyl-amine
[3-Benzotriazol-2-yl-3-(3,4-dichloro-phenyl)-propyl]-dimethyl-amine
Dimethyl-(3-naphthalen-2-yl-3-[1,2,3]triazol-2-yl-propyl)-amine
Dimethyl-(3-naphthalen-2-yl-3-tetrazol-2-yl-propyl)-amine
(3-Benzotriazol-2-yl-3-naphthalen-2-yl-propyl)-dimethyl-amine
Dimethyl-[3-(5-methyl-tetrazol-2-yl)-3-naphthalen-2-yl-propyl]-amine
[3-(6-Chloro-naphthalen-2-yl)-3-tetrazol-1-yl-propyl]-dimethyl-amine
Dimethyl-[3-(4-phenoxy-phenyl)-3-tetrazol-2-yl-propyl]-amine
Dimethyl-[3-(4-phenoxy-phenyl)-3-tetrazol-1-yl-propyl]-amine
[3-(6-Chloro-naphthalen-2-yl)-3-[1,2,3]triazol-2-yl-propyl]-dimethyl-amine
[3-(6-Chloro-naphthalen-2-yl)-3-[1,2,3]triazol-1-yl-propyl]-dimethyl-amine
[3-(6-Chloro-naphthalen-2-yl)-3-tetrazol-2-yl-propyl]-dimethyl-amine
[3-(4-Methoxy-phenyl)-3-[1,2,3]triazol-2-yl-propyl]-dimethyl-amine
(3-Biphenyl-3-yl-3-tetrazol-2-yl-propyl)-dimethyl-amine
[3-(4-Benzyloxy-phenyl)-3-tetrazol-2-yl-propyl]-dimethyl-amine
(3-Biphenyl-4-yl-3-tetrazol-2-yl-propyl)-dimethyl-amine
Dimethyl-[3-naphthalen-2-yl-3-(5-phenyl-tetrazol-2-yl)-propyl]-amine
Dimethyl-[3-(6-methyl-naphthalen-2-yl)-3-tetrazol-2-yl-propyl]-amine
[3-(6-Methoxy-naphthalen-2-yl)-3-tetrazol-2-yl-propyl]-dimethyl-amine
[3-(6-Methoxy-naphthalen-2-yl)-3-[1,2,3]triazol-2-yl-propyl]-dimethyl-amine
[3-Benzotriazol-1-yl-3-(3,4-dichloro-phenyl)-propyl]-dimethyl-amine
2-[1-(3,4-Dichloro-phenyl)-3-pyrrolidin-1-yl-propyl]-2H-[1,2,3]triazole
[3-(3,4-Dichloro-phenyl)-3-[1,2,3]triazol-2-yl-propyl]-diethyl-amine
[3-(3,4-Dichloro-phenyl)-3-tetrazol-2-yl-propyl]-diethyl-amine
2-[1-(3,4-Dichloro-phenyl)-3-pyrrolidin-1-yl-propyl]-2H-tetrazole
Dimethyl-(3-naphthalen-1-yl-3-tetrazol-2-yl-propyl)-amine
Dimethyl-(3-naphthalen-1-yl-3-[1,2,3]triazol-2-yl-propyl)-amine
[3-(3,4-Dichloro-phenyl)-3-(5-methyl-tetrazol-2-yl)-propyl]-dimethyl-amine
[3-(3,4-Dichloro-phenyl)-3-(5-phenyl-tetrazol-2-yl)-propyl]-dimethyl-amine
[3-(3,4-Difluoro-phenyl)-3-tetrazol-1-yl-propyl]-dimethyl-amine
[3-(3,4-Difluoro-phenyl)-3-tetrazol-2-yl-propyl]-dimethyl-amine
[3-(3,4-Difluoro-phenyl)-3-[1,2,3]triazo1-1-yl-propyl]-dimethyl-amine
[3-(3,4-Difluoro-phenyl)-3-[1,2,3]triazo1-2-yl-propyl]-dimethyl-amine
[3-(3,4-Dimethyl-phenyl)-3-[1,2,3]triazol-1-yl-propyl]-dimethyl-amine
[3-(3,4-Dimethyl-phenyl)-3-[1,2,3]triazol-2-yl-propyl]-dimethyl-amine
[3-(3,4-Dimethyl-phenyl)-3-tetrazol-1-yl-propyl]-dimethyl-amine
[3-(3,4-Dimethyl-phenyl)-3-tetrazol-2-yl-propyl]-dimethyl-amine
[3-(4-Chloro-phenyl)-3-tetrazol-1-yl-propyl]-dimethyl-amine
[3-(4-Chloro-phenyl)-3-tetrazol-2-yl-propyl]-dimethyl-amine
Dimethyl-(3-phenyl-3-[1,2,3]triazol-1-yl-propyl)-amine
Dimethyl-(3-phenyl-3-[1,2,3]triazol-2-yl-propyl)-amine
[3-(4-Chloro-phenyl)-3-[1,2,3]triazol-1-yl-propyl]-dimethyl-amine
[3-(4-Chloro-phenyl)-3-[1,2,3]triazol-2-yl-propyl]-dimethyl-amine
[3-(3,4-Dichloro-phenyl)-3-[1,2,3]triazol-2-yl-propyl]-dimethyl-amine
[3-(3-Methoxy-naphthalen-2-yl)-3-tetrazol-2-yl-propyl]-dimethyl-amine
[3-(3-Methoxy-naphthalen-2-yl)-3-(5-methyl-tetrazol-2-yl)-propyl]-dimethyl-amine
[3-(6-Fluoro-naphthalen-2-yl)-3-tetrazol-2-yl-propyl]-dimethyl-amine
[3-(6-Fluoro-naphthalen-2-yl)-3-(5-methyl-tetrazol-2-yl)-propyl]-dimethyl-amine
[3-(1,4-Dimethoxy-naphthalen-2-yl)-3-tetrazol-2-yl-propyl]-dimethyl-amine
[3-(1,4-Dimethoxy-naphthalen-2-yl)-3-(5-methyl-tetrazol-2-yl)-propyl]-dimethyl-amine
[3-(3,5-Dimethoxy-naphthalen-2-yl)-3-tetrazol-2-yl-propyl]-dimethyl-amine
[3-(3,5-Dimethoxy-naphthalen-2-yl)-3-(5-methyl-tetrazol-2-yl)-propyl]-dimethyl-amine
[3-(3,5-Dimethoxy-naphthalen-2-yl)-3-tetrazol-1-yl-propyl]-dimethyl-amine
Dimethyl-[3-(4-methyl-naphthalen-1-yl)-3-tetrazol-2-yl-propyl]-amine
Dimethyl-[3-(4-methyl-naphthalen-1-yl)-3-(5-methyl-tetrazol-2-yl)-propyl]-amine
[3-(4-Fluoro-naphthalen-1-yl)-3-tetrazol-2-yl-propyl]-dimethyl-amine
[3-(4-Fluoro-naphthalen-1-yl)-3-(5-methyl-tetrazol-2-yl)-propyl]-dimethyl-amine
(3-Isoquinolin-1-yl-3-tetrazol-2-yl-propyl)-dimethyl-amine
[3-Isoquinolin-1-yl-3-(5-methyl-tetrazol-2-yl)-propyl]-dimethyl-amine
(3-Benzo[1,3]dioxol-5-yl-3-tetrazol-2-yl-propyl)-dimethyl-amine
[3-Benzo[1,3]dioxol-5-yl-3-(5-methyl-tetrazol-2-yl)-propyl]-dimethyl-amine
[3-Benzo[1,3]dioxol-5-yl-3-(5-methyl-tetrazol-1-yl)-propyl]-dimethyl-amine
[3-(3,4-Dimethoxy-phenyl)-3-tetrazol-2-yl-propyl]-dimethyl-amine
[3-(2,3-Dihydro-benzo[1,4]dioxin-2-yl)-3-tetrazol-2-yl-propyl]-dimethyl-amine
[3-(2,3-Dihydro-benzo[1,4]dioxin-2-yl)-3-(5-methyl-tetrazol-2-yl)-propyl]-dimethyl-amine
Dimethyl-(3-quinolin-2-yl-3-tetrazol-2-yl-propyl)-amine
Dimethyl-[3-(5-methyl-tetrazol-2-yl)-3-quinolin-2-yl-propyl]-amine
Dimethyl-[3-(1-methyl-1H-indol-2-yl)-3-tetrazol-2-yl-propyl]-amine
Dimethyl-[3-(1-methyl-1H-indol-2-yl)-3-(5-methyl-tetrazol-2-yl)-propyl]-amine
Dimethyl-[3-(1,2,3,4-tetrahydro-naphthalen-2-yl)-3-tetrazol-2-yl-propyl]-amine
[3-(6-Chloro-pyridin-3-yl)-3-tetrazol-2-yl-propyl]-dimethyl-amine
(3-Benzo[b]thiophen-2-yl-3-tetrazol-2-yl-propyl)-dimethyl-amine
[3-Benzo[b]thiophen-2-yl-3-(5-methyl-tetrazol-2-yl)-propyl]-dimethyl-amine
(3-Benzofuran-2-yl-3-tetrazol-2-yl-propyl)-dimethyl-amine
[3-Benzofuran-2-yl-3-(5-methyl-tetrazol-2-yl)-propyl]-dimethyl-amine
(3-Benzofuran-2-yl-3-tetrazol-1-yl-propyl)-dimethyl-amine
[3-(3,4-Difluoro-phenyl)-3-(5-methyl-tetrazol-2-yl)-propyl]-dimethyl-amine
Dimethyl-[3-tetrazol-2-yl-3-(2,4,5-trifluoro-phenyl)-propyl]-amine
Dimethyl-[3-(5-methyl-tetrazol-2-yl)-3-(2,4,5-trifluoro-phenyl)-propyl]-amine
[3-(3-Bromo-4-fluoro-phenyl)-3-tetrazol-2-yl-propyl]-dimethyl-amine
[3-(3-Bromo-4-fluoro-phenyl)-3-(5-methyl-tetrazol-2-yl)-propyl]-dimethyl-amine
[3-(6-Methoxy-naphthalen-2-yl)-3-(5-methyl-tetrazol-2-yl)-propyl]-dimethyl-amine
Dimethyl-[3-(5-methyl-tetrazol-2-yl)-3-(4-phenoxy-phenyl)-propyl]-amine
[3-(4-Bromo-phenyl)-3-tetrazol-2-yl-propyl]-dimethyl-amine
[3-(4-Bromo-phenyl)-3-(5-methyl-tetrazol-2-yl)-propyl]-dimethyl-amine
Dimethyl-[3-tetrazol-2-yl-3-(4-trifluoromethyl-phenyl)-propyl]-amine
Dimethyl-[3-(5-methyl-tetrazol-2-yl)-3-(4-trifluoromethyl-phenyl)-propyl]-amine
Dimethyl-[3-tetrazol-2-yl-3-(2,3,4-trichloro-phenyl)-propyl]-amine
Dimethyl-[3-(5-methyl-tetrazol-2-yl)-3-(2,3,4-trichloro-phenyl)-propyl]-amine
[3-(3-Chloro-4-methoxy-phenyl)-3-tetrazol-2-yl-propyl]-dimethyl-amine
[3-(3-Chloro-4-methoxy-phenyl)-3-(5-methyl-tetrazol-2-yl)-propyl]-dimethyl-amine
[3-(4-tert-Butyl-phenyl)-3-(5-methyl-tetrazol-2-yl)-propyl]-dimethyl-amine
[3-(2,5-Difluoro-phenyl)-3-(5-methyl-tetrazol-2-yl)-propyl]-dimethyl-amine
[3-(2,4-Dichloro-phenyl)-3-(5-methyl-tetrazol-2-yl)-propyl]-dimethyl-amine
[3-(3-Chloro-phenyl)-3-(5-methyl-tetrazol-2-yl)-propyl]-dimethyl-amine
[3-(2-Chloro-phenyl)-3-(5-methyl-tetrazol-2-yl)-propyl]-dimethyl-amine
[3-(2,5-Difluoro-phenyl)-3-tetrazol-2-yl-propyl]-dimethyl-amine
[3-(2,4-Dichloro-phenyl)-3-tetrazol-2-yl-propyl]-dimethyl-amine
[3-(3-Chloro-phenyl)-3-tetrazol-2-yl-propyl]-dimethyl-amine
Dimethyl-[3-(5-methyl-tetrazol-2-yl)-3-(4-trifluoromethoxy-phenyl)-propyl]-amine
[3-(3-Bromo-4-methyl-phenyl)-3-(5-methyl-tetrazol-2-yl)-propyl]-dimethyl-amine
Dimethyl-[3-tetrazol-2-yl-3-(4-trifluoromethoxy-phenyl)-propyl]-amine
[3-(4-Bromo-3-methyl-phenyl)-3-tetrazol-2-yl-propyl]-dimethyl-amine
[3-(4-Methanesulfonyl-phenyl)-3-(5-methyl-tetrazol-2-yl)-propyl]-dimethyl-amine
[3-(4-Isopropyl-phenyl)-3-(5-methyl-tetrazol-2-yl)-propyl]-dimethyl-amine
[3-(9H-Fluoren-2-yl)-3-(5-methyl-tetrazol-2-yl)-propyl]-dimethyl-amine
Methyl-(3-naphthalen-2-yl-3-[1,2,3]triazol-2-yl-propyl)-amine
[3-Benzotriazol-2-yl-3-(3,4-dichloro-phenyl)-propyl]-methyl-amine
Methyl-[3-(5-methyl-tetrazol-2-yl)-3-naphthalen-2-yl-propyl]-amine
(3-Benzotriazol-2-yl-3-naphthalen-2-yl-propyl)-methyl-amine
(3-Biphenyl-4-yl-3-tetrazol-2-yl-propyl)-methyl-amine
[3-(3,4-Dichloro-phenyl)-3-(5-methyl-tetrazol-2-yl)-propyl]-methyl-amine
[3-(4-Chloro-phenyl)-3-tetrazol-2-yl-propyl]-methyl-amine
[3-(3,4-Dichloro-phenyl)-3-[1,2,3]triazol-2-yl-propyl]-methyl-amine
[3-(3,4-Dichloro-phenyl)-3-tetrazol-2-yl-propyl]-methyl-amine
Methyl-(3-naphthalen-2-yl-3-tetrazol-2-yl-propyl)-amine
[3-(6-Chloro-naphthalen-2-yl)-3-tetrazol-2-yl-propyl]-methyl-amine
Methyl-(3-naphthalen-1-yl-3-tetrazol-2-yl-propyl)-amine
Methyl-[3-(6-methyl-naphthalen-2-yl)-3-tetrazol-2-yl-propyl]-amine
Methyl-[3-naphthalen-2-yl-3-(5-phenyl-tetrazol-2-yl)-propyl]-amine
(3*S*)-3-(3,4-dichlorophenyl)-*N*-methyl-3-(2*H*-tetrazol-2-yl)propan-1-amine
(3*R*)-3-(3,4-dichlorophenyl)-*N*-methyl-3-(2*H*-tetrazol-2-yl)propan-1-amine
(3*S*)-3-(2H-benzotriazol-2-yl)-3-(3,4-dichlorophenyl)-*N*-methylpropan-1-amine
(3*R*)-3-(2H-benzotriazol-2-yl)-3-(3,4-dichlorophenyl)-*N*-methylpropan-1-amine
(3*S*)-3-(3,4-dichlorophenyl)-*N*-methyl-3-(2*H*-1,2,3-triazol-2-yl)propan-1-amine
(3*S*)-3-(3,4-dichlorophenyl)-*N*-methyl-3-(5-methyl-2*H*-tetrazol-2-yl)propan-1-amine
(3*R*)-3-(3,4-dichlorophenyl)-*N*-methyl-3-(5-methyl-2*H*-tetrazol-2-yl)propan-1-amine
(3*R*)-3-(3,4-dichlorophenyl)-*N*-methyl-3-(2*H*-1,2,3-triazol-2-yl)propan-1-amine
(3*R*)-*N*-methyl-3-(2-naphthyl)-3-(2*H*-tetrazol-2-yl)propan-1-amine
(3*S*)-*N*-methyl-3-(2-naphthyl)-3-(2*H*-tetrazol-2-yl)propan-1-amine
[3-(3-Methoxy-naphthalen-2-yl)-3-tetrazol-2-yl-propyl]-methyl-amine
[3-(3-Methoxy-naphthalen-2-yl)-3-(5-methyl-tetrazol-2-yl)-propyl]-methyl-amine
[3-(6-Fluoro-naphthalen-2-yl)-3-tetrazol-2-yl-propyl]-methyl-amine
[3-(6-Fluoro-naphthalen-2-yl)-3-(5-methyl-tetrazol-2-yl)-propyl]-methyl-amine
[3-(1,4-Dimethoxy-naphthalen-2-yl)-3-tetrazol-2-yl-propyl]-methyl-amine
[3-(1,4-Dimethoxy-naphthalen-2-yl)-3-(5-methyl-tetrazol-2-yl)-propyl]-methyl-amine
[3-(3,5-Dimethoxy-naphthalen-2-yl)-3-tetrazol-2-yl-propyl]-methyl-amine
[3-(3,5-Dimethoxy-naphthalen-2-yl)-3-(5-methyl-tetrazol-2-yl)-propyl]-methyl-amine
[3-(3,5-Dimethoxy-naphthalen-2-yl)-3-tetrazol-1-yl-propyl]-methyl-amine
Methyl-[3-(4-methyl-naphthalen-1-yl)-3-tetrazol-2-yl-propyl]-amine
Methyl-[3-(4-methyl-naphthalen-1-yl)-3-(5-methyl-tetrazol-2-yl)-propyl]-amine
[3-(4-Fluoro-naphthalen-1-yl)-3-tetrazol-2-yl-propyl]-methyl-amine
[3-(4-Fluoro-naphthalen-1-yl)-3-(5-methyl-tetrazol-2-yl)-propyl]-methyl-amine
(3-Benzo[1,3]dioxol-5-yl-3-tetrazol-2-yl-propyl)-methyl-amine
[3-Benzo[1,3]dioxol-5-yl-3-(5-methyl-tetrazol-2-yl)-propyl]-methyl-amine
[3-Benzo[1,3]dioxol-5-yl-3-(5-methyl-tetrazol-1-yl)-propyl]-methyl-amine
[3-(3,4-Dimethoxy-phenyl)-3-tetrazol-2-yl-propyl]-methyl-amine
[3-(2,3-Dihydro-benzo[1,4]dioxin-2-yl)-3-tetrazol-2-yl-propyl]-methyl-amine
[3-(2,3-Dihydro-benzo[1,4]dioxin-2-yl)-3-(5-methyl-tetrazol-2-yl)-propyl]-methyl-amine
Methyl-[3-(1,2,3,4-tetrahydro-naphthalen-2-yl)-3-tetrazol-2-yl-propyl]-amine
[3-(6-Chloro-pyridin-3-yl)-3-tetrazol-2-yl-propyl]-methyl-amine
(3-Benzo[b]thiophen-2-yl-3-tetrazol-2-yl-propyl)-methyl-amine
[3-Benzo[b]thiophen-2-yl-3-(5-methyl-tetrazol-2-yl)-propyl]-methyl-amine
(3-Benzofuran-2-yl-3-tetrazol-2-yl-propyl)-methyl-amine
[3-Benzofuran-2-yl-3-(5-methyl-tetrazol-2-yl)-propyl]-methyl-amine
(3-Benzofuran-2-yl-3-tetrazol-1-yl-propyl)-methyl-amine
[3-(3,4-Difluoro-phenyl)-3-tetrazol-2-yl-propyl]-methyl-amine
[3-(3,4-Difluoro-phenyl)-3-(5-methyl-tetrazol-2-yl)-propyl]-methyl-amine
Methyl-[3-tetrazol-2-yl-3-(2,4,5-trifluoro-phenyl)-propyl]-amine
Methyl-[3-(5-methyl-tetrazol-2-yl)-3-(2,4,5-trifluoro-phenyl)-propyl]-amine
[3-(3-Bromo-4-fluoro-phenyl)-3-tetrazol-2-yl-propyl]-methyl-amine
[3-(3-Bromo-4-fluoro-phenyl)-3-(5-methyl-tetrazol-2-yl)-propyl]-methyl-amine
[3-(6-Methoxy-naphthalen-2-yl)-3-tetrazol-2-yl-propyl]-methyl-amine
[3-(6-Methoxy-naphthalen-2-yl)-3-(5-methyl-tetrazol-2-yl)-propyl]-methyl-amine
Methyl-[3-(4-phenoxy-phenyl)-3-tetrazol-2-yl-propyl]-amine
Methyl-[3-(5-methyl-tetrazol-2-yl)-3-(4-phenoxy-phenyl)-propyl]-amine
[3-(4-Bromo-phenyl)-3-tetrazol-2-yl-propyl]-methyl-amine
[3-(4-Bromo-phenyl)-3-(5-methyl-tetrazol-2-yl)-propyl]-methyl-amine
Methyl-[3-tetrazol-2-yl-3-(4-trifluoromethyl-phenyl)-propyl]-amine
Methyl-[3-(5-methyl-tetrazol-2-yl)-3-(4-trifluoromethyl-phenyl)-propyl]-amine
Methyl-[3-tetrazol-2-yl-3-(2,3,4-trichloro-phenyl)-propyl]-amine
Methyl-[3-(5-methyl-tetrazol-2-yl)-3-(2,3,4-trichloro-phenyl)-propyl]-amine
[3-(3-Chloro-4-methoxy-phenyl)-3-tetrazol-2-yl-propyl]-methyl-amine
[3-(3-Chloro-4-methoxy-phenyl)-3-(5-methyl-tetrazol-2-yl)-propyl]-methyl-amine
[3-(2-Chloro-phenyl)-3-(5-methyl-tetrazol-2-yl)-propyl]-methyl-amine
[3-(3-Chloro-phenyl)-3-(5-methyl-tetrazol-2-yl)-propyl]-methyl-amine
[3-(2,4-Dichloro-phenyl)-3-(5-methyl-tetrazol-2-yl)-propyl]-methyl-amine
[3-(2,5-Difluoro-phenyl)-3-(5-methyl-tetrazol-2-yl)-propyl]-methyl-amine
[3-(3-Chloro-phenyl)-3-tetrazol-2-yl-propyl]-methyl-amine
[3-(2,4-Dichloro-phenyl)-3-tetrazol-2-yl-propyl]-methyl-amine
[3-(2,5-Difluoro-phenyl)-3-tetrazol-2-yl-propyl]-methyl-amine
[3-(4-Methanesulfonyl-phenyl)-3-(5-methyl-tetrazol-2-yl)-propyl]-methyl-amine
[3-(4-Isopropyl-phenyl)-3-(5-methyl-tetrazol-2-yl)-propyl]-methyl-amine
[3-(9H-Fluoren-2-yl)-3-(5-methyl-tetrazol-2-yl)-propyl]-methyl-amine
(3S)-Methyl-[3-(5-methyl-tetrazol-2-yl)-3-naphthalen-2-yl-propyl]-amine
(3S)-Methyl-[3-naphthalen-2-yl-3-(5-phenyl-tetrazol-2-yl)-propyl]-amine
(3R)-Methyl-[3-(5-methyl-tetrazol-2-yl)-3-naphthalen-2-yl-propyl]-amine
(3R)-Methyl-[3-naphthalen-2-yl-3-(5-phenyl-tetrazol-2-yl)-propyl]-amine
(3R)-[3-(6-Fluoro-naphthalen-2-yl)-3-(5-methyl-tetrazol-2-yl)-propyl]-methyl-amine and
(3S)-[3-(6-Fluoro-naphthalen-2-yl)-3-(5-methyl-tetrazol-2-yl)-propyl]-methyl-amine.

5. A compound in accordance with claim 3 wherein said compound is selected from the group consisting of:
[3-(3,4-Dichloro-phenyl)-3-tetrazol-2-yl-propyl]-methyl-amine,
(R)-[3-(3,4-Dichloro-phenyl)-3-tetrazol-2-yl-propyl]-methyl-amine,
(S)-[3-(3,4-Dichloro-phenyl)-3-tetrazol-2-yl-propyl]-methyl-amine,
[3-(3,4-Dichloro-phenyl)-3-(5-methyl-tetrazol-2-yl)-propyl]-methyl-amine,
(R)-[3-(3,4-Dichloro-phenyl)-3-(5-methyl-tetrazol-2-yl)-propyl]-methyl-amine,
(S)-[3-(3,4-Dichloro-phenyl)-3-(5-methyl-tetrazol-2-yl)-propyl]-methyl-amine,
[3-(3,4-Dichloro-phenyl)-3-[1,2,3]triazol-2-yl-propyl]-methyl-amine,
(R)-[3-(3,4-Dichloro-phenyl)-3-[1,2,3]triazol-2-yl-propyl]-methyl-amine,
(S)-[3-(3,4-Dichloro-phenyl)-3-[1,2,3]triazol-2-yl-propyl]-methyl-amine,
Methyl-(3-naphthalen-2-yl-3-tetrazol-2-yl-propyl)-amine,
(R)-Methyl-(3-naphthalen-2-yl-3-tetrazol-2-yl-propyl)-amine, and
(S)-Methyl-(3-naphthalen-2-yl-3-tetrazol-2-yl-propyl)-amine.

6. A pharmaceutical composition which comprises an amount of a racemic or enantiomerically enriched compound of formula (I) in accordance with claim 1 effective for use in treating depression, anxiety and pain disorders.

7. A pharmaceutical composition which comprises an amount of a racemic or enantiomerically enriched compound of formula (III) in accordance with claim 2 effective for use in treating depression, anxiety and pain disorders.

8. A pharmaceutical composition which comprises an amount of a racemic or enantiomerically enriched compound of formula (XI) in accordance with claim 3 effective for use treating depression, anxiety and pain disorders.

9. Racemic or enantiomerically enriched compound of formula (I) according to claim 1, for use in a method of treating depression, anxiety and pain disorders in a mammal in need thereof

10. Racemic or enantiomerically enriched compound of formula (III) according to claim 2, for use in a method of treating depression anxiety and pain disorders, in a mammal in need thereof

11. Racemic or enantiomerically enriched compound of formula (XI) according to claim 3, for use in a method of treating depression, anxiety and pain disorders in a mammal in need thereof.

## Patentansprüche

1. Racemische oder Enantiomer-angereicherte 3-substituierte Propanamin-Verbindung, dargestellt durch die folgende Strukturformel (I): 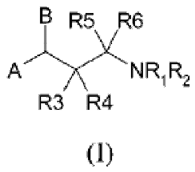 oder ein pharmazeutisch verträgliches Salz davon, wobei:
A ausgewählt ist aus der Gruppe bestehend aus Phenyl, Naphthyl, Benzothienyl, Pyridyl, Chinolyl, Isochinolyl, Benzodioxolyl, Benzodioxinyl, Indolyl, Fluorenyl und Benzofuranyl, das mit einem oder mehreren gleichen oder verschiedenen Substituenten ausgewählt aus der Gruppe bestehend aus Wasserstoff, Halogen, gerad- oder verzweigtkettiges C₁₋₄-Alkyl, gerad- oder verzweigtkettigen C₁₋₃-Alkoxy, Phenyl, Phenyloxy, Nitro, Cyano, Trifluormethyl, Trifluormethoxy, Methansulfonyl und Thienyl, substituiert sein kann;
R₁ und R₂ gleich oder verschieden und unabhängig ausgewählt sind aus der Gruppe bestehend aus Wasserstoff, Niederalkyl mit 1-4 Kohlenstoffatomen, substituiertem oder unsubstituiertem Phenyl-C₁₋₄-Alkyl oder R₁ und R₂ verbunden mit dem Stickstoffatom eine cyclische Gruppe mit 4 bis 7 Kohlenstoffatomen bilden;
R₃, R₄, R₅ und R₆ gleich oder verschieden und unabhängig ausgewählt sind aus der Gruppe bestehend aus Wasserstoff, C₁₋₄-Alkyl und substituiertem oder unsubstituiertem Phenyl;
B ein Azol ist ausgewählt aus der Gruppe bestehend aus Triazol, Benzotriazol, Tetrazol, 5-Methyltetrazol und 5-Phenyltetrazol, die durch Stickstoff verbunden sind, wie durch die folgenden Strukturformeln (II) dargestellt:

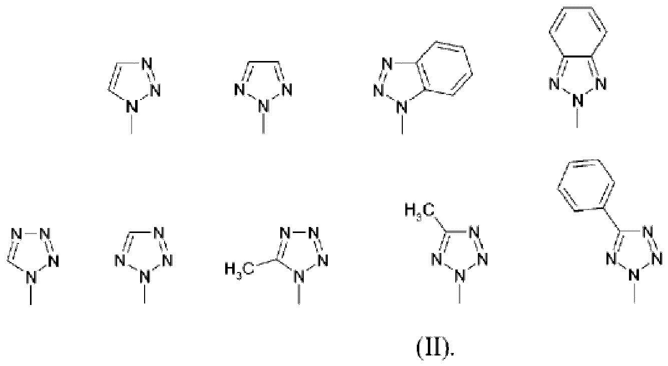

2. Racemische oder Enantiomer-angereicherte 3-substituierte Propanamin-Verbindung nach Anspruch 1, wobei A, B, R₁ und R₂ wie dort definiert sind, und jeder von R₃ bis R₆ Wasserstoff ist, wobei die Verbindung durch die folgende Strukturformel (III) dargestellt ist:

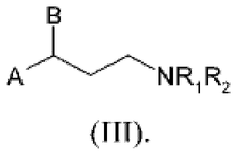

3. Racemische oder Enantiomer-angereicherte 3-substituierte Propanamin-Verbindung nach Anspruch 1, wobei A, B wie dort definiert sind, jeder von R₃ bis R₆ Wasserstoff und einer von R₁ und R₂ Wasserstoff und der andere Methyl ist, wobei die Verbindung durch die folgende Strukturformel (XI) dargestellt ist:

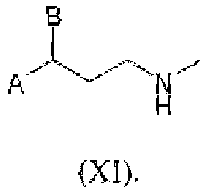

4. 3-substituierte Propanamin-Verbindungen nach Anspruch 2, wobei die Verbindungen ausgewählt sind der Gruppe bestehend aus:
[3-(3,4-Dichlorophenyl)-3-tetrazol-2-yl-propyl]-methylamin
S-[3-(3,4-Dichlorophenyl)-3-tetrazol-2-yl-propyl]-methylamin
[3-Benzotriazol-2-yl-3-(3,4-dichlorophenyl)-propyl]-dimethylamin
Dimethyl-(3-naphthalen-2-yl-3-[1,2,3]triazol-2-yl-propyl)-amin
Dimethyl-(3-naphthalen-2-yl-3-tetrazol-2-yl-propyl)-amin
(3-Benzotriazol-2-yl-3-naphthalen-2-yl-propyl)-dimethylamin
Dimethyl-[3-(5-methyltetrazol-2-yl)-3-naphthalen-2-yl-propyl]-amin
[3-(6-Chloronaphthalen-2-yl)-3-tetrazol-1-yl-propyl]-dimethylamin
Dimethyl-[3-(4-phenoxyphenyl)-3-tetrazol-2-yl-propyl]-amin
Dimethyl- [3-(4-phenoxyphenyl)-3-tetrazol-1-yl-propyl]-amin
[3-(6-Chloronaphthalen-2-yl)-3-[1,2,3]triazol-2-yl-propyl]-dimethylamin
[3-(6-Chloronaphthalen-2-yl)-3-[1,2,3]triazol-1-yl-propyl]-dimethylamin
[3-(6-Chloronaphthalen-2-yl)-3-tetrazol-2-yl-propyl]-dimethylamin
[3-(4-Methoxyphenyl)-3-[1,2,3]triazo1-2-yl-propyl]-dimethylamin
(3-Biphenyl-3-yl-3-tetrazol-2-yl-propyl)-dimethylamin
[3-(4-Benzyloxy-phenyl)-3-tetrazol-2-yl-propyl]-dimethylamin
(3-Biphenyl-4-yl-3-tetrazol-2-yl-propyl)-dimethylamin
Dimethyl-[3-naphthalen-2-yl-3-(5-phenyltetrazol-2-yl)-propyl]-amin
Dimethyl-[3-(6-methylnaphthalen-2-yl)-3-tetrazol-2-yl-propyl]-amin
[3-(6-Methoxynaphthalen-2-yl)-3-tetrazol-2-yl-propyl]-dimethylamin
[3-(6-Methoxynaphthalen-2-yl)-3-[1,2,3]triazol-2-yl-propyl]-dimethylamin
[3-Benzotriazol-1-yl-3-(3,4-dichlorophenyl)-propyl]-dimethylamin
2-[1-(3,4-Dichlorophenyl)-3-pyrrolidin-1-yl-propyl]-2H-[1,2,3]triazol
[3-(3,4-Dichlorophenyl)-3-[1,2,3]triazol-2-yl-propyl]-diethylamin
[3-(3,4-Dichlorophenyl)-3-tetrazol-2-yl-propyl]-diethylamin
2-[1-(3,4-Dichlorophenyl)-3-pyrrolidin-1-yl-propyl]-2H-tetrazol
Dimethyl-(3-naphthalen-1-yl-3-tetrazol-2-yl-propyl)-amin
Dimethyl-(3-naphthalen-1-yl-3-[1,2,3]triazol-2-yl-propyl)-amin
[3-(3,4-Dichlorophenyl)-3-(5-methyltetrazol-2-yl)-propyl]-dimethylamin
[3-(3,4-Dichlorophenyl)-3-(5-phenyltetrazol-2-yl)-propyl]-dimethylamin
[3-(3,4-Difluorophenyl)-3-tetrazol-1-yl-propyl]-dimethylamin
[3-(3,4-Difluorophenyl)-3-tetrazol-2-yl-propyl]-dimethylamin
[3-(3,4-Difluorophenyl)-3-[1,2,3]triazol-1-yl-propyl]-dimethylamin
[3-(3,4-Difluorophenyl)-3-[1,2,3]triazol-2-yl-propyl]-dimethylamin
[3-(3,4-Dimethylphenyl)-3-[1,2,3]triazol-1-yl-propyl]-dimethylamin
[3-(3,4-Dimethylphenyl)-3-[1,2,3]triazol-2-yl-propyl]-dimethylamin
[3-(3,4-Dimethylphenyl)-3-tetrazol-1-yl-propyl]-dimethylamin
[3-(3,4-Dimethylphenyl)-3-tetrazol-2-yl-propyl]-dimethylamin
[3-(4-Chlorophenyl)-3-tetrazol-1-yl-propyl]-dimethylamin
[3-(4-Chlorophenyl)-3-tetrazol-2-yl-propyl]-dimethylamin
Dimethyl-(3-phenyl-3-[1,2,3]triazol-1-yl-propyl)-amin
Dimethyl-(3-phenyl-3-[1,2,3]triazol-2-yl-propyl)-amin
[3-(4-Chlorophenyl)-3-[1,2,3]triazol-1-yl-propyl]-dimethylamin
[3-(4-Chlorophenyl)-3-[1,2,3]triazol-2-yl-propyl]-dimethylamin
[3-(3,4-Dichlorophenyl)-3-[1,2,3]triazol-2-yl-propyl]-dimethylamin
[3-(3-Methoxynaphthalen-2-yl)-3-tetrazol-2-yl-propyl]-dimethylamin
[3-(3-Methoxynaphthalen-2-yl)-3-(5-methyltetrazol-2-yl)-propyl]-dimethylamin
[3-(6-Fluoronaphthalen-2-yl)-3-tetrazol-2-yl-propyl]-dimethylamin
[3-(6-Fluoronaphthalen-2-yl)-3-(5-methyltetrazol-2-yl)-propyl]-dimethylamin
[3-(1,4-Dimethoxynaphthalen-2-yl)-3-tetrazol-2-yl-propyl]-dimethylamin
[3-(1,4-Dimethoxynaphthalen-2-yl)-3-(5-methyltetrazol-2-yl)-propyl]-dimethylamin
[3-(3,5-Dimethoxynaphthalen-2-yl)-3-tetrazol-2-yl-propyl]-dimethylamin
[3-(3,5-Dimethoxynaphthalen-2-yl)-3-(5-methyltetrazol-2-yl)-propyl]-dimethylamin
[3-(3,5-Dimethoxynaphthalen-2-yl)-3-tetrazol-1-yl-propyl]-dimethylamin
Dimethyl-[3-(4-methylnaphthalen-1-yl)-3-tetrazol-2-yl-propyl]-amin
Dimethyl-[3-(4-methylnaphthalen-1-yl)-3-(5-methyltetrazol-2-yl)-propyl]-amin
[3-(4-Fluoronaphthalen-1-yl)-3-tetrazol-2-yl-propyl]-dimethylamin
[3-(4-Fluoronaphthalen-1-yl)-3-(5-methyltetrazol-2-yl)-propyl]-dimethylamin
(3-Isochinolin-1-yl-3-tetrazol-2-yl-propyl)-dimethylamin
[3-Isochinolin-1-yl-3-(5-methyltetrazol-2-yl)-propyl]-dimethylamin
(3-Benzo[1,3]dioxol-5-yl-3-tetrazol-2-yl-propyl)-dimethylamin
[3-Benzo[1,3]dioxo1-5-yl-3-(5-methyltetrazol-2-yl)-propyl]-dimethylamin
[3-Benzo[1,3]dioxol-5-yl-3-(5-methyltetrazol-1-yl)-propyl]-dimethylamin
[3-(3,4-Dimethoxyphenyl)-3-tetrazol-2-yl-propyl]-dimethylamin
[3-(2,3-Dihydrobenzo[1,4]dioxin-2-yl)-3-tetrazol-2-yl-propyl]-dimethylamin
[3-(2,3-Dihydrobenzo[1,4]dioxin-2-yl)-3-(5-methyltetrazol-2-yl)-propyl]-dimethylamin
Dimethyl-(3-chinolin-2-yl-3-tetrazol-2-yl-propyl)-amin
Dimethyl-[3-(5-methyltetrazol-2-yl)-3-chinolin-2-yl-propyl]-amin
Dimethyl-[3-(1-methyl-1H-indol-2-yl)-3-tetrazol-2-yl-propyl]-amin
Dimethyl-[3-(1-methyl-1H-indol-2-yl)-3-(5-methyltetrazol-2-yl)-propyl]-amin
Dimethyl-[3-(1,2,3,4-tetrahydronaphthalen-2-yl)-3-tetrazol-2-yl-propyl]-amin
[3-(6-Chloropyridin-3-yl)-3-tetrazol-2-yl-propyl]-dimethylamin
(3-Benzo[b]thiophen-2-yl-3-tetrazol-2-yl-propyl)-dimethylamin
[3-Benzo[b]thiophen-2-yl-3-(5-methyltetrazol-2-yl)-propyl]-dimethylamin
(3-Benzofuran-2-yl-3-tetrazol-2-yl-propyl)-dimethylamin
[3-Benzofuran-2-yl-3-(5-methyltetrazol-2-yl)-propyl]-dimethylamin
(3-Benzofuran-2-yl-3-tetrazol-1-yl-propyl)-dimethylamin
[3-(3,4-Difluorophenyl)-3-(5-methyltetrazol-2-yl)-propyl]-dimethylamin
Dimethyl-[3-tetrazol-2-yl-3-(2,4,5-trifluorophenyl)-propyl]-amin
Dimethyl-[3-(5-methyltetrazol-2-yl)-3-(2,4,5-trifluorophenyl)-propyl]-amin
[3-(3-Bromo-4-fluorophenyl)-3-tetrazol-2-yl-propyl]-dimethylamin
[3-(3-Bromo-4-fluorophenyl)-3-(5-methyltetrazol-2-yl)-propyl]-dimethylamin
[3-(6-Methoxynaphthalen-2-yl)-3-(5-methyltetrazol-2-yl)-propyl]-dimethylamin
Dimethyl-[3-(5-methyltetrazol-2-yl)-3-(4-phenoxyphenyl)-propyl]-amin
[3-(4-Bromophenyl)-3-tetrazol-2-yl-propyl]-dimethylamin
[3-(4-Bromophenyl)-3-(5-methyltetrazol-2-yl)-propyl]-dimethylamin
Dimethyl-[3-tetrazol-2-yl-3-(4-trifluoromethylphenyl)-propyl]-amin
Dimethyl-[3-(5-methyltetrazol-2-yl)-3-(4-trifluoromethylphenyl)-propyl]-amin
Dimethyl-[3-tetrazol-2-yl-3-(2,3,4-trichlorophenyl)-propyl]-amin
Dimethyl-[3-(5-methyltetrazol-2-yl)-3-(2,3,4-trichlorophenyl)-propyl]-amin
[3-(3-Chloro-4-methoxyphenyl)-3-tetrazol-2-yl-propyl]-dimethylamin
[3-(3-Chloro-4-methoxyphenyl)-3-(5-methyltetrazol-2-yl)-propyl]-dimethylamin
[3-(4-tert-Butylphenyl)-3-(5-methyltetrazol-2-yl)-propyl]-dimethylamin
[3-(2,5-Difluorophenyl)-3-(5-methyltetrazol-2-yl)-propyl]-dimethylamin
[3-(2,4-Dichlorophenyl)-3-(5-methyltetrazol-2-yl)-propyl]-dimethylamin
[3-(3-Chlorophenyl)-3-(5-methyltetrazol-2-yl)-propyl]-dimethylamin
[3-(2-Chlorophenyl)-3-(5-methyltetrazol-2-yl)-propyl]-dimethylamin
[3-(2,5-Difluorophenyl)-3-tetrazol-2-yl-propyl]-dimethylamin
[3-(2,4-Dichlorophenyl)-3-tetrazol-2-yl-propyl]-dimethylamin
[3-(3-Chlorophenyl)-3-tetrazol-2-yl-propyl]-dimethylamin
Dimethyl-[3-(5-methyltetrazol-2-yl)-3-(4-trifluoromethoxyphenyl)-propyl]-amin
[3-(3-Bromo-4-methylphenyl)-3-(5-methyltetrazol-2-yl)-propyl]-dimethylamin
Dimethyl-[3-tetrazol-2-yl-3-(4-trifluoromethoxyphenyl)-propyl]-amin
[3-(4-Bromo-3-methylphenyl)-3-tetrazol-2-yl-propyl]-dimethylamin
[3-(4-Methanesulfonylphenyl)-3-(5-methyltetrazol-2-yl)-propyl]-dimethylamin
[3-(4-Isopropylphenyl)-3-(5-methyltetrazol-2-yl)-propyl]-dimethylamin
[3-(9H-Fluoren-2-yl)-3-(5-methyltetrazol-2-yl)-propyl]-dimethylamin
Methyl-(3-naphthalen-2-yl-3-[1,2,3]triazol-2-yl-propyl)-amin
[3-Benzotriazol-2-yl-3-(3,4-dichlorophenyl)-propyl]-methylamin
Methyl-[3-(5-methyltetrazol-2-yl)-3-naphthalen-2-yl-propyl]-amin
(3-Benzotriazol-2-yl-3-naphthalen-2-yl-propyl)-methylamin
(3-Biphenyl-4-yl-3-tetrazol-2-yl-propyl)-methylamin
[3-(3,4-Dichlorophenyl)-3-(5-methyltetrazol-2-yl)-propyl]-methylamin
[3-(4-Chlorophenyl)-3-tetrazol-2-yl-propyl]-methylamin
[3-(3,4-Dichlorophenyl)-3-[1,2,3]triazol-2-yl-propyl]-methylamin
[3-(3,4-Dichlorophenyl)-3-tetrazol-2-yl-propyl]-methylamin
Methyl-(3-naphthalen-2-yl-3-tetrazol-2-yl-propyl)-amin
[3-(6-Chloronaphthalen-2-yl)-3-tetrazol-2-yl-propyl]-methylamin
Methyl-(3-naphthalen-1-yl-3-tetrazol-2-yl-propyl)-amin
Methyl-[3-(6-methylnaphthalen-2-yl)-3-tetrazol-2-yl-propyl]-amin
Methyl-[3-naphthalen-2-yl-3-(5-phenyltetrazol-2-yl)-propyl]-amin
(3S)-3-(3,4-Dichlorophenyl)-N-methyl-3-(2H-tetrazol-2-yl)propan-1-amin
(3R)-3-(3,4-Dichlorophenyl)-N-methyl-3-(2H-tetrazol-2-yl)propan-1-amin
(3S)-3-(2H-Benzotriazol-2-yl)-3-(3,4-dichlorophenyl)-N-methylpropan-1-amin
(3R)-3-(2H-Benzotriazol-2-yl)-3-(3,4-dichlorophenyl)-N-methylpropan-1-amin
(3S)-3-(3,4-Dichlorophenyl)-N-methyl-3-(2H-1,2,3-triazol-2-yl)propan-1-amin
(3S)-3-(3,4-Dichlorophenyl)-N-methyl-3-(5-methyl-2H-tetrazol-2-yl)propan-1-amin
(3R)-3-(3,4-Dichlorophenyl)-N-methyl-3-(5-methyl-2H-tetrazol-2-yl)propan-1-amin
(3R)-3-(3,4-Dichlorophenyl)-N-methyl-3-(2H-1,2,3-triazol-2-yl)propan-1-amin
(3R)-N-Methyl-3-(2-naphthyl)-3-(2H-tetrazol-2-yl)propan-1-amin
(3S)-N-Methyl-3-(2-naphthyl)-3-(2H-tetrazol-2-yl)propan-1-amin
[3-(3-Methoxynaphthalen-2-yl)-3-tetrazol-2-yl-propyl]-methylamin
[3-(3-Methoxynaphthalen-2-yl)-3-(5-methyltetrazol-2-yl)-propyl]-methylamin
[3-(6-Fluoronaphthalen-2-yl)-3-tetrazol-2-yl-propyl]-methylamin
[3-(6-Fluoronaphthalen-2-yl)-3-(5-methyltetrazol-2-yl)-propyl]-methylamin
[3-(1,4-Dimethoxynaphthalen-2-yl)-3-tetrazol-2-yl-propyl]-methylamin
[3-(1,4-Dimethoxynaphthalen-2-yl)-3-(5-methyltetrazol-2-yl)-propyl]-methylamin
[3-(3,5-Dimethoxynaphthalen-2-yl)-3-tetrazol-2-yl-propyl]-methylamin
[3-(3,5-Dimethoxynaphthalen-2-yl)-3-(5-methyltetrazol-2-yl)-propyl]-methylamin
[3-(3,5-Dimethoxynaphthalen-2-yl)-3-tetrazol-1-yl-propyl]-methylamin
Methyl-[3-(4-methylnaphthalen-1-yl)-3-tetrazol-2-yl-propyl]-amin
Methyl-[3-(4-methylnaphthalen-1-yl)-3-(5-methyltetrazol-2-yl)-propyl]-amin
[3-(4-Fluoronaphthalen-1-yl)-3-tetrazol-2-yl-propyl]-methylamin
[3-(4-Fluoronaphthalen-1-yl)-3-(5-methyltetrazol-2-yl)-propyl]-methylamin
(3-Benzo[1,3]dioxo1-5-yl-3-tetrazol-2-yl-propyl)-methylamin
[3-Benzo[1,3]dioxol-5-yl-3-(5-methyltetrazol-2-yl)-propyl]-methylamin
[3-Benzo[1,3]dioxol-5-yl-3-(5-methyltetrazol-1-yl)-propyl]-methylamin
[3-(3,4-Dimethoxyphenyl)-3-tetrazol-2-yl-propyl]-methylamin
[3-(2,3-Dihydrobenzo[1,4]dioxin-2-yl)-3-tetrazol-2-yl-propyl]-methylamin
[3-(2,3-Dihydrobenzo[1,4]dioxin-2-yl)-3-(5-methyltetrazol-2-yl)-propyl]-methylamin
Methyl-[3-(1,2,3,4-tetrahydronaphthalen-2-yl)-3-tetrazol-2-yl-propyl]-amin
[3-(6-Chloropyridin-3-yl)-3-tetrazol-2-yl-propyl]-methylamin
(3-Benzo[b]thiophen-2-yl-3-tetrazol-2-yl-propyl)-methylamin
[3-Benzo[b]thiophen-2-yl-3-(5-methyltetrazol-2-yl)-propyl]-methylamin
(3-Benzofuran-2-yl-3-tetrazol-2-yl-propyl)-methylamin
[3-Benzofuran-2-yl-3-(5-methyltetrazol-2-yl)-propyl]-methylamin
(3-Benzofuran-2-yl-3-tetrazol-1-yl-propyl)-methylamin
[3-(3,4-Difluorophenyl)-3-tetrazol-2-yl-propyl]-methylamin
[3-(3,4-Difluorophenyl)-3-(5-methyltetrazol-2-yl)-propyl]-methylamin
Methyl-[3-tetrazol-2-yl-3-(2,4,5-trifluorophenyl)-propyl]-amin
Methyl-[3-(5-methyltetrazol-2-yl)-3-(2,4,5-trifluorophenyl)-propyl]-amin
[3-(3-Bromo-4-fluorophenyl)-3-tetrazol-2-yl-propyl]-methylamin
[3-(3-Bromo-4-fluorophenyl)-3-(5-methyltetrazol-2-yl)-propyl]-methylamin
[3-(6-Methoxynaphthalen-2-yl)-3-tetrazol-2-yl-propyl]-methylamin
[3-(6-Methoxy-naphthalen-2-yl)-3-(5-methyltetrazol-2-yl)-propyl]-methylamin
Methyl-[3-(4-phenoxyphenyl)-3-tetrazol-2-yl-propyl]-amin
Methyl-[3-(5-methyltetrazol-2-yl)-3-(4-phenoxyphenyl)-propyl]-amin
[3-(4-Bromophenyl)-3-tetrazol-2-yl-propyl]-methylamin
[3-(4-Bromophenyl)-3-(5-methyltetrazol-2-yl)-propyl]-methylamin
Methyl-[3-tetrazol-2-yl-3-(4-trifluoromethylphenyl)-propyl]-amin
Methyl-[3-(5-methyltetrazol-2-yl)-3-(4-trifluoromethylphenyl)-propyl]-amin
Methyl-[3-tetrazol-2-yl-3-(2,3,4-trichlorophenyl)-propyl]-amin
Methyl-[3-(5-methyltetrazol-2-yl)-3-(2,3,4-trichlorophenyl)-propyl]-amin
[3-(3-Chloro-4-methoxyphenyl)-3-tetrazol-2-yl-propyl]-methylamin
[3-(3-Chloro-4-methoxyphenyl)-3-(5-methyltetrazol-2-yl)-propyl]-methylamin
[3-(2-Chlorophenyl)-3-(5-methyltetrazol-2-yl)-propyl]-methylamin
[3-(3-Chlorophenyl)-3-(5-methyltetrazol-2-yl)-propyl]-methylamin
[3-(2,4-Dichlorophenyl)-3-(5-methyltetrazol-2-yl)-propyl]-methylamin
[3-(2,5-Difluorophenyl)-3-(5-methyltetrazol-2-yl)-propyl]-methylamin
[3-(3-Chlorophenyl)-3-tetrazol-2-yl-propyl]-methylamin
[3-(2,4-Dichlorophenyl)-3-tetrazol-2-yl-propyl]-methylamin
[3-(2,5-Difluorophenyl)-3-tetrazol-2-yl-propyl]-methylamin
[3-(4-Methanesulfonylphenyl)-3-(5-methyltetrazol-2-yl)-propyl]-methylamin
[3-(4-Isopropylphenyl)-3-(5-methyltetrazol-2-yl)-propyl]-methylamin
[3-(9H-Fluoren-2-yl)-3-(5-methyltetrazol-2-yl)-propyl]-methylamin
(3S)-Methyl-[3-(5-methyltetrazol-2-yl)-3-naphthalen-2-yl-propyl]-amin
(3S)-Methyl-[3-naphthalen-2-yl-3-(5-phenyltetrazol-2-yl)-propyl]-amin
(3R)-Methyl-[3-(5-methyltetrazol-2-yl)-3-naphthalen-2-yl-propyl]-amin
(3R)-Methyl-[3-naphthalen-2-yl-3-(5-phenyltetrazol-2-yl)-propyl]-amin
(3R)-[3-(6-Fluoronaphthalen-2-yl)-3-(5-methyltetrazol-2-yl)-propyl]-methylamin, und
(3S)-[3-(6-Fluoronaphthalen-2-yl)-3-(5-methyltetrazol-2-yl)-propyl]-methylamin.

5. Verbindung nach Anspruch 3, wobei die Verbindung ausgewählt ist aus der Gruppe bestehend aus:
[3-(3,4-Dichlorophenyl)-3-tetrazol-2-yl-propyl]-methylamin,
(R)-[3-(3,4-Dichlorophenyl)-3-tetrazol-2-yl-propyl]-methylamin,
(S)-[3-(3,4-Dichlorophenyl)-3-tetrazol-2-yl-propyl]-methylamin,
[3-(3,4-Dichlorophenyl)-3-(5-methyltetrazol-2-yl)-propyl]-methylamin,
(R)-[3-(3,4-Dichlorophenyl)-3-(5-methyltetrazol-2-yl)-propyl]-methylamin,
(S)-[3-(3,4-Dichlorophenyl)-3-(5-methyltetrazol-2-yl)-propyl]-methylamin,
[3-(3,4-Dichlorophenyl)-3-[1,2,3]triazol-2-yl-propyl]-methylamin,
(R)-[3-(3,4-Dichlorophenyl)-3-[1,2,3]triazol-2-yl-propyl]-methylamin,
(S)-[3-(3,4-Dichlorophenyl)-3-[1,2,3]triazol-2-yl-propyl]-methylamin,
Methyl-(3-naphthalen-2-yl-3-tetrazol-2-yl-propyl)-amin,
(R)-Methyl-(3-naphthalen-2-yl-3-tetrazol-2-yl-propyl)-amin und
(S)-Methyl-(3-naphthalen-2-yl-3-tetrazol-2-yl-propyl)-amin.

6. Pharmazeutische Zusammensetzung, die eine Menge einer racemischen oder Enantiomer-angereicherten Verbindung der Formel (I) nach Anspruch 1 enthält, die zur Verwendung bei der Behandlung von Depression, Angst und Schmerzstörungen wirksam ist.

7. Pharmazeutische Zusammensetzung, die eine Menge einer racemischen oder Enantiomer-angereicherten Verbindung der Formel (III) nach Anspruch 2 enthält, die zur Verwendung bei der Behandlung von Depression, Angst und Schmerzstörungen wirksam ist.

8. Pharmazeutische Zusammensetzung, die eine Menge einer racemischen oder Enantiomer-angereicherten Verbindung der Formel (XI) nach Anspruch 3 enthält, die zur Verwendung bei der Behandlung von Depression, Angst und Schmerzstörungen wirksam ist.

9. Racemische oder Enantiomer-angereicherte Verbindung der Formel (I) nach Anspruch 1, zur Verwendung in einem Verfahren zur Behandlung von Depression, Angst und Schmerzstörungen in einem Säuger, der dessen bedarf.

10. Racemische oder Enantiomer-angereicherte Verbindung der Formel (III) nach Anspruch 2, zur Verwendung in einem Verfahren zur Behandlung von Depression, Angst und Schmerzstörungen in einem Säuger, der dessen bedarf.

11. Racemische oder Enantiomer-angereicherte Verbindung der Formel (XI) nach Anspruch 3, zur Verwendung in einem Verfahren zur Behandlung von Depression, Angst und Schmerzstörungen in einem Säuger, der dessen bedarf.

## Revendications

1. Composé de propanamine substitué en position 3, racémique ou énantiomériquement enrichi, représenté par la formule structurale suivante (I) : 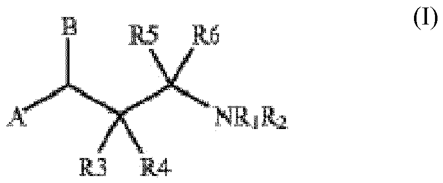 ou un sel pharmaceutiquement acceptable de celui-ci, où ;
A est choisi dans le groupe constitué de phényle, de naphtyle, de benzothiényle, de pyridyle, de quinolyle, d'isoquinolyle, de benzodioxolyle, de benzodioxinyle, d'indolyle, de fluorényle et de benzofuranyle, qui peut être substitué par un ou plusieurs substituants identiques ou différents choisis dans le groupe constitué d'hydrogène, d'halogène, d'alkyle en C₁ à C₄ à chaîne linéaire ou ramifiée, d'alcoxy en C₁ à C₃ à chaîne linéaire ou ramifiée, de phényle, de phényloxy, de nitro, de cyano, de trifluorométhyle, de trifluorométhoxy, de méthanesulfonyle et de thiényle ;
R₁ et R₂ sont identiques ou différents et sont indépendamment choisis dans le groupe constitué d'hydrogène, d'alkyle inférieur ayant de 1 à 4 atomes de carbone, de phényl-alkyle en C₁ à C₄ substitué ou non substitué, ou R₁ et R₂ sont fusionnés avec l'atome d'azote pour former un groupe cyclique qui a de 4 à 7 atomes de carbone ;
R₃, R₄, R₅ et R₆ sont identiques ou différents et sont indépendamment choisis dans le groupe constitué d'hydrogène, d'alkyle en C₁ à C₄ et de phényle substitué ou non substitué ;
B est un azole choisi dans le groupe constitué de triazole, de benzotriazole, de tétrazole, de 5-méthyl tétrazole et de 5-phényl tétrazole qui sont liés à l'azote, comme représenté par les formules structurales suivantes (II) :

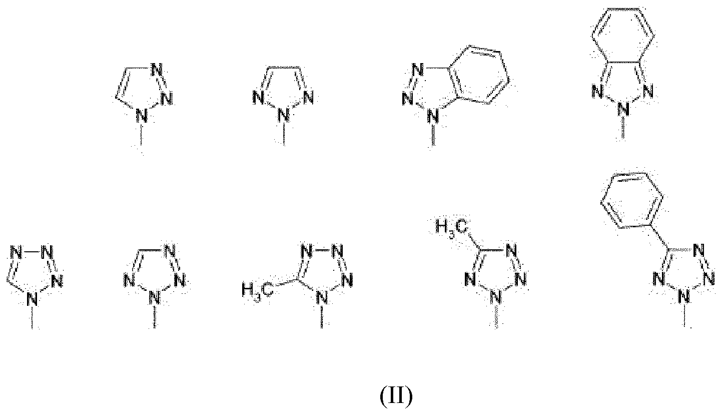

2. Composé de propanamine substitué en position 3, racémique ou énantiomériquement enrichi selon la revendication 1 dans lequel A, B, R₁ et R₂ sont tels que définis dans celui-ci et chacun de R₃ à R₆ est un hydrogène, ledit composé étant représenté par la formule structurale suivante (III) :

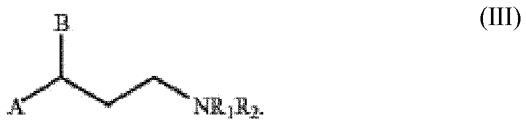

3. Composé de propanamine substitué en position 3, racémique ou énantiomériquement enrichi selon la revendication 1 dans lequel A, B sont tels que définis dans celui-ci, chacun de R₃ à R₆ est un hydrogène et l'un de R₁ et de R₂ est un hydrogène et l'autre est un méthyle, ledit composé étant représenté par la formule structurale suivante (XI) :

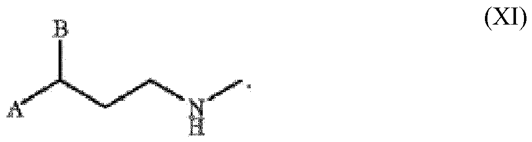

4. Composés de propanamine substitué en position 3, selon la revendication 2 dans lesquels lesdits composés sont choisis dans le groupe constitué de :
[3-(3,4-Dichloro-phényl)-3-tétrazol-2-yl-propyl]-méthyl-amine
S-[3-(3,4-Dichloro-phényl)-3-tétrazol-2-yl-propyl]-méthyl-amine
[3-Benzotriazol-2-yl-3-(3,4-dichloro-phényl)-propyl]-diméthyl-amine
Diméthyl-(3-naphtalén-2-yl-3-[1,2,3]triazol-2-yl-propyl)-amine
Diméthyl-(3-naphtalén-2-yl-3-tétrazol-2-yl-propyl)-amine
(3-Benzotriazol-2-yl-3-naphtalén-2-yl-propyl)-diméthyl-amine
Diméthyl-[3-(5-méthyl-tétrazol-2-yl)-3-naphtalén-2-yl-propyl]-amine
[3-(6-Chloro-naphtalén-2-yl)-3-tétrazol-1-yl-propyl]-diméthyl-amine
Diméthyl-[3-(4-phénoxy-phényl)-3-tétrazol-2-yl-propyl]-amine
Diméthyl-[3-(4-phénoxy-phényl)-3-tétrazol-1-yl-propyl]-aminé
[3-(6-Chloro-naphtalén-2-yl)-3-[1,2,3]triazol-2-yl-propyl]-diméthyl-amine
[3-(6-Chloro-naphtalén-2-yl)-3-[1,2,3]triazol-1-yl-propyl]-diméthyl-amine
[3-(6-Chloro-naphtalén-2-yl)-3-tétrazol-2-yl-propyl]-diméthyl-amine
[3-(4-Méthoxy-phényl)-3-[1,2,3]triazol-2-yl-propyl]-diméthyl-amine
(3-Biphényl-3-yl-3-tétrazol-2-yl-propyl)-diméthyl-amine
[3-(4-Benzyloxy-phényl)-3-tétrazol-2-yl-propyl]-diméthyl-amine
(3-Biphényl-4-yl-3-tétrazol-2-yl-propyl)-diméthyl-amine
Diméthyl-[3-naphtalén-2-yl-3-(5-phényl-tétrazol-2-yl)-propyl]-amine
Diméthyl-[3-(6-méthyl-naphtalén-2-yl)-3-tétrazol-2-yl-propyl]-amine
[3-(6-Méthoxy-naphtalén-2-yl)-3-tétrazol-2-yl-propyl]-diméthyl-amine
[3-(6-Méthoxy-naphtalén-2-yl)-3-[1,2,3]triazol-2-yl-propyl]-diméthyl-amine
[3-Benzotriazol-1-yl-3-(3,4-dichloro-phényl)-propyl]-diméthyl-amine
2-[1-(3,4-Dichloro-phényl)-3-pyrrolidin-1-yl-propyl]-2H-[1,2,3]triazole
[3-(3,4-Dichloro-phényl)-3-[1,2,3]triazol-2-yl-propyl]-diéthyl-amine
[3-(3,4-Dichloro-phényl)-3-tétrazol-2-yl-propyl]-diéthyl-amine
2-[1-(3,4-Dichloro-phényl)-3-pyrrolidin-1-yl-propyl]-2H-tétrazole
Diméthyl-(3-naphtalén-1-yl-3-tétrazol-2-yl-propyl)-amine
Diméthyl-(3-naphtalén-1-yl-3-[1,2,3]triazol-2-yl-propyl)-amine
[3-(3,4-Dichloro-phényl)-3-(5-méthyl-tétrazol-2-yl)-propyl]-diméthyl-amine
[3-(3,4-Dichloro-phényl)-3-(5-phényl-tétrazol-2-yl)-propyl]-diméthyl-amine
[3-(3,4-Difluoro-phényl)-3-tétrazol-1-yl-propyl]-diméthyl-amine
[3-(3,4-Difluoro-phényl)-3-tétrazol-2-yl-propyl]-diméthyl-amine
[3-(3,4-Difluoro-phényl)-3-[1,2,3]triazol-1-yl-propyl]-diméthyl-amine
[3-(3,4-Difluoro-phényl)-3-[1,2,3]triazo1-2-yl-propyl]-diméthyl-amine
[3-(3,4-Diméthyl-phényl)-3-[1,2,3]triazol-1-yl-propyl]-diméthyl-amine
[3-(3,4-Diméthyl-phényl)-3-[1,2,3]triazol-2-yl-propyl]-diméthyl-amine
[3-(3,4-Diméthyl-phényl)-3-tétrazol-1-yl-propyl]-diméthyl-amine
[3-(3,4-Diméthyl-phényl)-3-tétrazol-2-yl-propyl]-diméthyl-amine
[3-(4-Chloro-phényl)-3-tétrazol-1-yl-propyl]-diméthyl-amine
[3-(4-Chloro-phényl)-3-tétrazol-2-yl-propyl]-diméthyl-amine
Diméthyl-(3-phényl-3-[1,2,3]triazol-1-yl-propyl)-amine
Diméthyl-(3-phényl-3-[1,2,3]triazol-2-yl-propyl)-amine
[3-(4-Chloro-phényl)-3-[1,2,3]triazol-1-yl-propyl]-diméthyl-amine
[3-(4-Chloro-phényl)-3-[1,2,3]triazol-2-yl-propyl]-diméthyl-amine
[3-(3,4-Dichloro-phényl)-3-[1,2,3]triazol-2-yl-propyl]-diméthyl-amine
[3-(3-Méthoxy-naphtalén-2-yl)-3-tétrazol-2-yl-propyl]-diméthyl-amine
[3-(3-Méthoxy-naphtalén-2-yl)-3-(5-méthyl-tétrazol-2-yl)-propyl]-diméthyl-amine
[3-(6-Fluoro-naphtalén-2-yl)-3-tétrazol-2-yl-propyl]-diméthyl-amine
[3-(6-Fluoro-naphtalén-2-yl)-3-(5-méthyl-tétrazol-2-yl)-propyl]-diméthyl-amine
[3-(1,4-Diméthoxy-naphtalén-2-yl)-3-tétrazol-2-yl-propyl]-diméthyl-amine
[3-(1,4-Diméthoxy-naphtalén-2-yl)-3-(5-méthyl-tétrazol-2-yl)-propyl]-diméthyl-amine
[3-(3,5-Diméthoxy-naphtalén-2-yl-3-tétrazol-2-yl-propyl]-diméthyl-amine
[3-(3,5-Diméthoxy-naphtalén-2-yl)-3-(5-méthyl-tétrazol-2-yl)-propyl]-diméthyl-amine
[3-(3,5-Diméthoxy-naphtalén-2-yl)-3-tétrazol-1-yl-propyl]-diméthyl-amine
Diméthyl-[3-(4-méthyl-naphtalén-1-yl)-3-tétrazol-2-yl-propyl]-amine
Diméthyl-[3-(4-méthyl-naphtalén-1-yl)-3-(5-méthyl-tétrazol-2-yl)-propyl]-amine
[3-(4-Fluoro-naphtalén-1-yl)-3-tétrazol-2-yl-propyl]-diméthyl-amine
[3-(4-Fluoro-naphtalén-1-yl)-3-(5-méthyl-tétrazol-2-yl)-propyl]-diméthyl-amine
(3-Isoquinolin-1-yl-3-tétrazol-2-yl-propyl)-diméthyl-amine
[3-Isoquinolin-1-yl-3-(5-méthyl-tétrazol-2-yl)-propyl]-diméthyl-amine
(3-Benzo[1,3]dioxo1-5-yl-3-tétrazol-2-yl-propyl)-dimethyl-amine
[3-Benzo[1,3]dioxol-5-yl-3-(5-méthyl-tétrazol-2-yl)-propyl]-diméthyl-amine
[3-Benzo[1,3]dioxol-5-yl-3-(5-méthyl-tétrazol-1-yl)-propyl]-diméthyl-amine
[3-(3,4-Diméthoxy-phényl)-3-tétrazol-2-yl-propyl]-diméthyl-amine
[3-(2,3-Dihydro-benzo[1,4]dioxin-2-yl)-3-tétrazol-2-yl-propyl]-diméthyl-amine
[3-(2,3-Dihydro-benzo[1,4]dioxin-2-yl)-3-(5-méthyl-tétrazol-2-yl)-propyl]-diméthyl-amine
Diméthyl-(3-quinolin-2-yl-3-tétrazol-2-yl-propyl)-amine
Diméthyl-[3-(5-méthyl-tétrazol-2-yl)-3-quinolin-2-yl-propyl]-amine
Diméthyl-[3-(1-méthyl-H-indo1-2-yl)-3-tétrazol-2-yl-propyl]-amine
Diméthyl-[3-(1-méthyl-1H-indol-2-yl)-3-(5-méthyl-tétrazol-2-yl)-propyl]-amine
Diméthyl-[3-(1,2,3,4-tétrahydro-naphtalén-2-yl)-3-tétrazol-2-yl-propyl]-amine
[3-(6-Chloro-pyridin-3-yl)-3-tétrazol-2-yl-propyl]-diméthyl-amine
(3-Benzo[b]thiophén-2-yl-3-tétrazol-2-yl-propyl)-diméthyl-amine
[3-Benzo[b]thiophén-2-yl-3-(5-méthyl-tétrazol-2-yl)-propyl]-diméthyl-amine
(3-Benzofuran-2-yl-3-tétrazol-2-yl-propyl)-diméthyl-amine
[3-Benzofuran-2-yl-3-(5-méthyl-tétrazol-2-yl)-propyl]-diméthyl-amine
(3-Benzofuran-2-yl-3-tétrazol-1-yl-propyl)-diméthyl-amine
[3-(3,4-Difluoro-phényl)-3-(5-méthyl-tétrazol-2-yl)-propyl]-diméthyl-amine
Diméthyl-[3-tétrazol-2-yl-3-(2,4,5-trifluoro-phényl)-propyl]-amine
Diméthyl-[3-(5-méthyl-tétrazol-2-yl)-3-(2,4,5-trifluoro-phényl)-propyl]-amine
[3-(3-Bromo-4-fluoro-phényl)-3-tétrazol-2-yl-propyl]-diméthyl-amine
[3-(3-Bromo-4-fluoro-phényl)-3-(5-méthyl-tétrazol-2-yl)-propyl]-diméthyl-amine
[3-(6-Méthoxy-naphtalén-2-yl)-3-(5-méthyl-tétrazol-2-yl)-propyl]-diméthyl-amine
Diméthyl-[3-(5-méthyl-tétrazol-2-yl)-3-(4-phénoxy-phényl)-propyl]-amine
[3-(4-Bromo-phényl)-3-tétrazol-2-yl-propyl]-diméthyl-amine
[3-(4-Bromo-phényl)-3-(5-méthyl-tétrazol-2-yl)-propyl]-diméthyl-amine
Diméthyl-[3-tétrazol-2-yl-3-(4-trifluorométhyl-phényl)-propyl]-amine
Diméthyl-[3-(5-méthyl-tétrazol-2-yl)-3-(4-trifluorométhyl-phényl)-propyl]-amine
Diméthyl-[3-tétrazol-2-yl-3-(2,3,4-trichloro-phényl)-propyl]-amine
Diméthyl-[3-(5-méthyl-tétrazol-2-yl)-3-(2,3,4-trichloro-phényl)-propyl]-amine
[3-(3-Chloro-4-méthoxy-phényl)-3-tétrazol-2-yl-propyl]-diméthyl-amine
[3-(3-Chloro-4-méthoxy-phényl)-3-(5-méthyl-tétrazol-2-yl)-propyl]-diméthyl-amine
[3-(4-tert-Butyl-phényl)-3-(5-méthyl-tétrazol-2-yl)-propyl]-diméthyl-amine
[3-(2,5-Difluoro-phényl)-3-(5-méthyl-tétrazol-2-yl)-propyl]-diméthyl-amine
[3-(2,4-Dichloro-phényl)-3-(5-méthyl-tétrazol-2-yl)-propyl]-diméthyl-amine
[3-(3-Chloro-phényl)-3-(5-méthyl-tétrazol-2-yl)-propyl]-diméthyl-amine
[3-(2-Chloro-phényl)-3-(5-méthyl-tétrazol-2-yl)-propyl]-diméthyl-amine
[3-(2,5-Difluoro-phényl)-3-tétrazol-2-yl-propyl]-diméthyl-amine
[3-(2,4-Dichloro-phényl)-3-tétrazol-2-yl-propyl]-diméthyl-amine
[3-(3-Chloro-phényl)-3-tétrazol-2-yl-propyl]-diméthyl-amine
Diméthyl-[3-(5-méthyl-tétrazol-2-yl)-3-(4-trifluorométhoxy-phényl)-propyl]-amine
[3-(3-Bromo-4-méthyl-phényl)-3-(5-méthyl-tétrazol-2-yl)-propyl]-diméthyl-amine
Diméthyl-[3-tétrazol-2-yl-3-(4-trifluorométhoxy-phényl)-propyl]-amine [3-(4-Bromo-3-méthyl-phényl)-3-tétrazol-2-yl-propyl]-diméthyl-amine
[3-(4-Méthanesulfonyl-phényl)-3-(5-méthyl-tétrazol-2-yl)-propyl]-diméthyl-amine
[3-(4-Isopropyl-phényl)-3-(5-méthyl-tétrazol-2-yl)-propyl]-diméthyl-amine
[3-(9H-Fluorèn-2-yl)-3-(5-méthyl-tétrazol-2-yl)-propyl]-diméthyl-amine
Méthyl-(3-naphtalén-2-yl-3-[1,2,3]triazol-2-yl-propyl)-amine
[3-Benzotriazol-2-yl-3-(3,4-dichloro-phényl)-propyl]-méthyl-amine
Méthyl-[3-(5-méthyl-tétrazol-2-yl)-3-naphtalén-2-yl-propyl]-amine
(3-Benzotriazol-2-yl-3-naphtalén-2-yl-propyl)-méthyl-amine
(3-Biphényl-4-yl-3-tétrazol-2-yl-propyl)-méthyl-amine
[3-(3,4-Dichloro-phényl)-3-(5-méthyl-tétrazol-2-yl)-propyl]-méthyl-amine
[3-(4-Chloro-phényl)-3-tétrazol-2-yl-propyl]-méthyl-amine
[3-(3,4-Dichloro-phényl)-3-[1,2,3]triazol-2-yl-propyl]-méthyl-amine
[3-(3,4-Dichloro-phényl)-3-tétrazol-2-yl-propyl]-méthyl-amine
Méthyl-(3-naphtalén-2-yl-3-tétrazol-2-yl-propyl)-amine
[3-(6-Chloro-naphtalén-2-yl)-3-tétrazol-2-yl-propyl]-méthyl-amine
Méthyl-(3-naphtalén-1-yl-3-tétrazol-2-yl-propyl)-amine
Méthyl-[3-(6-méthyl-naphtalén-2-yl)-3-tétrazol-2-yl-propyl]-amine
Méthyl-[3-naphtalén-2-yl-3-(5-phényl-tétrazol-2-yl)-propyl]-amine
(3*S*)-3-(3,4-dichlorophényl)-*N*-méthyl-3-(2*H*-tétrazol-2-yl)propan-1-amine
(3*R*)-3-(3,4-dichlorophényl)-*N*-méthyl-3-(2*H*-tétrazol-2-yl)propan-1-amine
(3*S*)-3-(2*H*-benzotriazol-2-yl)-3-(3,4-dichlorophényl)-*N*-méthylpropan-1-amine
(3*R*)-3-(2*H*-benzotriazol-2-yl)-3-(3,4-dichlorophényl)-*N*-méthylpropan-1-amine
(3*S*)-3-(3,4-dichlorophényl)-*N*-méthyl-3-(2*H*-1,2,3-triazol-2-yl)propan-1-amine
(3*S*)-3-(3,4-dichlorophényl)-*N-*méthyl-3-(5-méthyl-2*H-*tétrazol-2-yl)propan-1-amine
(3*R*)-3-(3,4-dichlorophényl)-*N*-méthyl-3-(5-méthyl-2*H*-tétrazol-2-yl)propan-1-amine
(3*R*)-3-(3,4-dichlorophényl)-*N*-méthyl-3-(2*H*-1,2,3-triazol-2-yl)propan-l-amine
(3*R*)-N-méthyl-3-(2-naphtyl)-3-(2*H*-tétrazol-2-yl)propan-1-amine
(3*S*)-N-méthyl-3-(2-naphtyl)-3-(2*H*-tétrazol-2-yl)propan-1-amine
[3-(3-Méthoxy-naphtalén-2-yl)-3-tétrazol-2-yl-propyl]-méthyl-amine
[3-(3-Méthoxy-naphtalén-2-yl)-3-(5-méthyl-tétrazol-2-yl)-propyl]-méthyl-amine
[3-(6-Fluoro-naphtalén-2-yl)-3-tétrazol-2-yl-propyl]-méthyl-amine
[3-(6-Fluoro-naphtalén-2-yl)-3-(5-méthyl-tétrazol-2-yl)-propyl]-méthyl-amine
[3-(1,4-Diméthoxy-naphtalén-2-yl)-3-tétrazol-2-yl-propyl]-méthyl-amine
[3-(1,4-Diméthoxy-naphtalén-2-yl)-3-(5-méthyl-tétrazol-2-yl)-propyl]-méthyl-amine
[3-(3,5-Diméthoxy-naphtalén-2-yl)-3-tétrazol-2-yl-propyl]-méthyl-amine
[3-(3,5-Diméthoxy-naphtalén-2-yl)-3-(5-méthyl-tétrazol-2-yl)-propyl]-méthyl-amine
[3-(3,5-Diméthoxy-naphtalén-2-yl)-3-tétrazol-1-yl-propyl]-méthyl-amine
Méthyl-[3-(4-méthyl-naphtalén-1-yl)-3-tétrazol-2-yl-propyl]-amine
Méthyl-[3-(4-méthyl-naphtalén-1-yl)-3-(5-méthyl-tétrazol-2-yl)-propyl]-amine
[3-(4-Fluoro-naphtalén-1-yl)-3-tétrazol-2-yl-propyl]-méthyl-amine
[3-(4-Fluoro-naphtalén-1-yl)-3-(5-méthyl-tétrazol-2-yl)-propyl]-méthyl-amine
(3-Benzo[1,3]dioxol-5-yl-3-tétrazol-2-yl-propyl)-méthyl-amine
[3-Benzo[1,3]dioxol-5-yl-3-(5-méthyl-tétrazol-2-yl)-propyl]méthyl-amine
[3-Benzo[1,3]dioxo1-5-yl-3-(5-méthyl-tétrazol-1-yl)-propyl]méthyl-amine
[3-(3,4-Diméthoxy-phényl)-3-tétrazol-2-yl-propyl]-méthyl-amine
[3-(2,3-Dihydro-benzo[1,4]dioxin-2-yl)-3-tétrazol-2-yl-propyl]-méthyl-amine
[3-(2,3-Dihydro-benzo[1,4]dioxin-2-yl)-3-(5-méthyl-tétrazol-2-yl)-propyl]-méthyl-amine
Méthyl-[3-(1,2,3,4-tétrahydro-naphtalén-2-yl)-3-tétrazol-2-yl-propyl]-amine
[3-(6-Chloro-pyridin-3-yl)-3-tétrazol-2-yl-propyl]-méthyl-amine
(3-Benzo[b]thiophén-2-yl-3-tétrazol-2-yl-propyl)-méthyl-amine
[3-Benzo[b]thiophén-2-yl-3-(5-méthyl-tétrazol-2-yl)-propyl]-méthyl-amine
(3-Benzofuran-2-yl-3-tétrazol-2-yl-propyl)-méthyl-amine
[3-Benzofuran-2-yl-3-(5-méthyl-tétrazol-2-yl)-propyl]-méthyl-amine
(3-Benzofuran-2-yl-3-tétrazol-1-yl-propyl)-méthyl-amine
[3-(3,4-Difluoro-phényl)-3-tétrazol-2-yl-propyl]-méthyl-amine
[3-(3,4-Difluoro-phényl)-3-(5-méthyl-tétrazol-2-yl)-propyl]-méthyl-amine
Méthyl-[3-tétrazol-2-yl-3-(2,4,5-trifluoro-phényl)-propyl]-amine
Méthyl-[3-(5-méthyl-tétrazol-2-yl)-3-(2,4,5-trifluoro-phényl)-propyl]-amine
[3-(3-Bromo-4-fluoro-phényl)-3-tétrazol-2-yl-propyl]-méthyl-amine
[3-(3-Bromo-4-fluoro-phényl)-3-(5-méthyl-tétrazol-2-yl)-propyl]-méthyl-amine
[3-(6-Méthoxy-naphtalén-2-yl)-3-tétrazol-2-yl-propyl]-méthyl-amine
[3-(6-Méthoxy-naphtalén-2-yl)-3-(5-méthyl-tétrazol-2-yl)-propyl]-méthyl-amine
Méthyl-[3-(4-phénoxy-phényl)-3-tétrazol-2-yl-propyl]-amine
Méthyl-[3-(5-méthyl-tétrazol-2-yl)-3-(4-phénoxy-phényl)-propyl]-amine
[3-(4-Bromo-phényl)-3-tétrazol-2-yl-propyl]-méthyl-amine
[3-(4-Bromo-phényl)-3-(5-méthyl-tétrazol-2-yl)-propyl]-méthyl-amine
Méthyl-[3-tétrazol-2-yl-3-(4-trifluorométhyl-phényl)-propyl]-amine
Méthyl-[3-(5-méthyl-tétrazol-2-yl)-3-(4-trifluorométhyl-phényl)-propyl]-amine
Méthyl-[3-tétrazol-2-yl-3-(2,3,4-trichloro-phényl)-propyl]-amine
Méthyl-[3-(5-méthyl-tétrazol-2-yl)-3-(2,3,4-trichloro-phényl)-propyl]-amine
[3-(3-Chloro-4-méthoxy-phényl)-3-tétrazol-2-yl-propyl]-méthyl-amine
[3-(3-Chloro-4-méthoxy-phényl)-3-(5-méthyl-tétrazol-2-yl)-propyl]-méthyl-amine
[3-(2-Chloro-phényl)-3-(5-méthyl-tétrazol-2-yl)-propyl]-méthyl-amine
[3-(3-Chloro-phényl)-3-(5-méthyl-tétrazol-2-yl)-Propyl]-méthyl-amine
[3-(2,4-Dichloro-phényl)-3-(5-méthyl-tétrazol-2-yl)-propyl]-méthyl-amine
[3-(2,5-Difluoro-phényl)-3-(5-méthyl-tétrazol-2-yl)-propyl]-méthyl-amine
[3-(3-Chloro-phényl)-3-tétrazol-2-yl-propyl]-méthyl-amine
[3-(2,4-Dichloro-phényl)-3-tétrazol-2-yl-propyl]-méthyl-amine
[3-(2,5-Difluoro-phényl)-3-tétrazol-2-yl-propyl]-méthyl-amine
[3-(4-Méthanesulfonyl-phényl)-3-(5-méthyl-tétrazol-2-yl)-propyl]-méthyl-amine
[3-(4-Isopropyl-phényl)-3-(5-méthyl-tétrazol-2-yl)-propyl]-méthyl-amine
[3-(9H-Fluorèn-2-yl)-3-(5-méthyl-tétrazol-2-yl)-propyl]-méthyl-amine
(3S)-Méthyl-[3-(5-méthyl-tétrazol-2-yl)-3-naphtalén-2-yl-propyl]-amine
(3S)-Méthyl-[3-naphtalén-2-yl-3-(5-phényl-tétrazol-2-yl)-propyl]-amine
(3R)-Méthyl-[3-(5-méthyl-tétrazol-2-yl)-3-naphtalén-2-yl-propyl]-amine
(3R)-Méthyl-[3-naphtalén-2-yl-3-(5-phényl-tétrazol-2-yl)-propyl]-amine
(3R)-[3-(6-Fluoro-naphtalén-2-yl)-3-(5-méthyl-tétrazol-2-yl)-propyl]-méthyl-amine et
de (3S)-[3-(6-Fluoro-naphtalén-2-yl)-3-(5-méthyl-tétrazol-2-yl)-propyl]-méthyl-amine.

5. Composé selon la revendication 3 dans lequel ledit composé est choisi dans le groupe constitué de :
[3-(3,4-Dichloro-phényl)-3-tétrazol-2-yl-propyl]-méthyl-amine,
(R)-[3-(3,4-Dichloro-phényl)-3-tétrazol-2-yl-propyl]-méthyl-amine,
(S)-[3-(3,4-Dichloro-phényl)-3-tétrazol-2-yl-propyl]-méthyl-amine,
[3-(3,4-Dichloro-phényl)-3-(5-méthyl-tétrazol-2-yl)-propyl]-méthyl-amine,
(R)-[3-(3,4-Dichloro-phényl)-3-(5-méthyl-tétrazol-2-yl)-propyl]-méthyl-amine,
(S)-[3-(3,4-Dichloro-phényl)-3-(5-méthyl-tétrazol-2-yl)-propyl]-méthyl-amine,
[3-(3,4-Dichloro-phényl)-3-[1,2,3]triazol-2-yl-propyl]-méthyl-amine,
(R)-[3-(3,4-Dichloro-phényl)-3-[1,2,3]triazol-2-yl-propyl]-méthyl-amine,
(S)-[3-(3,4-Dichloro-phényl)-3-[1,2,3]triazol-2-yl-propyl]-méthyl-amine,
Méthyl-(3-naphtalén-2-yl-3-tétrazol-2-yl-propyl)-amine,
(R)-Méthyl-(3-naphtalén-2-yl-3-tétrazol-2-yl-propyl)-amine, et
de (S)-Méthyl-(3-naphtalén-2-yl-3-tétrazol-2-yl-propyl)-amine.

6. Composition pharmaceutique qui comprend une quantité d'un composé racémique ou énantiomériquement enrichi de formule (I) selon la revendication 1, efficace pour une utilisation dans le traitement de la dépression, de l'anxiété et des troubles de la douleur.

7. Composition pharmaceutique qui comprend une quantité d'un composé racémique ou énantiomériquement enrichi de formule (III) selon la revendication 2, efficace pour une utilisation dans le traitement de la dépression, de l'anxiété et des troubles de la douleur.

8. Composition pharmaceutique qui comprend une quantité d'un composé racémique ou énantiomériquement enrichi de formule (XI) selon la revendication 3, efficace pour une utilisation dans le traitement de la dépression, de l'anxiété et des troubles de la douleur.

9. Composé racémique ou énantiomériquement enrichi de formule (I) selon la revendication 1, destiné à être utilisé dans un procédé de traitement de la dépression, de l'anxiété et des troubles de la douleur chez un mammifère qui en a besoin.

10. Composé racémique ou énantiomériquement enrichi de formule (III) selon la revendication 2, destiné à être utilisé dans un procédé de traitement de la dépression, de l'anxiété et des troubles de la douleur, chez un mammifère qui en a besoin.

11. Composé racémique ou énantiomériquement enrichi de formule (XI) selon la revendication 3, destiné à être utilisé dans un procédé de traitement de la dépression, de l'anxiété et des troubles de la douleur chez un mammifère qui en a besoin.
